(19) Europäisches Patentamt
European Patent Office
Office européen des brevets

(11) EP 1 743 890 A1

(12) EUROPEAN PATENT APPLICATION

(43) Date of publication:
17.01.2007 Bulletin 2007/03

(21) Application number: 05384029.4

(22) Date of filing: 15.07.2005

(51) Int Cl.:
*C07D 231/06* (2006.01) *C07D 403/12* (2006.01)
*C07D 409/04* (2006.01) *A61K 31/4155* (2006.01)
*A61K 31/415* (2006.01) *A61P 3/04* (2006.01)
*A61P 25/30* (2006.01) *A61P 35/00* (2006.01)

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(71) Applicant: **LABORATORIOS DEL DR. ESTEVE, S.A.**
**08041 Barcelona (ES)**

(72) Inventors:
- **Torrens Jover, Antonio**
  **08221 Terrasa (Barcelona) (ES)**
- **Mas Prio, José**
  **08191 Rubí (Barcelona) (ES)**
- **Buschmann, Helmut, Heinrich**
  **08960 Sant Just Desvern (Barcelona) (ES)**
- **Quintana Ruiz, Jordi Ramón**
  **08391 Tiana (Barcelona) (ES)**
- **Yenes Minguez, Susana**
  **08750 Molins de Rei (Barcelona) (ES)**

# (54) 4,5-Dihydro-1H-pyrazole derivatives, their preparation and use as medicaments

(57) The present invention relates to 4-substituted pyrazoline compounds of formula I methods for their preparation, medicaments comprising these compounds as well as their use for the preparation of a medicament for the treatment of humans and animals.

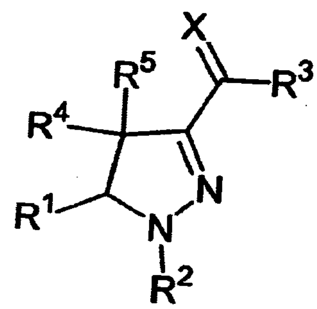

I

EP 1 743 890 A1

## Description

The present invention relates to 4-substituted pyrazoline compounds, methods for their preparation, medicaments comprising these compounds as well as their use for the preparation of a medicament for the treatment of humans and animals.

Cannabinoids are compounds, which are derived from the cannabis sativa plant which is commonly known as marijuana. The most active chemical compound of the naturally occurring cannabinoids is tetrahydrocannabinol (THC), particularly $\Delta^9$-THC.

These naturally occurring cannabinoids as well as their synthetic analogues promote their physiological effects via binding to specific G-coupled receptors, the so-called cannabinoid-receptors.

At present, two distinct types of receptors that bind both the naturally occurring and synthetic cannabinoids have been identified and cloned. These receptors, which are designated $CB_1$ and $CB_2$ are involved in a variety of physiological or pathophysiological processes in humans and animals, e. g. processes related to the central nervous system, immune system, cardiovascular system, endocrinous system, respiratory system, the gastrointestinal tract or to reproduction, as described for example, in Hollister, Pharm. Rev. 38, 1986, 1-20; Reny and Singha, Prog. Drug. Res., 36, 71-114, 1991; Consroe and Sandyk, in Marijuana/Cannabinoids, Neurobiology and Neurophysiology, 459, Murphy L. and Barthe A. Eds., CRC Press, 1992.

Therefore, compounds, which have a high binding affinity for these cannabinoid receptors and which are suitable for modulating these receptors are useful in the prevention and/or treatment of cannabinoid-receptor related disorders.

In particular, the $CB_1$-receptor is involved in many different food-intake related disorders such as bulimia or obesity, including obesity associated with type II diabetes (non-insulin-dependent diabetes) and thus, compounds suitable for regulating this receptor may be used in the prophylaxis and/or treatment of these disorders.

Thus, it was an object of the present invention to provide novel compounds for use as active substances in medicaments. In particular, these active substances should be suitable for the modulation of cannabinoid receptors, more particularly for the modulation of cannabinoid 1 ($CB_1$) receptors.

Said object was achieved by providing the 4-substituted pyrazoline compounds of general formula I given below, their stereoisomers, corresponding salts and corresponding solvates thereof.

It has been found that these compounds have a high affinity for cannabinoid receptors, particularly for the $CB_1$-receptor, and that they act as modulators e. g. antagonists, inverse agonists or agonists on these receptors. They are therefore suitable for the prophylaxis and/or treatment of various disorders related to the central nervous system, the immune system, the cardiovascular system, the endocrinous system, the respiratory system, the gastrointestinal tract or reproduction in humans and/or animals, preferably humans including infants, children and grown-ups.

Thus, in one of its aspects the present invention relates to 4-substituted pyrazoline compounds of general formula I,

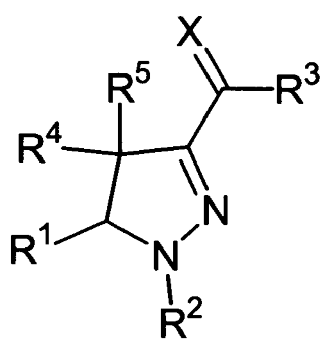

I

wherein

| | |
|---|---|
| X | is O or S; |
| $R^1$ and $R^2$, | independently of one another, in each case represent an unsubstituted or at |

least mono-substituted aryl or heteroaryl radical, which may be condensed with an unsubstituted or at least mono-substituted mono- or polycyclic ring system; or a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bridged by at least one unsubstituted or at least mono-substituted alkylene group;

$R^3$ an unsubstituted or at least mono-substituted aryl or heteroaryl radical, which may be condensed with an unsubstituted or at least mono-substituted mono- or polycyclic ring system;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bridged by at least one unsubstituted or at least mono-substituted alkylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
a $-O-R^6$ moiety; a $-NR^7R^8$ moiety or a $—NR^9-O-R^{10}$ moiety;

$R^4$ represents F; Cl; Br; I; -CN; $-NO_2$; -NC; -OH; $-NH_2$; -SH; -C(=O)-H;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an optionally at least mono-substituted alkylene group, alkenylene group or alkinylene group;
a $—O-R^{11}$ moiety; a $—S-R^{12}$ moiety; a $—NH-R^{13}$ moiety or a $—NR^{14}R^{15}$ moiety;

$R^5$ represents H; F; Cl; Br; I; -CN; $-NO_2$; -NC; -OH; $-NH_2$; -SH; -C(=O)-H;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
a $—O-R^{11}$ moiety; a $—S-R^{12}$ moiety; a $—NH-R^{13}$ moiety or a $—NR^{14}R^{15}$ moiety;

$R^6$ represents a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
a $—P(=O)(OR^{16})_2$ moiety; a $—C(=O)-OR^{17}$ moiety; a $—C(=O)-NH-R^{18}$ moiety or a $—C(=O)-R^{19}$ moiety;

$R^7$ and $R^8$, independently of one another, in each case represent a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono- substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
a $—P(=O)(OR^{16})_2$ moiety; a $—C(=O)-OR^{17}$ moiety; a $—C(=O)-NH-R^{18}$ moiety; a $— C(=O)-R^{19}$ moiety; a $-S(=O)_2-R^{20}$ moiety; or a $—NR^{21}R^{22}$ moiety;

$R^9$ represents hydrogen or a saturated or unsaturated, unsubstituted or at least mono-substituted aliphatic radical;

$R^{10}$, $R^{11}$, $R^{12}$, $R^{13}$, $R^{14}$, $R^{15}$ and $R^{20}$, independently of one another, in each case represent a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

$R^{16}$, $R^{17}$, $R^{18}$ and $R^{19}$, independently of one another, in each case represent a saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

and

$R^{21}$ and $R^{22}$, independently of one another, in each case represent a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof. The term stereoisomers includes tautomers as well.

If one or more of the residues $R^1$ to $R^{22}$ represents or comprises an aryl or heteroaryl radical, which may be substituted, unless defined otherwise, preferably said aryl or heteroaryl radical may optionally be substituted with 1, 2, 3, 4 or 5 substituent(s) independently selected from the group consisting of $C_{1-6}$-perfluoralkyl, -$C_{1-6}$-alkyl substituted with one or more methoxy and/or ethoxy groups, -$C_{1-6}$-alkyl, -$C_{1-6}$-alkyl substituted with one or more hydroxy groups, -$C_{1-6}$-alkyl substituted with one or more chlorine atoms, -O-$C_{1-6}$-alkyl, -O-$C_{1-6}$-alkyl substituted with one or more methoxy and/or ethoxy groups, -S-$C_{1-6}$-alkyl, -C(=O)-OH, -C(=O)-O-$C_{1-6}$-alkyl, -O-C(=O)-$C_{1-6}$-alkyl, F, Cl, Br, I, -CN, -$OCF_3$, -O-$C_2F_5$, -O-$C_3F_7$, -O-$C_4F_9$, -$SCF_3$, -$SCF_3$, -$SCF_2H$, 6$SCFH_2$, -OH, -SH, -$SO_3H$, -NH-C(=O)-$C_{1-6}$-alkyl, -N($C_{1-6}$-alkyl)-C(=O)-$C_{1-6}$-alkyl, -$NO_2$, -CHO, -C(=O)-$C_{1-6}$-alkyl, -C(=O)-$C_{1-6}$-perfluoroalkyl, -C(=S)-NH-$C_{1-6}$-alkyl, -$CF_2H$,- $CFH_2$, -C(=O)-$NR^AR^B$, -C(=O)-NH-$NR^CR^D$, -S(=O)-$C_{1-6}$-alkyl, -$S(=O)_2$-$C_{1-6}$-alkyl, -$S(=O)_2$-phenyl, -($C_{1-5}$-alkylene)-S-$C_{1-6}$-alkyl, -($C_{1-5}$-alkylene)-S(=O)-$C_{1-6}$-alkyl, -($C_{1-5}$-alkylene)-$S(=O)_2$-$C_{1-6}$-alkyl, -$NR^ER^F$, -($C_{1-5}$-alkylene)-$NR^ER^F$, -S(=O)-$NH_2$, -$S(=O)_2$-NH-$C_{1-6}$-alkyl, -$S(=O)_2$-NH-phenyl, -NH-$S(=O)_2$-$C_{1-6}$-alkyl, cyclopropyl, cyclobutyl, cyclopentyl, cyclohexyl, pyrrolidinyl, piperidinyl, phenyl, thiophenyl, phenoxy and benzyl;
whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, -$CF_3$, -CN, -$NO_2$, -$C_{1-6}$-alkyl, -O-$C_{1-6}$-alkyl, -O-$CF_3$ and -S-$CF_3$ and whereby $R^A$, $R^B$, $R^E$ and $R^F$, independently of one another, represent hydrogen or -$C_{1-6}$-alkyl or $R^A$ and $R^B$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different $C_{1-6}$ alkyl radicals
and whereby $R^C$ and $R^D$, independently of one another, represent hydrogen, -$C_{1-6}$-alkyl, -C(=O)-O-$C_{1-6}$-alkyl, $C_{3-8}$-cycloalkyl, -($C_{1-5}$-alkylene)-$C_{3-8}$-cycloalkyl, -($C_{1-6}$-alkylene)-O-$C_{1-6}$-alkyl or -$C_{1-6}$-alkyl substituted with one or more hydroxy groups or $R^C$ and $R^D$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting -$C_{1-6}$-alkyl, -C(=O)-$C_{1-6}$-alkyl, -C(=O)-O-$C_{1-6}$-alkyl, -C(=O)-NH-$C_{1-6}$-alkyl, -C(=S)-NH-$C_{1-6}$-alkyl, oxo (=O), -$C_{1-6}$-alkyl substituted with one or more hydroxy groups, -($C_{1-6}$-alkylene)-O-$C_{1-6}$-alkyl and -C(=O)-$NH_2$.

More preferably said aryl and heteroaryl radicals may optionally be substituted with 1, 2, 3, 4 or 5 substituent (s) independently selected from the group consisting of —$CF_3$, -$C_2F_5$, -$C_3F_7$, -$C_4F_9$, -$CH_2Cl$, -$CHCl_2$, -$C_2H_4Cl$, methyl,

ethyl, n-propyl, isopropyl, n-butyl, isobutyl, 2-butyl, tert-butyl, n-pentyl, 2-pentyl, n-hexyl, $-CH_2-OH$, $-CH_2-CH_2-OH$,$-CH_2-CH_2-CH_2-OH$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -C(=O)-OH, $-C(=O)-O-CH_3$, $-C(=O)-O-C_2H_5$, $-C(=O)-O-C_3H_7$, $-C(=O)-O-C(CH_3)_3$, $-O-C(=O)-CH_3$, $-O-C(=O)-C_2H_5$, $-O-C(=O)-CH(CH_3)_2$, $-O-C(=O)-CH_2-CH_2-CH_3$, $-O-C(=O)-C(CH_3)_3$, F, Cl, Br, I, -CN, $-OCF_3$, $-O-C_2F_5$, $-O-C_3F_7$, $-O-C_4F_9$, $-SCF_3$, $-SCF_2H$, $-SCFH_2$, -OH, -SH, $-SO_3H$, $-NH-C(=O)-CH_3$, $-NH-C(=O)-C_2H_5$, $-NH-C(=O)-C(CH_3)_3$, $-NO_2$, -CHO, $-C(=O)-CH_3$, $-C(=O)-C_2H_5$, $-C(=O)-C(CH_3)_3$, $-C(=O)-CF_3$, $-C(=O)-C_2F_5$, $-C(=O)-C_3F_7$, $-C(=S)-NH-CH_3$, $-C(=S)-NH-C_2H_5$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-C(=O)-NH-NH-CH_3$, $-C(=O)-NH-NH-C_2H_5$, $-C(=O)-NH-NH_2$, $-C(=O)-NH-N(CH_3)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-S(=O)_2$-phenyl, $-NH_2$, $-NH-CH_3$, $-NH-C_2H_5$, $-N(CH_3)_2$, $-N(C_2H_5)_2$, $-CH_2-N(CH_3)_2$, —$(CH_2)$-morpholinyl, -$(CH_2)$-piperidinyl, -$(CH_2)$-piperazinyl, $-(CH_2)-N(C_2H_5)_2$, $-CH_2-N(C_3H_7)_2$, $-CH_2-N(C_4H_9)_2$, $-CH_2-N(CH_3)(C_2H_5)$, $-S(=O)-NH_2$, $-S(=O)_2-NH-CH_3$, $-S(=O)_2$-NH-phenyl, $-NH-S(=O)_2-CH_3$, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituent(s) independently selected from the group consisting of F, Cl, Br, methyl, ethyl and n-propyl.

Preferred aryl radicals which are optionally at least mono-substituted are phenyl and naphthyl (1- and 2-naphthyl).

Preferably the heteroatoms which are present as ring members in the heteroaryl radical may, unless defined otherwise, independently be selected from the group consisting of nitrogen, oxygen and sulfur. More preferably a heteroaryl radical is 5- to 14-membered and may comprise 1, 2, 3 or 4 heteroatoms independently selected from the group consisting of nitrogen, oxygen and sulfur.

Preferred heteroaryl radicals which are unsubstituted or at least mono-substituted are pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl, imidazo[2,1-b]thiazolyl, 2H-chromenyl, pyranyl, indazolyl and quinazolinyl.

Preferred aryl and heteroaryl radicals which are condensed with a mono- or polycyclic ring system are [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl.

If one or more of the residues $R^1$ to $R^{22}$ represents or comprises a saturated or unsaturated, optionally at least one heteroatom as a ring member containing cycloaliphatic radical, preferably a $C_{1-18}$ cycloaliphatic radical, which may be substituted, unless defined otherwise, preferably said cycloaliphatic radical may optionally be substituted with 1, 2, 3, 4 or 5 substituent(s) independently selected from the group consisting of oxo (=O), thioxo (=S), $C_{1-6}$-perfluoralkyl, $-C_{1-6}$-alkyl, $-C_{1-6}$-alkyl substituted with one or more hydroxy groups, $-C_{1-6}$-alkyl substituted with one or more chlorine atoms, $-C_{1-6}$-alkyl substituted with one or more methoxy and/or ethoxy groups, $-O-C_{1-6}$-alkyl, $-O-C_{1-6}$-alkyl substituted with one or more methoxy and/or ethoxy groups, $-S-C_{1-6}$-alkyl, -C(=O)-OH, $-C(=O)-O-C_{1-6}$-alkyl, $-O-C(=O)-C_{1-6}$-alkyl, F, Cl, Br, I, -CN, $-OCF_3$, $-O-C_2F_5$, $-O-C_3F_7$, $-O-C_4F_9$, $-SCF_3$, $-SCF_2H$, $-SCFH_2$, -OH, -SH, $-SO_3H$, $-NH-C(=O)-C_{1-6}$-alkyl, $-N(C_{1-6}$-alkyl$)-C(=O)-C_{1-6}$-alkyl, $-NO_2$, -CHO, $-C(=O)-C_{1-6}$-alkyl, $-C(=O)-C_{1-6}$-perfluoroalkyl, $-C(=S)-NH-C_{1-6}$-alkyl, $-CF_2H$, $-CFH_2$, $-C(=O)-NR^AR^B$, $-C(=O)-NH-NR^CR^D$, $-S(=O)-C_{1-6}$-alkyl, $-S(=O)_2-C_{1-6}$-alkyl, $-S(=O)_2$-phenyl, $-(C_{1-5}$-alkylene$)-S-C_{1-6}$-alkyl, $-(C_{1-5}$-alkylene$)-S(=O)-C_{1-6}$-alkyl, $-(C_{1-5}$-alkylene$)-S(=O)_2-C_{1-6}$-alkyl, $-NR^ER^F$, $-(C_{1-5}$-alkylene$)-NR^ER^F$, $-S(=O)-NH_2$, $-S(=O)_2-NH-C_{1-6}$-alkyl, $-S(=O)_2$-NH-phenyl, $-NH-S(=O)_2-C_{1-6}$-alkyl, cyclopropyl, cyclobutyl, cyclopentyl, cyclohexyl, pyrrolidinyl, piperidinyl, phenyl, thiophenyl, phenoxy and benzyl;

whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, $-CF_3$, -CN, $-NO_2$, $-C_{1-6}$-alkyl, $-O-C_{1-6}$-alkyl, $-O-CF_3$ and $-S-CF_3$ and

whereby $R^A$, $R^B$, $R^E$ and $R^F$, independently of one another, represent hydrogen or $-C_{1-6}$-alkyl or $R^A$ and $R^B$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different $C_{1-6}$ alkyl radicals

and whereby $R^C$ and $R^D$, independently of one another, represent hydrogen, $-C_{1-6}$-alkyl, $-C(=O)-O-C_{1-6}$-alkyl, $C_{3-8}$-cycloalkyl, $-(C_{1-5}$-alkylene$)-C_{3-8}$-cycloalkyl, $-(C_{1-6}$-alkylene$)-O-C_{1-6}$-alkyl or $-C_{1-6}$-alkyl substituted with one or more hydroxy groups or $R^C$ and $R^D$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting $-C_{1-6}$-alkyl, $-C(=O)-C_{1-6}$-alkyl, $-C(=O)-O-C_{1-6}$-alkyl, $-C(=O)-NH-C_{1-6}$-alkyl, $-C(=S)-NH-C_{1-6}$-alkyl, oxo (=O), $-C_{1-6}$-alkyl substituted with one or more hydroxy groups, $-(C_{1-6}$-alkylene$)-O-C_{1-6}$-alkyl and —$C(=O)-NH_2$.

More preferably said cycloaliphatic radicals may optionally be substituted with 1, 2, 3, 4 or 5 substituent(s)

independently selected from the group consisting of oxo (=O), thioxo (=S), $-CF_3$, $-C_2F_5$, $-C_3F_7$, $-C_4F_9$, $-CH_2Cl$, $-CHCl_2$, $-C_2H_4Cl$, methyl, ethyl, n-propyl, isopropyl, n-butyl, isobutyl, 2-butyl, tert-butyl, n-pentyl, 2-pentyl, n-hexyl, $-CH_2-OH$, $-CH_2-CH_2-OH$, $-CH_2-CH_2-CH_2-OH$, $-O-CH_2-O-CH_3$, $-O-CH_2-CH_2-O-CH_3$, $-O-CH_2-O-C_2H_s$, $-C(OCH_3)(C_2H_5)_2$, $-C(OCH_3)(CH_3)_2$, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -C(=O)-OH, $-C(=O)-O-CH_3$, $-C(=O)-O-C_2H_5$, $-C(=O)-O-C_3H_7$, $-C(=O)-O-C(CH_3)_3$, $-O-C(=O)-CH_3$, $-O-C(=O)-C_2H_5$, $-0-C(=O)-CH(CH_3)_2$, $-O-C(=O)-CH_2-CH_2-CH_3$, $-O-C(=O)-C(CH_3)_3$, F, Cl, Br, I, -CN, $-OCF_3$, $-O-C_2F_5$, $-O-C_3F_7$, $-O-C_4F_9$, $-SCF_3$, $-SCF_2H$, $-SCFH_2$, -OH, -SH, $-SO_3H$, $-NH-C(=O)-CH_3$, $-NH-C(=O)-C_2H_5$, $-NH-C(=O)-C(CH_3)_3$, $-NO_2$, -CHO, $-C(=O)-CH_3$, $-C(=O)-C_2H_5$, $-C(=O)-C(CH_3)_3$, $-C(=O)-CF_3$, $-C(=O)-C_2F_5$, $-C(=O)-C_3F_7$, $-C(=S)-NH-CH_3$, $-C(=S)-NH-C_2H_5$, $-CF_2H$, $-CFH_2$, $-C(=O)-NH_2$, $-C(=O)-NH-CH_3$, $-C(=O)-NH-C_2H_5$,- $C(=O)-NH-C_3H_7$, $-C(=O)-N(CH_3)_2$, $-C(=O)-N(C_2H_5)_2$, $-C(=O)-NH-NH-CH_3$, $-C(=O)-NH-NH-C_2H_5$, $-C(=O)-NH-NH_2$, $-C(=O)-NH-N(CH_3)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-S(=O)_2$-phenyl, $-NH_2$, $-NH-CH_3$, $-NH-C_2H_5$, $-N(CH_3)_2$, $-N(C_2H_5)_2$, $-CH_2-N(CH_3)_2$, —$(CH_2)$-morpholinyl, $-(CH_2)$-piperidinyl, $-(CH_2)$-piperazinyl, $-(CH_2)-N(C_2H_5)_2$, $-CH_2-N(C_3H_7)_2$, $-CH_2-N(C_4H_9)_2$, $-CH_2-N(CH_3)(C_2H_5)$, $-S(=O)-NH_2$, $-S(=O)_2-NH-CH_3$, $-S(=O)_2$-NH-phenyl, $-NH-S(=O)_2-CH_3$, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituents independently selected from the group consisting of F, Cl, Br, methyl, ethyl and n-propyl.

If one or more of the residues $R^1$ to $R^{22}$ represents or comprises a cycloaliphatic radical, preferably a $C_{3-16}$ cycloaliphatic radical, which contains one or more heteroatoms as ring members, unless defined otherwise, each of these heteroatoms may preferably be selected from the group consisting of nitrogen, oxygen and sulfur. More preferably a cycloaliphatic group may optionally contain 1, 2, 3 or 4 heteroatom(s) independently selected from the group consisting of N, O and S as ring members.

Suitable saturated or unsaturated, optionally at least one heteroatom as ring member containing cycloaliphatic radicals may preferably be selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl and (1,3)-dioxolanyl.

Suitable saturated or unsaturated, optionally at least one heteroatom as ring member containing cycloaliphatic radicals which are condensed with an unsubstituted or at least mono-substituted mono- or polycyclic ring system may preferably be selected from the group consisting of indolinyl, isoindolinyl, decahydronaphthyl, (1,2,3,4)-tetrahydroquinolinyl, (1,2,3,4)-tetrahydroisoquinolinyl, (1,2,3,4)-tetrahydronaphthyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl and (1,2,3,4)-tetrahydroquinoxazlinyl.

Suitable saturated or unsaturated, optionally at least one heteroatom as ring member containing cycloaliphatic radicals which are bridged by at least one unsubstituted or at least mono-substituted alkylene group may preferably be selected from the group consisting of adamantyl, bicyclo[2.2.1]heptyl, bicyclo[3.1.1]heptyl, norbomenyl and 8-aza-bicyclo [3.2.1]octyl.

A suitable saturated or unsaturated, optionally at least one heteroatom as ring member containing cycloaliphatic radical which together with a saturated or unsaturated, unsubstituted or at least mono-substituted cycloaliphatic radical forms a spirocyclic residue via a common ring atom is 8-aza-spiro[4.5]decanyl.

A mono- or polycyclic ring system according to the present invention — if not defined otherwise - means a mono- or polycyclic hydrocarbon ring system, preferably a mono- or bicyclic ring system, that may be saturated, unsaturated or aromatic. Each of its different rings may show a different degree of saturation, i.e. they may be saturated, unsaturated or aromatic. Optionally each of the rings of the mono- or bicyclic ring system may contain one or more, preferably 1, 2 or 3, heteroatom(s) as ring member(s), which may be identical or different and which can preferably be selected from the group consisting of nitrogen, oxygen and sulfur. The rings of the mono- or bicyclic ring system are preferably 5-, 6- or 7-membered.

The term "condensed" according to the present invention means that a ring or ring system is attached to another ring or ring system, whereby the terms "annulated" or "annelated" are also used by those skilled in the art to designate this kind of attachment.

If one or more of the residues $R^1$ to $R^{22}$ comprises a mono- or polycyclic ring system, which may be substituted, unless defined otherwise, preferably said mono- or polycyclic ring system may optionally be substituted with 1, 2, 3, 4 or 5 substituent(s) independently selected from the group consisting of oxo (=O), thioxo (=S), $C_{1-6}$-perfluoralkyl,

$-C_{1-6}$-alkyl, $-C_{1-6}$-alkyl substituted with one or more hydroxy groups, $-C_{1-6}$-alkyl substituted with one or more chlorine atoms, $-C_{1-6}$-alkyl substituted with one or more methoxy and/or ethoxy groups, $-O-C_{1-6}$-alkyl, $-O-C_{1-6}$-alkyl substituted with one or more methoxy and/or ethoxy groups, $-S-C_{1-6}$-alkyl, -C(=O)-OH, $-C(=O)-O-C_{1-6}$-alkyl, $-O-C(=O)-C_{1-6}$-alkyl, F, Cl, Br, I, -CN, $-OCF_3$, $-O-C_2F_5$, $-O-C_3F_7$, $-O-C_4F_9$, $-SCF_3$, $-SCF_2H$, $-SCFH_2$, -OH, -SH, $-SO_3H$, $-NH-C(=O)-C_{1-6}$-alkyl, $-N(C_{1-6}$-alkyl)$-C(=O)-C_{1-6}$-alkyl, $-NO_2$, -CHO, $-C(=O)-C_{1-6}$-alkyl, $-C(=O)-C_{1-6}$-perfluoroalkyl, $-C(=S)-NH-C_{1-6}$-alkyl, $-CF_2H$, $-CFH_2$, $-C(=O)-NR^AR^B$, $-C(=O)-NH-NR^CR^D$, $-S(=O)-C_{1-6}$-alkyl, $-S(=O)_2-C_{1-6}$-alkyl, $-S(=O)_2$-phenyl, $-(C_{1-5}$-alkylene)$-S-C_{1-6}$-alkyl, $-(C_{1-5}$-alkylene)$-S(=O)-C_{1-6}$-alkyl, $-(C_{1-5}$-alkylene)$-S(=O)_2-C_{1-6}$-alkyl, $-NR^ER^F$, $-(C_{1-5}$-alkylene)$-NR^ER^F$, $-S(=O)-NH_2$, $-S(=O)_2-NH-C_{1-6}$-alkyl, $-S(=O)_2$-NH-phenyl, $-NH-S(=O)_2-C_{1-6}$-alkyl, cyclopropyl, cyclobutyl, cyclopentyl, cyclohexyl, pyrrolidinyl, piperidinyl, phenyl, thiophenyl, phenoxy and benzyl;

whereby in each case the cyclic moieties cyclopropyl, cyclobutyl, cyclopentyl, cyclohexyl, pyrrolidinyl, piperidinyl, phenyl, thiophenyl, phenoxy and benzyl can optionally be substituted with 1, 2, 3, 4 or 5 substituent(s) independently selected from the group consisting of F, Cl, Br, I, -OH, $-CF_3$, -CN, $-NO_2$, $-C_{1-6}$-alkyl, $-O-C_{1-6}$-alkyl, $-O-CF_3$ and $-S-CF_3$ and whereby $R^A$, $R^B$, $R^E$ and $R^F$, independently of one another, represent hydrogen or $-C_{1-6}$-alkyl or $R^A$ and $R^B$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more identical or different $C_{1-6}$ alkyl radicals

and whereby $R^C$ and $R^D$, independently of one another, represent hydrogen, $-C_{1-6}$-alkyl, $-C(=O)-O-C_{1-6}$-alkyl, $C_{3-8}$-cycloalkyl, $-(C_{1-5}$-alkylene)$-C_{3-8}$-cycloalkyl, $-(C_{1-6}$-alkylene)$-O-C_{1-6}$-alkyl or $-C_{1-6}$-alkyl substituted with one or more hydroxy groups or $R^C$ and $R^D$ in each case together with the bridging nitrogen atom form a radical selected from the group consisting of pyrrolidinyl, imidazolidinyl, piperazinyl, piperidinyl, thiomorpholinyl, morpholinyl, azepanyl and diazepanyl which may be at least mono-substituted with one or more substituents independently selected from the group consisting $-C_{1-6}$-alkyl, $-C(=O)-C_{1-6}$-alkyl, $-C(=O)-O-C_{1-6}$-alkyl, $-C(=O)-NH-C_{1-6}$-alkyl, $-C(=S)-NH-C_{1-6}$-alkyl, oxo (=O), $-C_{1-6}$-alkyl substituted with one or more hydroxy groups, $-(C_{1-6}$-alkylene)$-O-C_{1-6}$-alkyl and —$C(=O)-NH_2$.

More preferably said mono- or polycyclic ring system may optionally be substituted with 1, 2, 3, 4 or 5 substituent (s) independently selected from the group consisting of oxo (=O), thioxo (=S), $-CF_3$, $-C_2F_5$, $-C_3F_7$, $-C_4F_9$, $-CH_2Cl$, $-CHCl_2$, $-C_2H_4Cl$, methyl, ethyl, n-propyl, isopropyl, n-butyl, isobutyl, 2-butyl, tert-butyl, n-pentyl, 2-pentyl, n-hexyl, $-CH_2-OH$, $-CH_2-CH_2-OH$, $-CH_2-CH_2-CH_2-OH$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, -C(=O)-OH, $-C(=O)-O-CH_3$, $-C(=O)-O-C_2H_5$, $-C(=O)-O-C_3H_7$, $-C(=O)-O-C(CH_3)_3$, $-O-C(=O)-CH_3$, $-O-C(=O)-C_2H_5$, $-O-C(=O)-CH(CH_3)_2$, $-O-C(=O)-CH_2-CH_2-CH_3$, $-O-C(=O)-C(CH_3)_3$, F, Cl, Br, I, -CN, $-OCF_3$, $-O-C_2F_5$, $-O-C_3F_7$, $-O-C_4F_9$, $-SCF_3$, $-SCF_2H$, $-SCFH_2$, -OH, -SH, $-SO_3H$, $-NH-C(=O)-CH_3$, $-NH-C(=O)-C_2H_5$, $-NH-C(=O)-C(CH_3)_3$, $-NO_2$, -CHO, $-C(=O)-CH_3$, $-C(=O)-C_2H_5$, $-C(=O)-C(CH_3)_3$, $-C(=O)-CF_3$, $-C(=O)-C_2F_5$, $-C(=O)-C_3F_7$, $-C(=S)-NH-CH_3$, $-C(=S)-NH-C_2H_5$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-C(=O)-NH-NH-CH_3$, $-C(=O)-NH-NH-C_2H_5$, $-C(=O)-NH-NH_2$, $-C(=O)-NH-N(CH_3)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)-C_3H_7$, $-S(=O)_2$-phenyl, $-NH_2$, $-NH-CH_3$, $-NH-C_2H_5$, $-N(CH_3)_2$, $-N(C_2H_5)_2$, $-CH_2-N(CH_3)_2$, —$(CH_2)$-morpholinyl, $-(CH_2)$-piperidinyl, $-(CH_2)$-piperazinyl, $-(CH_2)-N(C_2H_5)_2$, $-CH_2-N(C_3H_7)_2$, $-CH_2-N(C_4H_9)_2$, $-CH_2-N(CH_3)(C_2H_5)$, $-S(=O)-NH_2$, $-S(=O)_2-NH-CH_3$, $-S(=O)_2$-NH-phenyl, $-NH-S(=O)_2-CH_3$, cyclopropyl, cyclobutyl, cyclopentyl, cyclohexyl, pyrrolidinyl, piperidinyl, phenyl, thiophenyl, phenoxy and benzyl, whereby said thiophenyl radical can be substituted with 1, 2 or 3 substituents independently selected from the group consisting of F, Cl, Br, methyl, ethyl and n-propyl.

If one or more of the residues $R^4$ to $R^{22}$ represent or comprise a saturated or unsaturated, unsubstituted or at least mono-substituted aliphatic radical, preferably a $C_{1-16}$ aliphatic radical, said aliphatic radical may be linear or branched.

Preferably said aliphatic radicals, unless defined otherwise, may optionally be substituted with 1, 2, 3, 4, 5, 6, 7, 8 or 9 substituents independently selected from the group consisting of —OH, F, Cl, Br, I, $-O-C_{1-6}$-alkyl, $-OCF_3$, $-O-C_2F_5$, $-O-C_3F_7$, $-O-C_4F_9$, $-CF_3$, $-C_2F_5$, $-C_3F_7$, $-C_4F_9$, $-NH_2$, $-NH-C_{1-6}$-alkyl, $-N(C_{1-6}$-alkyl$)_2$, -C(=O)-OH, $-C(=O)-O-C_{1-6}$-alkyl, $-C(=O)-NH_2$, $-C(=O)-NH-C_{1-6}$-alkyl, $-C(=O)-N(C_{1-6}$-alkyl$)_2$, -CN, $-NO_2$, $-S(=O)-NH_2$, -CHO, $-C(=O)-C_{1-6}$-alkyl, $-S(=O)-C_{1-6}$-alkyl, $-S(=O)_2-C_{1-6}$-alkyl, $-NH-S(=O)-C_{1-6}$-alkyl, $-NH-C(=O)-O-C_{1-6}$-alkyl and —$NH-C(=O)-C_{1-6}$-alkyl.

More preferably said aliphatic radicals may optionally be substituted with 1, 2, 3, 4, 5, 6, 7, 8 or 9 substituents independently selected from the group consisting of —OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, —$N(C_2H_5)_2$, -CN, $-NO_2$, $-NH-C(=O)-CH_3$, $-NH-C(=O)-C_2H_5$, $-NH-C(=O)-C(CH_3)_3$, $-NH-C(=O)-O-CH_3$, $-NH-C(=O)-O-C_2H_5$, $-NH-C(=O)-O-C(CH_3)_3$, $-C(=O)-NH-CH_3$, $-C(=O)-NH-C_2H_5$, $-C(=O)-NH-C(CH_3)_3$, $-C(=O)-N(CH_3)_2$, $-C(=O)-N(C_2H_5)_2$, -C(=O)-OH, $-C(=O)-O-CH_3$, $-C(=O)-O-C_2H_5$, $-C(=O)-O-C(CH_3)_3$, $-C(=O)-CH_3$, $-C(=O)-C_2H_5$ and $-C(=O)-C(CH_3)_3$.

Suitable alkyl radicals, preferably $C_{1-16}$ alkyl radicals, are selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl,

2-(5-methyl)-hexyl, 2-(4-methyl)-hexyl, 2-(7-methyl)-octyl, 2-(6-methyl)-octyl, n-nonyl, n-decyl, n-undecyl, n-dodecyl, n-tridecyl, n-tetradecyl, n-pentadecycl and n-hexadecyl.

Suitable at least mono-substituted alkyl radicals are selected from the group consisting of $-CF_3$, $-CH_2F$, $-CF_2H$, $-CH_2-O-CH_3$, $-C_2F_5$, $-CH_2-CH_2-F$, $-CH_2-CN$, $-CH_2-OH$,- $CH_2-CH_2-CN$, $-CH_2-CH_2-OH$, $-CH_2-CH_2-OCH_3$, $-CH_2-CH_2-CH_2-CN$, $-CH_2-CH_2-CH_2-OH$, $-CH_2-CH_2-CH_2-O-CH_3$, $-CH_2-CH_2-CH_2-CH_2-O-CH_3$, $-CH_2-NH_2$, $-CH_2-N(CH_3)_2$, $-CH_2-N(C_2H_5)_2$, $-CH_2-CH-NH_2$, $-CH_2-CH_2-N(CH_3)_2$, $-CH_2-CH_2-N(C_2H_5)_2$, $-CH_2-CH_2-CH_2-NH_2$, $-CH_2-CH_2-CH_2-N(CH_3)_2$ and $—CH_2-CH_2-CH_2-N(C_2H_5)_2$.

An alkenyl radical according to the present invention comprises at least one carbon-carbon double bond. Suitable alkenyl radicals, preferably $C_{2-16}$ alkenyl radicals, are selected from the group consisting of vinyl, n-propenyl, n-butenyl, n-pentenyl, n-hexenyl, n-heptenyl, n-octenyl, n-nonenyl, n-decenyl, n-undecenyl, n-dodecenyl, n-tridecenyl, n-tetradecenyl, n-pentadecenyl and n-hexadecenyl.

An alkinyl radical comprises at least one carbon-carbon triple bond. Suitable alkinyl radicals, preferably $C_{2-16}$ alkinyl radicals, are selected from the group consisting of ethinyl, propinyl, n-butinyl, n-pentinyl, n-hexinyl, n-octinyl, n-noninyl, n-decinyl, n-undecinyl, n-dodecinyl, n-tridecinyl, n-tetradecinyl, n-pentadecinyl and n-hexadecinyl.

If any of the substituents represents an alkylene group, an alkenylene group or an alkinylene group, which may be substituted, said alkylene group, alkenylene group or alkinylene group may - if not defined otherwise - be unsubstituted or substituted by one or more substituents, preferably unsubstituted or substituted with 1, 2 or 3 substituent(s). Said substituent(s) may preferably be selected independently from the group consisting of $-O-C_{1-6}$-alkyl, $-S-C_{1-6}$-alkyl, -F, Cl, Br, I, -CN, $-CF_3$, $-OCF_3$, $-SCF_3$, -OH, -SH, $-SO_3H$, $-NH_2$, $-NH(C_{1-6}$-alkyl), $-N(C_{1-6}$-alkyl$)_2$ and phenyl. More preferably said substituent(s) may be selected from the group consisting of -F, Cl, Br, I, -CN, $-CF_3$, $-OCF_3$, $-SCF_3$, -OH, -SH, $-SO_3H$, $-NH_2$, $-NH-CH_3$, $-N(CH_3)_2$, $-O-CH_3$ and $—O-C_2H_5$. An alkenylene group comprises at least one carbon-carbon double bond, an alkinylene group comprises at least one carbon-carbon triple bond.

Suitable alkylene groups, preferably $C_{1-5}$-alkylene groups, include $—(CH_2)-$, $-CH(CH_3)-$, -CH(phenyl), $—(CH_2)_2-$, $—(CH_2)_3-$, $—(CH_2)_4-$, $—(CH_2)_5$ and $—(CH_2)_6-$, suitable alkenylene groups, preferably $C_{2-5}$-alkenylene groups, include $—CH=CH-$, $—CH_2-CH=CH-$ and $-CH=CH-CH_2-$ and suitable alkinylene groups, preferably $C_{2-5}$-alkinylene groups, include $-C≡C-$, $-CH_2-C≡C-$ and $-C≡C-CH_2-$.

Preferred are 4-substituted pyrazoline compounds of general formula I given above, wherein

X is O or S;

$R^1$ and $R^2$, independently of one another, in each case represent an unsubstituted or at least mono-substituted 6- or 10-membered aryl radical, which may be condensed with an unsubstituted or at least mono-substituted mono- or polycyclic ring system;

an unsubstituted or at least mono-substituted 5- to 14-membered heteroaryl radical, which may be condensed with an unsubstituted or at least mono-substituted mono- or polycyclic ring system;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;

or an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;

$R^3$ represents an unsubstituted or at least mono-substituted 6- or 10-membered aryl radical, which may be condensed with an unsubstituted or at least mono-substituted mono- or polycyclic ring system;

an unsubstituted or at least mono-substituted 5- to 14-membered heteroaryl radical, which may be condensed with an unsubstituted or at least mono-substituted mono- or polycyclic ring system;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;

an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an unsubstituted or at least mono-substituted spirocyclic residue via a common ring atom;

a $-O-R^6$ moiety; a $—NR^7R^8$ moiety or a $-NR^9-O-R^{10}$ moiety;

$R^4$ represents F; Cl; Br; I; -CN; $-NO_2$; -NC; -OH; $-NH_2$; -SH; -C(=O)-H;

an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be

bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
a —O-$R^{11}$ moiety; a -S-$R^{12}$ moiety; a —NH-$R^{13}$ moiety or a —$NR^{14}R^{15}$ moiety;
$R^5$ represents H; F; Cl; Br; I; -CN; -$NO_2$; -NC; -OH; -$NH_2$; -SH; -C(=O)-H;
an unsubstituted or at least mono-substituted $C_{1-16}$ alkyl radical, $C_{2-16}$ alkenyl radical or $C_{2-16}$ alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
—O-$R^{11}$ moiety; a —S-$R^{12}$ moiety; a —NH-$R^{13}$ moiety or a —$NR^{14}R^{15}$ moiety;
$R^6$ represents a hydrogen atom;
an unsubstituted or at least mono-substituted $C_{1-16}$ alkyl radical, $C_{2-16}$ alkenyl radical or $C_{2-16}$ alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
a —P(=O)($OR^{16})_2$ moiety; a —C(=O)-$OR^{17}$ moiety; a —C(=O)-NH-$R^{18}$ moiety or a —C(=O)-$R^{19}$ moiety;
$R^7$ and $R^8$, independently of one another, in each case represent a hydrogen atom;
an unsubstituted or at least mono-substituted $C_{1-16}$ alkyl radical, $C_{2-16}$ alkenyl radical or $C_{2-16}$ alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system

and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an unsubstituted or at least mono-substituted spirocyclic residue via a common ring atom;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

a —$P(=O)(OR^{16})_2$ moiety; a —$C(=O)$-$OR^{17}$ moiety; a —$C(=O)$-NH-$R^{18}$ moiety; a —$C(=O)$-$R^{19}$ moiety; a —$S(=O)_2$-$R^{20}$ moiety; or a —$NR^{21}R^{22}$ moiety;

$R^9$ represents a hydrogen atom or an unsubstituted or at least mono-substituted $C_{1-16}$ alkyl radical, $C_{2-16}$ alkenyl radical or $C_{2-16}$ alkinyl radical;

$R^{10}$, $R^{11}$, $R^{12}$, $R^{13}$, $R^{14}$, $R^{15}$ and $R^{20}$, independently of one another, in each case represent an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group; an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

$R^{16}$, $R^{17}$, $R^{18}$ and $R^{19}$, independently of one another, in each case represent an unsubstituted or at least mono-substituted $C_{1-16}$ alkyl radical, $C_{2-16}$ alkenyl radical or $C_{2-16}$ alkinyl radical;

or an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

and

$R^{21}$ and $R^{22}$, independently of one another, in each case represent a hydrogen atom; an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an unsubstituted or at least mono-substituted spirocyclic residue via a common ring atom;

an unsubstituted or at least mono-substituted 6- or 10-membered aryl radical, which may be condensed with an unsubstituted or at least mono-substituted mono- or polycyclic ring system and/or may be bonded via an unsubstituted or at

least mono-substituted $C_{1-5}$ alkylene group, $C_{2-5}$ alkenylene group or $C_{2-5}$ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
whereby
the rings of the aforementioned ring system are in each case independently of one another 5- 6- or 7-membered and may in each case independently of one another optionally contain 1, 2 or 3 heteroatom(s) independently selected from the group consisting of nitrogen, oxygen and sulfur;
the aforementioned heteroaryl radicals in each case optionally contain 1, 2, 3 or 4 heteroatom(s) independently selected from the group consisting of nitrogen, oxygen and sulfur as ring member(s);
the aforementioned heterocycloalkyl radicals and heterocycloalkenyl radicals in each case optionally contain 1, 2, 3 or 4 heteroatom(s) independently selected from the group consisting of nitrogen, oxygen and sulfur as ring member(s);
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

Also preferred are 4-substituted pyrazoline compounds of general formula I given above, wherein $R^1$ and $R^2$, independently of one another, in each case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl, imidazo[2,1-b]thiazolyl, 2H-chromenyl, pyranyl, indazolyl, quinazolinyl, [1,3]-benzodioxolyl, [1,4]-benzodioxanyl, [1,2,3,4]-tetrahydronaphthyl, (2,3)-dihydro-1H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl, which in each case is optionally bonded to the pyrazoline compound of general formula I via the aromatic or heteroaromatic part of the aforementioned radicals and may optionally be substituted with 1, 2, 3, 4 or 5 substituent(s) independently selected from the group consisting of $-CF_3$, $-CH_2-Cl$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, phenoxy and thiophenyl, whereby the thiophenyl radical can be substituted with 1, 2 or 3 substituents independently selected from the group consisting of F, Cl, Br, methyl, ethyl and n-propyl;
or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyn-olyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, (1,2,3,4)-tetrahydroquinoxazlinyl, adamantyl, bicyclo[2.2.1]heptyl, bicyclo[3.1.1]heptyl and norbornenyl, which in each case is optionally bonded to the pyrazoline compound of general formula I via the (hetero)cycloaliphatic part of the aforementioned radicals and may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-0-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;
and X and $R^3$ to $R^{22}$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Preference is also given to 4-substituted pyrazoline compounds of general formula I given above, wherein $R^3$ represents a radical selected from the group consisting of (2,3)-dihydro-1 H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyddinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl, (1,2,3,4)-tetrahydroquinoxazlinyl, adamantyl, bicyclo[2.2.1]heptyl, bicyclo[3.1.1]heptyl, norbornenyl, 8-aza-bicyclo[3.2.1]octyl and 8-aza-spiro[4.5]decanyl, which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C(=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;
a $-O-R^6$ moiety; a $-NR^7R^8$ moiety or a $-NR^9-O-R^{10}$ moiety;
and X, $R^1$, $R^2$ and $R^4$ to $R^{22}$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are 4-substituted pyrazoline compounds of general formula I given above, wherein $R^4$ represents F; Cl; Br; I; -CN; $-NO_2$; -NC; -OH; $-NH_2$; -SH; -C(=O)-H;
a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4, 5, 6, 7, 8 or 9 substituent(s) independently selected from the group consisting of -OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$,- $NH-CH_3$, $-NH-C_2H_5$, $-N(CH_3)_2$, $-N(C_2H_5)_2$, -CN and $-NO_2$;
a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or $-CH=CH-$group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;
a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;
a $-O-R^{11}$ moiety; a $-S-R^{12}$ moiety, a $-NH-R^{13}$ moiety or a $-NR^{14}R^{15}$ moiety;
and X, $R^1$, $R^2$, $R^3$ and $R^5$ to $R^{22}$ have the meaning given above, optionally in form of one of the stereoisomers, preferably

enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Preference is also given to 4-substituted pyrazoline compounds of general formula I, wherein

$R^5$ represents H, F; Cl; Br; I; -CN; -$NO_2$; -NC; -OH; -$NH_2$; -SH; -C(=O)-H;

a radical selected from the group consisting of methyl, -$CF_3$, -$CH_2F$, -$CF_2H$, -$C_2F_5$, ethyl, -$CH_2$-CN, -$CH_2$-OH, n-propyl, isopropyl, -$CH_2$-$CH_2$-CN, -$CH_2$-$CH_2$-OH, n-butyl, -$CH_2$-$CH_2$-$CH_2$-CN, -$CH_2$-$CH_2$-$CH_2$-OH, isobutyl, sec-butyl, tert-butyl, n-pentyl, 2-pentyl, 3-pentyl, neo-pentyl, n-hexyl, 2-hexyl and 3-hexyl;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl,

a radical selected from the group consisting of phenyl, naphthyl, pyridinyl, furyl (furanyl), thienyl (thiophenyl) and pyrrolyl, which may be bonded via a —($CH_2$)-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, F, Cl and Br;

a —O-$R^{11}$ moiety; a —S-$R^{12}$ moiety, a —NH-$R^{13}$ moiety or a —$NR^{14}R^{15}$ moiety;

and X, $R^1$ to $R^4$ and $R^6$ to $R^{22}$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are 4-substituted pyrazoline compounds of general formula I given above, wherein $R^6$ represents a hydrogen atom; a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -$NH_2$, -NH-$CH_3$, -NH-$C_2H_5$, -$N(CH_3)_2$, -$N(C_2H_5)_2$, -CN, -NO, -NH-C(=O)-$CH_3$, -NH-C(=O)-$C_2H_5$, -NH-C(=O)-$C(CH_3)_3$, -NH-C(=O)-O-$CH_3$, -NH-C(=O)-O-$C_2H_5$, -NH-C(=O)-O-$C(CH_3)_3$, -C(=O)-NH-$CH_3$, -C(=O)-NH-$C_2H_5$, -C(=O)-NH-$C(CH_3)_3$, -C(=O)-$N(CH_3)_2$, -C(=O)-$N(C_2H_5)_2$, -C(=O)-OH, -C(=O)-O-$CH_3$, -C(=O)-O-$C_2H_5$, -C(=O)-O-$C(CH_3)_3$, -C(=O)-$CH_3$, -C(=O)-$C_2H_5$ and -C(=O)-$C(CH_3)_3$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-azabicyclo[3.2.1]octyl and diazepanyl, which may be bonded via a —($CH_2$)-, -($CH_2$)-($CH_2$)-, -($CH_2$)-($CH_2$)-($CH_2$)- or -CH=CH-group and/or may optionally be substituted with 1, 2, 3, 4 or 5 substituent (s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -0-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$,- $S(=O)_2$-$C_2H_5$, -$S(=O)_2$-$C_3H_7$, -$NH_2$, -NH-$CH_3$, -NH-$C_2H_5$, -$N(CH_3)_2$ and —$N(C_2H_5)_2$;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$, -$N(CH_3)_2$, —$N(C_2H_5)_2$, -O-C(=O)-$CH_3$, -O-C(=O)-$C_2H_5$, -O-C(=O)-$CH(CH_3)_2$, -O-C(=O)-$CH_2$-$CH_2$-$CH_3$, -$CH_2$-$N(CH_3)_2$, -($CH_2$)-$N(C_2H_5)_2$, -$CH_2$-$N(C_3H_7)_2$, -$CH_2$-$N(C_4H_9)_2$, -$CH_2$-$N(CH_3)(C_2H_5)$ and —($CH_2$)-morpholinyl;

a —$P(=O)(OR^{16})_2$ moiety; a —C(=O)-$OR^{17}$ moiety; a —C(=O)-NH-$R^{18}$ moiety or a —C(=O)-$R^{19}$ moiety;

and X, $R^1$ to $R^5$ and $R^7$ to $R^{22}$ have the meaning given above, optionally in form of one of the stereoisomers, preferably enantiomers or diastereomers, a racemate or in form of a mixture of at least two of the stereoisomers, preferably

enantiomers and/or diastereomers, in any mixing ratio, or a corresponding N-oxide thereof, or a corresponding salt thereof, or a corresponding solvate thereof.

Preference is also given to 4-substituted pyrazoline compounds of general formula I given above, wherein $R^7$ and $R^8$, independently of another, in each case represent a hydrogen atom;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $—N(C_2H_5)_2$, -CN and $-NO_2$;

a radical selected from the group consisting of (1,2,3,4)-tetrahydronaphthyl, (2,3)-dihydro-1H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydrocyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl, (1,2,3,4)-tetrahydroquinoxazlinyl, adamantyl, bicyclo[2.2.1]heptyl, bicyclo[3.1.1]heptyl, norbomenyl, 8-aza-bicyclo[3.2.1]octyl and 8-aza-spiro[4.5]decanyl, which may be bonded via a $—(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C(=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $—(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of $—CF_3$, methyl, ethyl, n-propyl, isopropyl, n-butyl, isobutyl, 2-butyl, tert-butyl, n-pentyl, 2-pentyl, n-hexyl, $-O-CH_3$, $-0-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$;

a $—P(=O)(OR^{16})_2$ moiety; a $—C(=O)-OR^{17}$ moiety; a $—C(=O)-NH-R^{18}$ moiety; a $-C(=O)-R^{19}$ moiety; a $—S(=O)_2-R^{20}$ moiety; or a $—NR^{21}R^{22}$ moiety;

and X, $R^1$ to $R^6$ and $R^9$ to $R^{22}$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are 4-substituted pyrazoline compounds of general formula I given above, wherein $R^9$ represents hydrogen or an alkyl radical selected from the group consisting of methyl, ethyl and n-propyl;

and X, $R^1$ to $R^8$ and $R^{10}$ to $R^{22}$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Preference is also given to 4-substituted pyrazoline compounds of general formula I, wherein $R^{10}$, $R^{11}$, $R^{12}$, $R^{13}$, $R^{14}$, $R^{15}$ and $R^{20}$, independently of another, in each case represent a radical selected from the group consisting

of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of -OH, F, Cl, Br, I, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -$NH_2$, -NH-$CH_3$, -NH-$C_2H_5$, -$N(CH_3)_2$, —N$(C_2H_5)_2$, -CN and -$NO_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-aza-bicyclo[3.2.1]octyl and diazepanyl, which may be bonded via a —$(CH_2)$-, -$(CH_2)$-$(CH_2)$-, -$(CH_2)$-$(CH_2)$-$(CH_2)$- or —CH=CH-group and/or may optionally be substituted with 1, 2, 3, 4 or 5 substituent (s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$, -N$(CH_3)_2$ and —$N(C_2H_5)_2$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a -$(CH_2)$-, -$(CH_2)$-$(CH_2)$-, -$(CH_2)$-$(CH_2)$-$(CH_2)$- or -CH=CH-group and/or may optionally be substituted with 1, 2, 3, 4 or 5 substituent(s) independently selected from the group consisting of -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C (=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -S $(=O)_2$-$C_3H_7$, -$NH_2$, -NH-$CH_3$, -NH-$C_2H_5$, -$N(CH_3)_2$ and -$N(C_2H_5)_2$;

and X, $R^1$ to $R^9$, $R^{16}$ to $R^{19}$, $R^{21}$ and $R^{22}$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are 4-substituted pyrazoline compounds of general formula I given above, wherein $R^{16}$, $R^{17}$, $R^{18}$ and $R^{19}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of $NH_2$, -NH-$CH_3$, -NH-$C_2H_5$, -N $(CH_3)_2$, —$N(C_2H_5)_2$, -NH-C(=O)-$CH_3$, -NH-C(=O)-$C_2H_5$, -NH-C(=O)-$C(CH_3)_3$, -NH-C(=O)-O-$CH_3$, -NH-C(=O)-O-$C_2H_5$, -NH-C(=O)-O-$C(CH_3)_3$, -C(=O)-NH-$CH_3$, -C(=O)-NH-$C_2H_5$, -C(=O)-NH-$C(CH_3)_3$, -C(=O)-$N(CH_3)_2$, -C(=O)-$N(C_2H_5)_2$, -OH, -C(=O)-OH, -C(=O)-O-$CH_3$, -C(=O)-O-$C_2H_5$, -C(=O)-O-$C(CH_3)_3$, -C(=O)-$CH_3$, -C(=O)-$C_2H_5$ and -C(=O)-$C(CH_3)_3$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —$(CH_2)$-, -$(CH_2)$-$(CH_2)$-, -$(CH_2)$-$(CH_2)$-$(CH_2)$- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C (=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -S $(=O)_2$-$C_2H_5$, -$S(=O)_2$-$C_3H_7$, -$NH_2$, -NH-$CH_3$, -NH-$C_2H_5$, -$N(CH_3)_2$, —$N(C_2H_5)_2$, -O-C(=O)-$CH_3$, -O-C(=O)-$C_2H_5$, -O-C (=O)-$CH(CH_3)_2$, -O-C(=O)-$CH_2$-$CH_2$-$CH_3$, -$CH_2$-$N(CH_3)_2$, -$(CH_2)$-$N(C_2H_5)_2$, -$CH_2$-$N(C_3H_7)_2$, -$CH_2$-$N(C_4H_9)_2$, -$CH_2$-N $(CH_3)(C_2H_5)$ and —$(CH_2)$-morpholinyl;

and X, $R^1$ to $R^{15}$ and $R^{20}$ to $R^{22}$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are 4-substituted pyrazoline compounds of general formula I given above, wherein $R^{21}$ and $R^{22}$, independently of another, in each case represent hydrogen;
a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of -OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $-N(C_2H_5)_2$, -CN and $-NO_2$;
a radical selected from the group consisting of (2,3)-dihydro-1 H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, (1,2,3,4)-tetrahydroquinoxazlinyl, adamantyl, bicyclo[2.2.1]heptyl, bicyclo [3.1.1]heptyl, norbomenyl, 8-aza-bicyclo[3.2.1]octyl and 8-aza-spiro[4.5]decanyl, which may be bonded via a $—(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent (s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C(=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $—N(C_2H_5)_2$, cyclopentyl, cyclohexyl, pyrrolidinyl and piperidinyl;
or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $—(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of $—CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;
and X and $R^1$ to $R^{20}$ have the meaning given above, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof.

Also preferred are 4-substituted pyrazoline compounds of general formula I given above, wherein
X is S or O;
$R^1$ and $R^2$, independently of one another, in each case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl, imidazo[2,1-b]thiazolyl, 2H-chromenyl, pyranyl, indazolyl, quinazolinyl, [1,3]-benzodioxolyl, [1,4]-benzodioxanyl, [1,2,3,4]-tetrahydronaphthyl, (2,3)-dihydro-1H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl, which in each case is optionally bonded to the pyrazoline compound of general formula I via the aromatic or heteroaromatic part of the aforementioned radicals and may optionally be substituted with 1, 2, 3, 4 or 5 substituent(s) independently selected from the group consisting of $—CF_3$, -CH, -Cl, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, -O-

$CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, phenoxy and thiophenyl, whereby the thiophenyl radical can be substituted with 1, 2 or 3 substituents independently selected from the group consisting of F, Cl, Br, methyl, ethyl and n-propyl;

or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyddinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydrocyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl, (1,2,3,4)-tetrahydroquinoxazlinyl, adamantyl, bicyclo[2.2.1]heptyl, bicyclo[3.1.1]heptyl and norbomenyl, which in each case is optionally bonded to the pyrazoline compound of general formula I via the (hetero)cycloaliphatic part of the aforementioned radicals and may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

$R^3$ represents a radical selected from the group consisting of (2,3)-dihydro-1 H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyddinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl, (1 ,2,3,4)-tetrahydroquinoxazlinyl, adamantyl, bicyclo[2.2.1]heptyl, bicyclo[3.1.1]heptyl, norbomenyl, 8-aza-bicyclo[3.2.1]octyl and 8-aza-spiro[4.5]decanyl, which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-C(=O)-OH$, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;

a $-O-R^6$ moiety; a $-NR^7R^8$ moiety or a $-NR^9-O-R^{10}$ moiety;

$R^4$ represents F; Cl; Br; I; -CN; $-NO_2$; -NC; -OH; $-NH_2$; -SH; -C(=O)-H;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4, 5, 6, 7, 8 or 9 substituent(s) independently selected from the group consisting of -OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $-N(C_2H_5)_2$, -CN and $-NO_2$;

a radical selected from the group consisting of phenyl, naphthyl, pyridinyl, furyl (furanyl), thienyl (thiophenyl), pyrrolyl,

oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —$(CH_2)$-, -$(CH_2)$-$(CH_2)$-, -$(CH_2)$-$(CH_2)$-$(CH_2)$- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$, -$N(CH_3)_2$ and —$N(C_2H_5)_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)--$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$, -$N(CH_3)_2$ and -$N(C_2H_5)_2$;

a —O-$R^{11}$ moiety; a -S-$R^{12}$ moiety, a -NH-$R^{13}$ moiety or a —$NR^{14}R^{15}$ moiety;

$R^5$ represents H, F; Cl; Br; I; -CN; -$NO_2$; -NC; -OH; -$NH_2$; -SH; -C(=O)-H;

a radical selected from the group consisting of methyl, -$CF_3$, -$CH_2F$, -$CF_2H$, -$C_2F_5$, ethyl, -$CH_2$-CN, -$CH_2$-OH, n-propyl, isopropyl, -$CH_2$-$CH_2$-CN, -$CH_2$-$CH_2$-OH, n-butyl, -$CH_2$-$CH_2$-$CH_2$-CN, -$CH_2$-$CH_2$-$CH_2$-OH, isobutyl, sec-butyl, tert-butyl, n-pentyl, 2-pentyl, 3-pentyl, neo-pentyl, n-hexyl, 2-hexyl and 3-hexyl;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl;

a radical selected from the group consisting of phenyl, naphthyl, pyridinyl, furyl (furanyl), thienyl (thiophenyl) and pyrrolyl, which may be bonded via a —$(CH_2)$-group and/or may optionally be substituted with 1, 2, 3, 4 or 5 substituent(s) independently selected from the group consisting of -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, F, Cl and Br;

a —O-$R^{11}$ moiety; a -S-$R^{12}$ moiety, a -NH-$R^{13}$ moiety or a —$NR^{14}R^{15}$ moiety;

$R^6$ represents a hydrogen atom; a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -$NH_2$, -NH-$CH_3$, -NH-$C_2H_5$, -$N(CH_3)_2$,— $N(C_2H_5)_2$, -CN, -NO, -NH-C(=O)-$CH_3$, -NH-C(=O)-$C_2H_5$, -NH-C(=O)-$C(CH_3)_3$, -NH-C(=O)-O-$CH_3$, -NH-C(=O)-O-$C_2H_5$, -NH-C(=O)-O-$C(CH_3)_3$, -C(=O)-NH-$CH_3$, -C(=O)-NH-$C_2H_5$, -C(=O)-NH-$C(CH_3)_3$, -C(=O)-$N(CH_3)_2$, -C(=O)-$N(C_2H_5)_2$, -C(=O)-OH, -C(=O)-O-$CH_3$, -C(=O)-O-$C_2H_5$, -C(=O)-O-$C(CH_3)_3$, -C(=O)-$CH_3$, -C(=O)-$C_2H_5$ and -C(=O)-$C(CH_3)_3$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-aza-bicyclo[3.2.1]octyl and diazepanyl, which may be bonded via a —$(CH_2)$-, -$(CH_2)$-$(CH_2)$-, -$(CH_2)$-$(CH_2)$-$(CH_2)$- or —CH=CH-group and/or may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$, -$N(CH_3)_2$ and —$N(C_2H_5)_2$;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a -$(CH_2)$-, -$(CH_2)$-$(CH_2)$-, -$(CH_2)$-$(CH_2)$-$(CH_2)$- or -CH=CH-group and/or may

optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$,- $C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, $-O-C(=O)-CH_3$, $-O-C(=O)-C_2H_5$, $-O-C(=O)-CH(CH_3)_2$, $-O-C(=O)-CH_2-CH_2-CH_3$, $-CH_2-N(CH_3)_2$, $-(CH_2)-N(C_2H_5)_2$, $-CH_2-N(C_3H_7)_2$, $-CH_2-N(C_4H_9)_2$, $-CH_2-N(CH_3)(C_2H_5)$ and $-(CH_2)$-morpholinyl;

a $-P(=O)(OR^{16})_2$ moiety; a $-C(=O)-OR^{17}$ moiety; a $-C(=O)-NH-R^{18}$ moiety or a $-C(=O)-R^{19}$ moiety;

$R^7$ and $R^8$, independently of another, in each case represent a hydrogen atom;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of -OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$,$-N(C_2H_5)_2$, -CN and $-NO_2$;

a radical selected from the group consisting of (1,2,3,4)-tetrahydronaphthyl, (2,3)-dihydro-1 H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyddinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydrocyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, (1,2,3,4)-tetrahydroquinoxazlinyl, adamantyl, bicyclo[2.2.1]heptyl, bicyclo[3.1.1]heptyl, norbomenyl, 8-aza-bicyclo[3.2.1]octyl and 8-aza-spiro[4.5]decanyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C(=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH--C_2H_5$,- $C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

a $-P(=O)(OR^{16})_2$ moiety; a $-C(=O)-OR^{17}$ moiety; a $-C(=O)-NH-R^{18}$ moiety; a $-C(=O)-R^{19}$ moiety; a $-S(=O)_2-R^{20}$ moiety; or a $-NR^{21}R^{22}$ moiety;

$R^9$ represents hydrogen or an alkyl radical selected from the group consisting of methyl, ethyl and n-propyl;

$R^{10}$, $R^{11}$, $R^{12}$, $R^{13}$, $R^{14}$, $R^{15}$ and $R^{20}$, independently of another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl,

n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of -OH, F, Cl, Br, I, -O-$CH_3$, -O-$C_2H_5$, -O-$CH_2$-$CH_2$-$CH_3$, -O-CH$(CH_3)_2$, -O-$CH_2$-$CH_2$-$CH_2$-$CH_3$, -O-C$(CH_3)_3$, -$NH_2$, -NH-$CH_3$, -NH-$C_2H_5$, -N$(CH_3)_2$, -N$(C_2H_5)_2$, -CN and -$NO_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-azabicyclo[3.2.1]octyl and diazepanyl, which may be bonded via a —$(CH_2)$-, -$(CH_2)$-$(CH_2)$-, -$(CH_2)$-$(CH_2)$-$(CH_2)$- or —CH=CH-group and/or may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH$(CH_3)_2$, -O-$CH_2$-$CH_2$-$CH_2$-$CH_3$, -O-C$(CH_3)_3$, -S-$CH_3$, -S-$C_2H_5$, -S-$CH_2$-$CH_2$-$CH_3$, -S-CH$(CH_3)_2$, -S-$CH_2$-$CH_2$-$CH_2$-$CH_3$, -S-C$(CH_3)_3$, F, Cl, Br, I, -CN, -$OCF_3$, -$SCF_3$, $SCF_2H$, -$SCFH_2$, -OH, -SH, -$NO_2$, -CHO, -C(=O)-$CH_3$, -C(=O)-$C_2H_5$, -C(=O)-C$(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N$(CH_3)_2$, -C(=O)-N$(C_2H_5)_2$, -S(=O)-$CH_3$, -S(=O)-$C_2H_5$, -S(=O)-$C_3H_7$, -S$(=O)_2$-$CH_3$, -S$(=O)_2$-$C_2H_5$, -S$(=O)_2$-$C_3H_7$, -$NH_2$, -NH-$CH_3$, -NH-$C_2H_5$, -N$(CH_3)_2$ and -N$(C_2H_5)_2$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —$(CH_2)$-, -$(CH_2)$-$(CH_2)$-, -$(CH_2)$-$(CH_2)$-$(CH_2)$- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH$(CH_3)_2$, -O-$CH_2$-$CH_2$-$CH_2$-$CH_3$, -O-C$(CH_3)_3$, -S-$CH_3$, -S-$C_2H_5$, -S-$CH_2$-$CH_2$-$CH_3$, -S-CH$(CH_3)_2$, -S-$CH_2$-$CH_2$-$CH_2$-$CH_3$, -S-C$(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-C$(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N$(CH_3)_2$, -C(=O)-N$(C_2H_5)_2$, -S(=O)-$CH_3$, -S(=O)-$C_2H_5$, -S(=O)-$C_3H_7$, -S$(=O)_2$-$CH_3$, -S$(=O)_2$-$C_2H_5$, -S$(=O)_2$-$C_3H_7$, -$NH_2$, -NH-$CH_3$, -NH-$C_2H_5$, -N$(CH_3)_2$ and —N$(C_2H_5)_2$;

$R^{16}$, $R^{17}$, $R^{18}$ and $R^{19}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of $NH_2$, -NH-$CH_3$, -NH-$C_2H_5$, -N$(CH_3)_2$, —N$(C_2H_5)_2$, -NH-C(=O)-$CH_3$, -NH-C(=O)-$C_2H_5$, -NH-C(=O)-C$(CH_3)_3$, -NH-C(=O)-O-$CH_3$, -NH-C(=O)-O-$C_2H_5$, -NH-C(=O)-O-C$(CH_3)_3$, -C(=O)-NH-$CH_3$, -C(=O)-NH-$C_2H_5$, -C(=O)-NH-C$(CH_3)_3$, -C(=O)-N$(CH_3)_2$, -C(=O)-N$(C_2H_5)_2$, -OH, -C(=O)-OH, -C(=O)-O-$CH_3$, -C(=O)-O-$C_2H_5$, -C(=O)-O-C$(CH_3)_3$, -C(=O)-$CH_3$, -C(=O)-$C_2H_5$ and -C(=O)-C$(CH_3)_3$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —$(CH_2)$-, -$(CH_2)$-$(CH_2)$-, -$(CH_2)$-$(CH_2)$-$(CH_2)$- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH$(CH_3)_2$, -O-$CH_2$-$CH_2$-$CH_2$-$CH_3$, -O-C$(CH_3)_3$, -S-$CH_3$, -S-$C_2H_5$, -S-$CH_2$-$CH_2$-$CH_3$, -S-CH$(CH_3)_2$, -S-$CH_2$-$CH_2$-$CH_2$-$CH_3$, -S-C$(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-C$(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N$(CH_3)_2$, -C(=O)-N$(C_2H_5)_2$, -S(=O)-$CH_3$, -S(=O)-$C_2H_5$, -S(=O)-$C_3H_7$, -S$(=O)_2$-$CH_3$, -S$(=O)_2$-$C_2H_5$, -S$(=O)_2$-$C_3H_7$, -$NH_2$, -NH-$CH_3$, -NH-$C_2H_5$, -N$(CH_3)_2$, -N$(C_2H_5)_2$, -O-C(=O)-$CH_3$, -O-C(=O)-$C_2H_5$, -O-C(=O)-CH$(CH_3)_2$, -O-C(=O)-$CH_2$-$CH_2$-$CH_3$, -$CH_2$-N$(CH_3)_2$, -$(CH_2)$-N$(C_2H_5)_2$, -$CH_2$-N$(C_3H_7)_2$, -$CH_2$-N$(C_4H_9)_2$, -$CH_2$-N$(CH_3)(C_2H_5)$ and -$(CH_2)$-morpholinyl;

and

$R^{21}$ and $R^{22}$, independently of another, in each case represent hydrogen; a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, -O-$CH_3$, -O-$C_2H_5$, -O-$CH_2$-$CH_2$-$CH_3$, -O-CH$(CH_3)_2$, -O-$CH_2$-$CH_2$-$CH_2$-$CH_3$, -O-C$(CH_3)_3$, -$NH_2$, -NH-$CH_3$, -NH-$C_2H_5$, -N$(CH_3)_2$, -N$(C_2H_5)_2$, -CN and -$NO_2$;

a radical selected from the group consisting of (2,3)-dihydro-1 H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl, (1 ,2,3,4)-tetrahydroquinoxazlinyl, adamantyl, bicyclo[2.2.1]heptyl, bicyclo [3.1.1]heptyl, norbomenyl, 8-aza-bicyclo[3.2.1]octyl and 8-aza-spiro[4.5]decanyl, which may be bonded via a —$(CH_2)$-, -$(CH_2)$-$(CH_2)$-, -$(CH_2)$-$(CH_2)$-$(CH_2)$- or —CH=CH-group and/or may optionally be substituted with 1, 2, 3, 4 or 5 substituent (s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -O-$CH_2$-O-$CH_3$, -O-$CH_2$-$CH_2$-O-$CH_3$, -O-$CH_2$-O-$C_2H_5$, -$C(OCH_3)(C_2H_5)_2$, -$C(OCH_3)(CH_3)_2$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -C(=O)-OH, -C(=O)-O-$CH_3$, -C(=O)-O-$C_2H_5$, -C(=O)-O-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$, -$N(CH_3)_2$, -$N(C_2H_5)_2$, cyclopentyl, cyclohexyl, pyrrolidinyl and piperidinyl;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —$(CH_2)$-, -$(CH_2)$-$(CH_2)$-, -$(CH_2)$-$(CH_2)$-$(CH_2)$- or- CH=CH-group and/or may optionally be substituted with 1, 2, 3, 4 or 5 substituent(s) independently selected from the group consisting of -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$, -$N(CH_3)_2$ and -$N(C_2H_5)_2$;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

Particularly preferred are 4-substituted pyrazoline compounds of general formula I given above, wherein

X is O or S;

$R^1$ represents a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3] thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of —$CF_3$, -$CH_2$-Cl, methyl, ethyl, n-propyl, isopropyl, n-butyl, isobutyl, 2-butyl, tert-butyl, n-pentyl, 2-pentyl, n-hexyl, -$NO_2$, F, Cl, Br, I, -CN, -$OCF_3$, -$SCF_3$, -$SCF_2H$, —$SCFH_2$, phenoxy and thiophenyl, whereby the thiophenyl radical can be substituted with 1, 2 or 3 substituents independently selected from the group consisting of F, Cl, Br, methyl, ethyl and n-propyl;

or a radical selected from the group consisting of cyclopropyl, cyclobutyl, cyclopentyl, cyclohexyl, cycloheptyl, cyclooctyl, cyclononyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, F, Cl, Br, I, -CN, -$OCF_3$, -$SCF_3$, -$SCF_2H$ and -$SCFH_2$;

$R^2$ represents a radical selected from the group consisting of phenyl, naphthyl, pyridinyl, furyl (furanyl), thienyl (thiophenyl); pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3] thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, F, Cl, Br, I, -CN, -$OCF_3$, -$SCF_3$, -$SCF_2H$ and —$SCFH_2$;

or a radical selected from the group consisting of cyclopropyl, cyclobutyl, cyclopentyl, cyclohexyl, cycloheptyl, cyclooctyl, cyclononyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, F, Cl, Br, I, -CN, $-OCF_3$, $-SCF_3$, $-SCF_2H$ and $—SCFH_2$;

$R^3$ represents a radical selected from the group consisting of

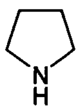 , 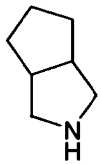 , 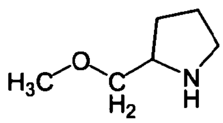 , 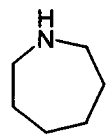 , 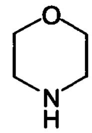 , 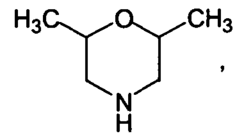 ,

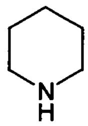 , 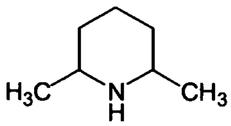 , 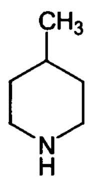 , 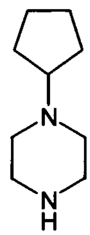 , 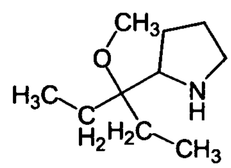 , 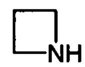 ,

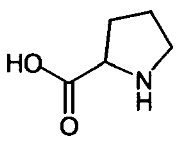 , 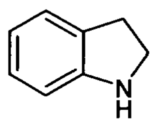 , 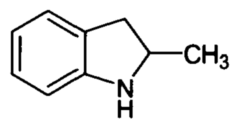 , 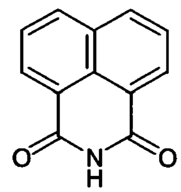 , 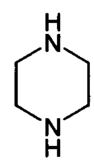 ,

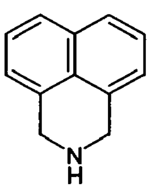 , 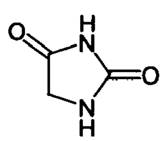 , 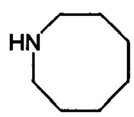 , 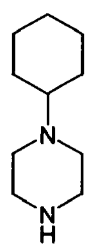 , 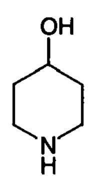 , 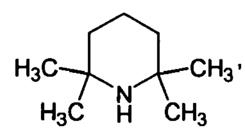 ,

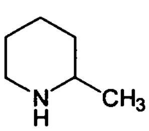 , 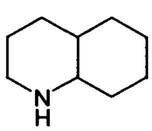 , 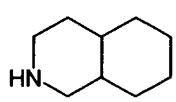 , 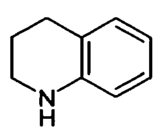 , 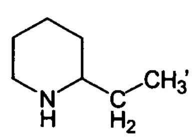

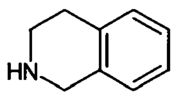, 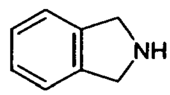, 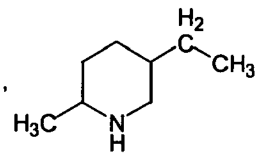, 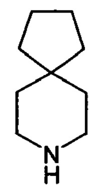 and 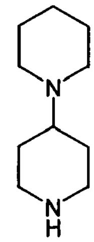;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;

a —O-$R^6$ moiety, a-$NR^7R^8$ moiety or a —$NR^9$-O-$R^{10}$ moiety;

$R^4$ represents F; Cl; Br; I; -CN; $-NO_2$; -NC; -OH; $-NH_2$; -SH; -C(=O)-H;

a radical selected from the group consisting of methyl, $-CF_3$, $-CH_2F$, $-CF_2H$, $-C_2F_5$, ethyl, $-CH_2$-CN, $-CH_2$-OH, n-propyl, isopropyl, $-CH_2$-$CH_2$-CN, $-CH_2$-$CH_2$-OH, n-butyl, $-CH_2$-$CH_2$-$CH_2$-CN, $-CH_2$-$CH_2$-$CH_2$-OH, isobutyl, sec-butyl, tert-butyl, n-pentyl, 2-pentyl, 3-pentyl, neo-pentyl, n-hexyl, 2-hexyl and 3-hexyl;

a radical selected from the group consisting of phenyl, naphthyl, pyridinyl, furyl (furanyl), thienyl (thiophenyl) and pyrrolyl, which may be bonded via a —($CH_2$)-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, F, Cl and Br;

a —O-$R^{11}$ moiety; a —S-$R^{12}$ moiety, a —NH-$R^{13}$ moiety or a —$NR^{14}R^{15}$ moiety;

$R^5$ represents H or an alkyl radical selected from the group consisting of methyl, ethyl, n-propyl, isopropyl and n-butyl;

$R^6$ represents a hydrogen atom; a radical selected from the group consisting of methyl, $-CF_3$, $-CH_2F$, $-CF_2H$, $-CH_2$-O-$CH_3$, $-C_2F_5$, $-CH_2$-$CH_2$-F, ethyl, $-CH_2$-CN, $-CH_2$-OH, n-propyl, isopropyl, $-CH_2$-$CH_2$-CN, $-CH_2$-$CH_2$-OH, $-CH_2$-$CH_2$-$OCH_3$, n-butyl, $-CH_2$-$CH_2$-$CH_2$-CN, $-CH_2$-$CH_2$-$CH_2$-OH, $-CH_2$-$CH_2$-$CH_2$-O-$CH_3$, isobutyl, sec-butyl, tert-butyl, n-pentyl, $-CH_2$-$CH_2$-$CH_2$-$CH_2$-O-$CH_3$, 2-pentyl, 3-pentyl, neo-pentyl, n-hexyl, 2-hexyl, 3-hexyl, n-heptyl and n-octyl;

a radical selected from the group consisting of cyclopropyl, cyclobutyl, cyclopentyl, cyclohexyl, cycloheptyl, pyrrolidinyl, piperidinyl, piperazinyl, homopiperazinyl, morpholinyl, imidazolidinyl, azepanyl, 8-aza-bicyclo[3.2.1]octyl and diazepanyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of oxo (=O), thioxo (=S), methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$ and -O-$C(CH_3)_3$;

a radical selected from the group consisting of pyridinyl, furyl (furanyl), thienyl (thiophenyl), pyrrolyl, oxazolyl, isoxazolyl, thiazolyl, isothiazolyl and imidazolyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, F, Cl, Br, I, -O-C(=O)-$CH_3$, -O-C(=O)-$C_2H_5$, -O-C(=O)-$CH(CH_3)_2$, -O-C(=O)-$CH_2$-$CH_2$-$CH_3$, $-CH_2$-$N(CH_3)_2$, -($CH_2$)-$N(C_2H_5)_2$, $-CH_2$-$N(C_3H_7)_2$, $-CH_2$-$N(C_4H_9)_2$, $-CH_2$-$N(CH_3)(C_2H_5)$ and —($CH_2$)-morpholinyl;

a —$P(=O)(OR^{16})_2$ moiety; a —C(=O)-$OR^{17}$ moiety; a —C(=O)-NH-$R^{18}$ moiety or a —C(=O)-$R^{19}$ moiety;

$R^7$ and $R^8$, independently of another, in each case represent a hydrogen atom;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, $-CH_2$-$NH_2$, $-CH_2$-$N(CH_3)_2$, $-CH_2$-CH-$NH_2$, $-CH_2$-$CH_2$-$N(CH_3)_2$, $-CH_2$-$CH_2$-$N(C_2H_5)_2$, $-CH_2$-$CH_2$-$CH_2$-$NH_2$, $-CH_2$-$CH_2$-$CH_2$-$N(CH_3)_2$ and $-CH_2$-$CH_2$-$CH_2$-$N(C_2H_5)_2$;

a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, [1,2,3,4]-tetrahydronaphthyl and bicyclo[2.2.1]heptyl, which may be bonded via a -($CH_2$)-, -($CH_2$)-($CH_2$)-, -($CH_2$)-($CH_2$)-($CH_2$)- or -CH=CH-group and/or may optionally be substituted with 1, 2, 3, 4 or 5 substituent(s) independently selected from the group consisting of —OH, methyl, ethyl, n-propyl, isopropyl, n-butyl, isobutyl, 2-butyl, tert-butyl, n-pentyl, 2-pentyl and n-hexyl;

a radical selected from the group consisting of phenyl, naphthyl, pyridinyl, furyl (furanyl), thienyl (thiophenyl) and triazolyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, F, Cl and Br;

a —S(=O)$_2$-$R^{20}$ moiety;
a —$NR^{21}R^{22}$ moiety;
a radical selected from the group consisting of

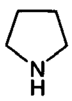 , 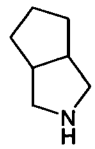 , 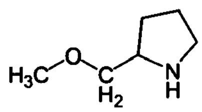 , 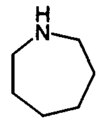 , 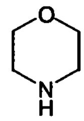 , 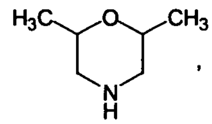 ,

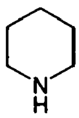 , 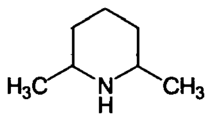 , 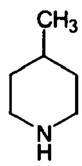 , 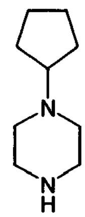 , 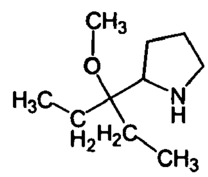 , 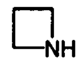 ,

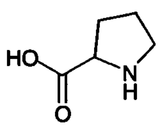 , 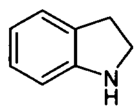 , 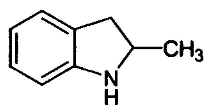 , 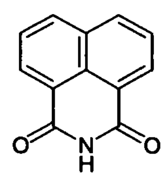 , 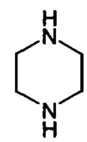 ,

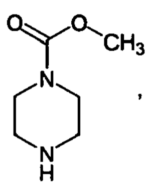 , 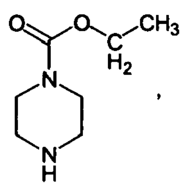 , 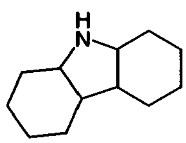 , 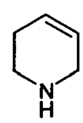 , 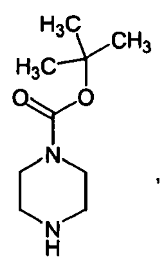 ,

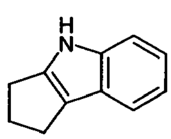 , 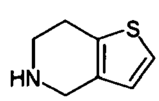 , 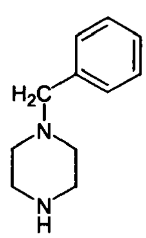 , 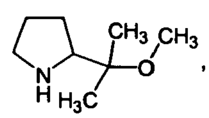 , 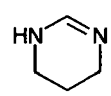 ,

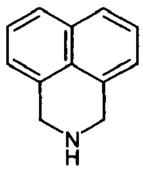, 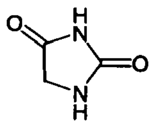, 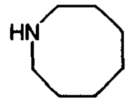, 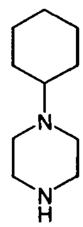, 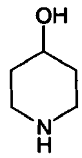, 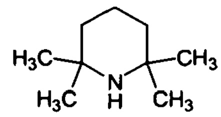,

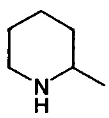, 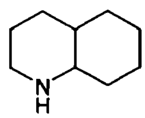, 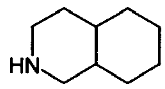, 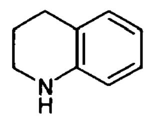, 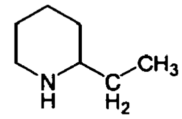,

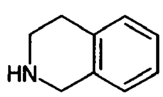, 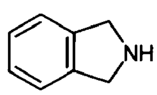, 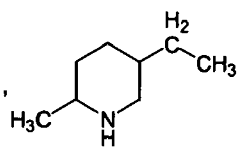, 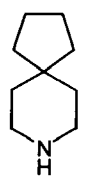 and 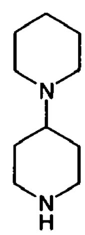 ;

which is in each case bonded to the pyrazoline compound of general formula I in any position of the cyclic part of the aforementioned radicals including the NH-groups, optionally via a —$(CH_2)$- or —$(CH_2)$-$(CH_2)$- group, preferably said radicals are bonded to the pyrazoline compound of general formula I at the nitrogen atom of the cyclic part of the aforementioned radicals;

$R^9$ represents hydrogen;

$R^{10}$, $R^{11}$, $R^{12}$, $R^{13}$, $R^{14}$, $R^{15}$ and $R^{20}$, independently of another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neopentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -$NH_2$, -NH-$CH_3$, -NH-$C_2H_5$, -N$(CH_3)_2$, -N$(C_2H_5)_2$, -CN and -$NO_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-azabicyclo[3.2.1]octyl and diazepanyl, which may be bonded via a —$(CH_2)$-, -$(CH_2)$-$(CH_2)$-, -$(CH_2)$-$(CH_2)$-$(CH_2)$- or—CH=CH-group and/or may optionally be substituted with 1, 2, 3, 4 or 5 substituent (s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N$(CH_3)_2$, -C(=O)-N$(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$, -N$(CH_3)_2$ and -N$(C_2H_5)_2$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —$(CH_2)$-, -$(CH_2)$-$(CH_2)$-, -$(CH_2)$-$(CH_2)$-$(CH_2)$- or -CH=CH-group and/or may optionally be substituted with 1, 2, 3, 4 or 5 substituent(s) independently selected from the group consisting of -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$,- O-

$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$, -$N(CH_3)_2$ and —$N(C_2H_5)_2$;

$R^{16}$, $R^{17}$, $R^{18}$ and $R^{19}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of $NH_2$, -NH-$CH_3$, -NH-$C_2H_5$, -$N(CH_3)_2$, —$N(C_2H_5)_2$, -NH-C(=O)-$CH_3$, -NH-C(=O)-$C_2H_5$, -NH-C(=O)-$C(CH_3)_3$, -NH-C(=O)-O-$CH_3$, -NH-C(=O)-O-$C_2H_5$, -NH-C(=O)-O-$C(CH_3)_3$, -C(=O)-NH-$CH_3$, -C(=O)-NH-$C_2H_5$, -C(=O)-NH-$C(CH_3)_3$, -C(=O)-$N(CH_3)_2$, -C(=O)-$N(C_2H_5)_2$, -OH, -C(=O)-OH, -C(=O)-O-$CH_3$, -C(=O)-O-$C_2H_5$, -C(=O)-O-$C(CH_3)_3$, -C(=O)-$CH_3$, -C(=O)-$C_2H_5$ and -C(=O)-$C(CH_3)_3$;
or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, -S-$CH_3$, -S-$C_2H_5$, -S-$CH_2$-$CH_2$-$CH_3$, -S-$CH(CH_3)_2$, -S-$CH_2$-$CH_2$-$CH_2$-$CH_3$, -S-$C(CH_3)_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-$C(CH_3)_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-$N(CH_3)_2$, -C(=O)-$N(C_2H_5)_2$, -S(=O)-$CH_3$, -S(=O)-$C_2H_5$, -S(=O)-$C_3H_7$, -$S(=O)_2$-$CH_3$, -$S(=O)_2$-$C_2H_5$, -$S(=O)_2$-$C_3H_7$, -$NH_2$, -NH-$CH_3$, -NH-$C_2H_5$,- $N(CH_3)_2$, —$N(C_2H_5)_2$, -O-C(=O)-$CH_3$, -O-C(=O)-$C_2H_5$, -O-C(=O)-$CH(CH_3)_2$, -O-C(=O)-$CH_2$-$CH_2$-$CH_3$, -$CH_2$-$N(CH_3)_2$, -($CH_2$)-$N(C_2H_5)_2$, -$CH_2$-$N(C_3H_7)_2$, -$CH_2$-$N(C_4H_9)_2$, -$CH_2$-N($CH_3$)($C_2H_5$) and -($CH_2$)-morpholinyl;
and
$R^{21}$ and $R^{22}$, independently of another, in each case represent hydrogen;
a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, -$CH_2$-$NH_2$, -CH-$N(CH_3)_2$, -$CH_2$-CH-$NH_2$, -$CH_2$-$CH_2$-$N(CH_3)_2$, -$CH_2$-$CH_2$-$N(C_2H_5)_2$, -$CH_2$-$CH_2$-$CH_2$-$NH_2$, -$CH_2$-$CH_2$-$CH_2$-$N(CH_3)_2$ and -$CH_2$-$CH_2$-$CH_2$-$N(C_2H_5)_2$;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl and bicyclo[2.2.1]heptyl, which may be bonded via a —($CH_2$)-, -($CH_2$)-($CH_2$)-, -($CH_2$)-($CH_2$)-($CH_2$)- or -CH=CH-group and/or may optionally be substituted with 1, 2, 3, 4 or 5 substituent(s) independently selected from the group consisting of —OH, methyl, ethyl, n-propyl, isopropyl, n-butyl, isobutyl, 2-butyl, tert-butyl, n-pentyl, 2-pentyl and n-hexyl;
a radical selected from the group consisting of phenyl, naphthyl, pyridinyl, furyl (furanyl), thienyl (thiophenyl) and triazolyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, F, Cl and Br;
or a radical selected from the group consisting of

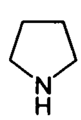 , 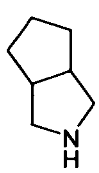 , 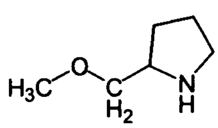 , 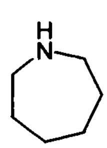 , 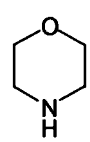 , 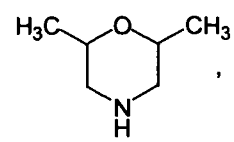 ,

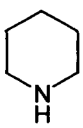, 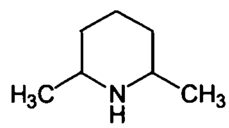, 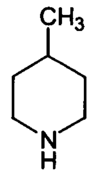, 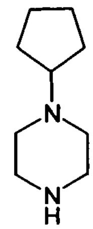, 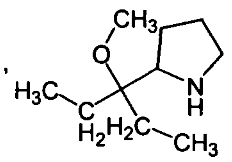, 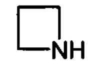,

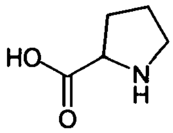, 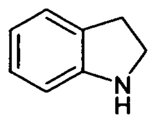, 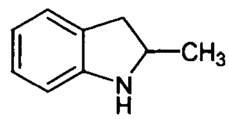, 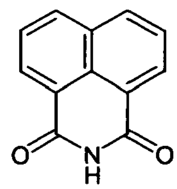, 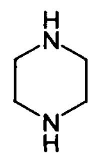,

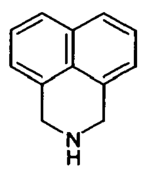, 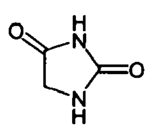, 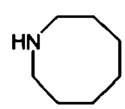, 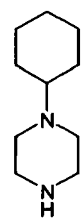, 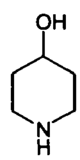, 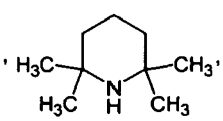,

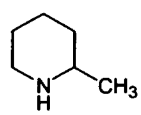, 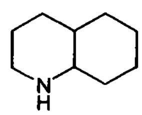, 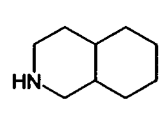, 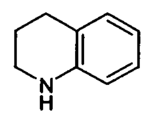, 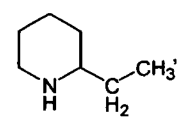,

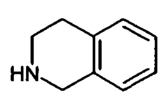, 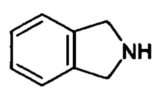, 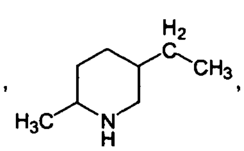, 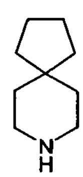 and 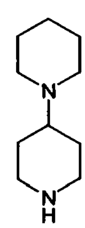 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals; optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

More particularly preferred are 4-substituted pyrazoline compounds of general formula I given above, wherein X is O

$R^1$ represents a radical selected from the group consisting of phenyl and thienyl (thiophenyl), which may optionally be substituted with 1, 2, 3, 4 or 5 substituent(s) independently selected from the group consisting of F, Cl, Br and I;

$R^2$ represents a phenyl radical, which may be substituted with 1, 2, 3, 4 or 5 substituent(s) independently selected from the group consisting of F, Cl, Br and I;

$R^3$ represents a radical selected from the group consisting of

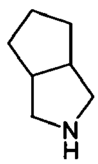 , 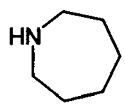 , 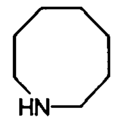 , 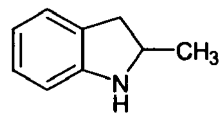 ,

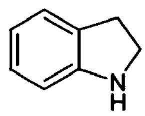 , 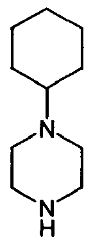 , 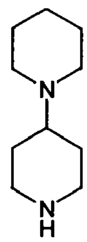 , 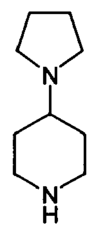 ,

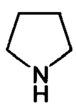 , 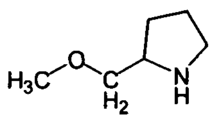 , 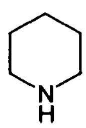 , 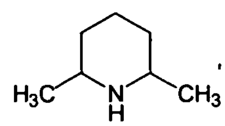 , 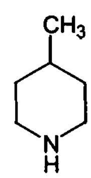 ,

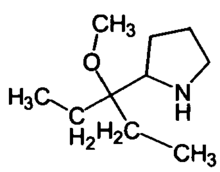 , 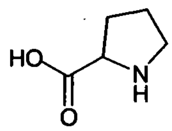 , 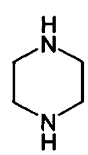 , 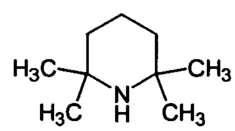 ,

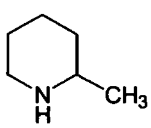 , 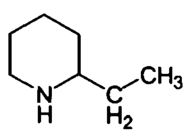 , 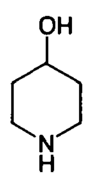 and 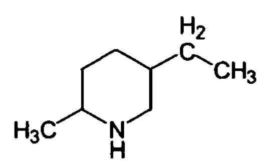 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;

a —$OR^6$-moiety or a -$NR^7R^8$ moiety;

$R^4$ represents F; Cl; Br; I; -OH, -CN; -C(=O)-H;

a radical selected from the group consisting of methyl, -$CF_3$, -$CH_2F$, -$CF_2H$, -$C_2F_5$, ethyl, -$CH_2$-CN, -$CH_2$-OH, n-propyl, isopropyl, -$CH_2$-$CH_2$-CN, -$CH_2$-$CH_2$-OH, n-butyl,- $CH_2$-$CH_2$-$CH_2$-CN, -$CH_2$-$CH_2$-$CH_2$-OH, isobutyl, sec-butyl, tert-butyl, n-pentyl, 2-pentyl, 3-pentyl, neo-pentyl, n-hexyl, 2-hexyl and 3-hexyl;

a benzyl radical or a —O-$R^{11}$ moiety;

$R^5$ represents H or a radical selected from the group consisting of methyl, ethyl, n-propyl, iso-propyl and n-butyl;

$R^6$ represents a hydrogen atom, a radical selected from the group consisting of methyl, ethyl, n-propyl, isopropyl, n-butyl and tert-butyl or a radical selected from the group consisting of cyclopropyl, cyclobutyl, cyclopentyl, cyclohexyl and cycloheptyl;

$R^7$ represents a hydrogen atom;

$R^8$ represents a radical selected from the group consisting of cyclopropyl, cyclobutyl, cyclopentyl, cyclohexyl and cycloheptyl, which may be bonded via a $-(CH_2)-$, $-(CH_2)-(CH_2)-$ or $-(CH_2)-(CH_2)-(CH_2)-$group; or a radical selected from the group consisting of

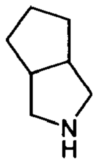 , 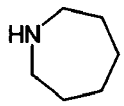 , 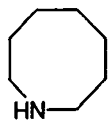 , 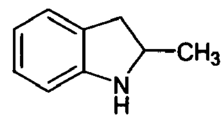 ,

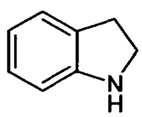 , 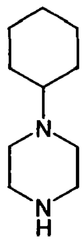 , 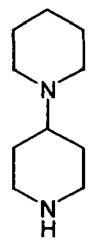 , 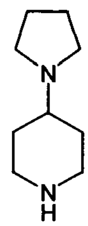 ,

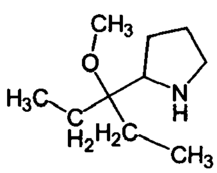 , 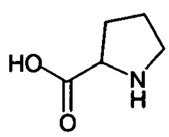 , 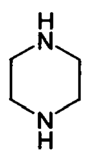 , 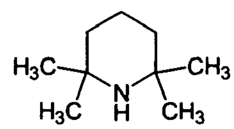 ,

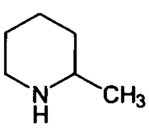 , 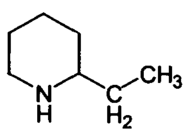 , 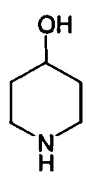 and 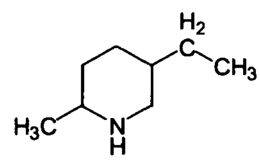 ;

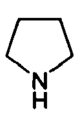 , 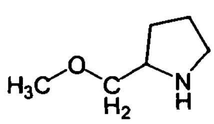 , 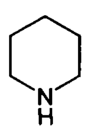 , 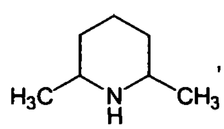 , 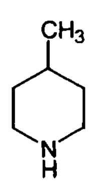 ,

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;

and

$R^{11}$ represents a radical selected from the group consisting of methyl, ethyl, n-propyl, isopropyl, n-butyl, isobutyl, sec-butyl, tert-butyl, n-pentyl, 2-pentyl, 3-pentyl and neo-pentyl; optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

Most particularly preferred are 4-substituted pyrazoline compounds of general formula I given above selected from the group consisting of

[1] *cis*-5-(4-Chloro-phenyl)-1-(2,4-dichloro-phenyl)-4-methyl-4,5-dihydro-1H-pyrazole-3-carboxylic acid piperidin-1-ylamide hydro-chloride

[2] *trans*-5-(4-Chloro-phenyl)-1-(2,4-dichloro-phenyl)-4-methyl-4,5-dihydro-1H-pyrazole-3-carboxylic acid piperidin-1-ylamide hydro-chloride

[3] *cis*-5-(4-Chloro-phenyl)-1-(2,4-dichloro-phenyl)-4-methyl-4,5-dihydro-1H-pyrazole-3-carboxylic acid azepan-1-ylamide

[4] *trans*-5-(4-Chloro-phenyl)-1-(2,4-dichloro-phenyl)-4-methyl-4,5-dihydro-1H-pyrazole-3-carboxylic acid azepan-1-ylamide

[5] *cis*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone

[6] *trans*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone

[7] *cis*-5-(4-Chloro-phenyl)-1-(2,4-dichloro-phenyl)-4-methyl-4,5-dihydro-1H-pyrazole-3-carboxylic acid pyn-olidin-1-ylamide

[8] *trans*-5-(4-Chloro-phenyl)-1-(2,4-dichloro-phenyl)-4-methyl-4,5-dihydro-1H-pyrazole-3-carboxylic acid pyrrolidin-1-ylamide

[9] *cis*-5-(4-Chloro-phenyl)-1-(2,4-dichloro-phenyl)-4-methyl-4,5-dihydro-1H-pyrazole-3-carboxylic acid (4-methyl-piperidin-1-yl)-amide

[10] *trans*-5-(4-Chloro-phenyl)-1-(2,4-dichloro-phenyl)-4-methyl-4,5-dihydro-1H-pyrazole-3-carboxylic acid (4-methyl-piperidin-1-yl)-amide

[11] *cis*-[5-(4-Chloro-phenyl)-1-(2,4-dichloro-phenyl)-4-methyl-4,5-dihydro-1H-pyrazol-3-yl]-piperidin-1-yl-methanone

[12] *trans*-[5-(4-Chloro-phenyl)-1-(2,4-dichloro-phenyl)-4-methyl-4,5-dihydro-1H-pyrazol-3-yl]-piperidin-1-yl-methanone

[13] *cis*-5-(4-Chloro-phenyl)-1-(2,4-dichloro-phenyl)-4-methyl-4,5-dihydro-1H-pyrazole-3-carboxylic acid (hexahydro-cyclopenta[c]pyrrol-2-yl)-amide

[14] *trans*-5-(4-Chloro-phenyl)-1-(2,4-dichloro-phenyl)-4-methyl-4,5-dihydro-1H-pyrazole-3-carboxylic acid (hexahydro-cyclopenta-[c]py-rrol-2-yl)-amide

[15] *cis*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide

[16] *trans*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide

[17] *cis*-5-(4-Chloro-phenyl)-1-(2,4-dichloro-phenyl)-4-methyl-4,5-dihydro-1H-pyrazole-3-carboxylic acid cyclobutylamide

[18] 5-(4-Chloro-phenyl)-1-(2,4-dichloro-phenyl)-4-methyl-4,5-dihydro-1H-pyrazole-3-carboxylic acid cyclo-hexyl methyl-amide

[19] *cis*-5-(4-Chloro-phenyl)-1-(2,4-dichloro-phenyl)-4-methyl-4,5-dihydro-1H-pyrazole-3-carboxylic acid

[20] *trans*-5-(4-Chloro-phenyl)-1-(2,4-dichloro-phenyl)-4-methyl-4,5-dihydro-1H-pyrazole-3-carboxylic acid

[21] *cis*-5-(4-Chloro-phenyl)-1-(2,4-dichloro-phenyl)-4-ethyl-4,5-dihydro-1H-pyrazole-3-carboxylic acid

[22] *trans*-5-(4-Chloro-phenyl)-1-(2,4-dichloro-phenyl)-4-ethyl-4,5-dihydro-1H-pyrazole-3-carboxylic acid piperidin-1-ylamide

[23] *cis*-5-(4-Chloro-phenyl)-1-(2,4-dichloro-phenyl)-4-methyl-4,5-dihydro-1H-pyrazole-3-carboxylic acid methyl ester

[24] *trans*-5-(4-Chloro-phenyl)-1-(2,4-dichloro-phenyl)-4-methyl-4,5-dihydro-1H-pyrazole-3-carboxylic acid methyl ester

[25] *cis*-5-(4-Chloro-phenyl)-1-(2,4-dichloro-phenyl)-4-methyl-4,5-dihydro-1H-pyrazole-3-carboxylic acid ethyl ester

[26] *trans*-5-(4-Chloro-phenyl)-1-(2,4-dichloro-phenyl)-4-methyl-4,5-dihydro-1H-pyrazole-3-carboxylic acid ethyl ester

[27] 5-(4-Chloro-phenyl)-1-(2,4-dichloro-phenyl)-4-methyl-4,5-dihydro-1H-pyrazole-3-carboxylic acid cyclohe-xyl ester

[28] 5-(4-Chloro-phenyl)-1-(2,4-dichloro-phenyl)-4,4-dimethyl-4,5-dihydro-1-H-pyrazole-3-carboxylic acid

[29] 5-(4-Chloro-phenyl)-1-(2,4-dichloro-phenyl)-4,4-dimethyl-4,5-dihydro-1-H-pyrazole-3-carboxylic acid piperidin-1-ylamide
[30] 5-(4-Chloro-phenyl)-1-(2,4-dichloro-phenyl)-4-trifluoromethyl-4,5-dihydro-1H-pyrazole-3-carboxylic acid piperidin-1-ylamide
[31] 5-(4-Chloro-phenyl)-1-(2,4-dichloro-phenyl)-4-hydroxy-4,5-dihydro-1H-pyrazole-3-carboxylic acid
[32] 5-(4-Chloro-phenyl)-1-(2,4-dichloro-phenyl)-4-hydroxy-4,5-dihydro-1H-pyrazole-3-carboxylic acid piperidin-1-ylamide
[33] 5-(4-Chloro-phenyl)-1-(2,4-dichloro-phenyl)-4-(2-hydroxy-ethyl)-4,5-di-hydro-1H-pyrazole-3-carboxylic acid piperidin-1-ylamide
[34] 5-(4-Chloro-phenyl)-1-(2,4-dichloro-phenyl)-4-(2-hydroxy-ethyl)-4,5-di-hydro-1H-pyrazole-3-carboxylic acid
[35] 5-(4-Chloro-phenyl)-1-(2,4-dichloro-phenyl)-4-fluoromethyl-4,5-dihydro-1H-pyrazole-3-carboxylic acid piperidin-1-ylamide
[36] 5-(4-Chloro-phenyl)-1-(2,4-dichloro-phenyl)-4-fluoromethyl-4,5-dihydro-1H-pyrazole-3-carboxylic acid
[37] 5-(4-Chloro-phenyl)-1-(2,4-dichloro-phenyl)-4-formyl-4,5-dihydro-1H-pyrazole-3-carboxylic acid piperidin-1-ylamide
[38] 5-(4-Chloro-phenyl)-1-(2,4-dichloro-phenyl)-4-formyl-4,5-dihydro-1H-pyrazole-3-carboxylic acid
[39] 1-(2,4-Dichloro-phenyl)-5-(4-fluoro-phenyl)-4-methyl-4,5-dihydro-1H-pyrazole-3-carboxylic acid piperidin-1-ylamide
[40] 5-(4-Chloro-phenyl)-4-cyano-1-(2,4-dichloro-phenyl)-4,5-dihydro-1H-pyrazole-3-carboxylic acid
[41] 1-(2,4-Dichloro-phenyl)-5-(4-fluoro-phenyl)-4-methyl-4,5-dihydro-1H-pyrazole-3-carboxylic acid piperidin-1-ylamide
[42] 1-(2,4-Dichloro-phenyl)-4-ethyl-5-(4-fluoro-phenyl)-4,5-dihydro-1H-pyrazole-3-carboxylic acid piperidin-1-ylamide
[43] 1-(2,4-Dichloro-phenyl)-5-(4-fluoro-phenyl)-4,4-dimethyl-4,5-dihydro-1-H-pyrazole-3-carboxylic acid piperidin-1-ylamide
[44] 1-(2,4-Dichloro-phenyl)-5-(4-fluoro-phenyl)-4-trifluoromethyl-4,5-dihydro-1H-pyrazole-3-carboxylic acid piperidin-1-ylamide
[45] 1-(2,4-Dichloro-phenyl)-5-(4-fluoro-phenyl)-4-hydroxy-4,5-dihydro-1H-pyrazole-3-carboxylic acid piperidin-1-ylamide
[46] 1-(2,4-Dichloro-phenyl)-5-(4-fluoro-phenyl)-4-(2-hydroxy-ethyl)-4,5-dihydro-1H-pyrazole-3-carboxylic acid piperidin-1-ylamide
[47] 1-(2,4-Dichloro-phenyl)-4-fluoromethyl-5-(4-fluoro-phenyl)-4,5-dihydro-1H-pyrazole-3-carboxylic acid piperidin-1-ylamide
[48] 1-(2,4-Dichloro-phenyl)-5-(4-fluoro-phenyl)-4-formyl-4,5-dihydro-1H-pyrazole-3-carboxylic acid piperidin-1-ylamide
[49] 4-Cyano-1-(2,4-dichloro-phenyl)-5-(4-fluoro-phenyl)-4,5-dihydro-1H-pyrazole-3-carboxylic acid piperidin-1-ylamide
[50] 5-(4-Bromo-phenyl)-1-(2,4-dichloro-phenyl)-4-methyl-4,5-dihydro-1H-pyrazole-3-carboxylic acid piperidin-1-ylamide
[51] 5-(4-Bromo-phenyl)-1-(2,4-dichloro-phenyl)-4-ethyl-4,5-dihydro-1H-pyrazole-3-carboxylic acid piperidin-1-ylamide
[52] 5-(4-Bromo-phenyl)-1-(2,4-dichloro-phenyl)-4,4-dimethyl-4,5-dihydro-1H-pyrazole-3-carboxylic acid piperidin-1-ylamide
[53] 5-(4-Bromo-phenyl)-1-(2,4-dichloro-phenyl)-4-trifluoromethyl-4,5-dihydro-1H-pyrazole-3-carboxylic acid piperidin-1-ylamide
[54] 5-(4-Bromo-phenyl)-1-(2,4-dichloro-phenyl)-4-hydroxy-4,5-dihydro-1H-pyrazole-3-carboxylic acid piperidin-1-ylamide
[55] 5-(4-Bromo-phenyl)-1-(2,4-dichloro-phenyl)-4-(2-hydroxy-ethyl)-4,5-dihydro-1H-pyrazole-3-carboxylic acid piperidin-1-ylamide
[56] 5-(4-Bromo-phenyl)-1-(2,4-dichloro-phenyl)-4-fluoromethyl-4,5-dihydro-1H-pyrazole-3-carboxylic acid piperidin-1-ylamide
[57] 5-(4-Bromo-phenyl)-1-(2,4-dichloro-phenyl)-4-formyl-4,5-dihydro-1H-pyrazole-3-carboxylic acid piperidin-1-ylamide
[58] 5-(4-Bromo-phenyl)-4-cyano-1-(2,4-dichloro-phenyl)-4,5-dihydro-1H-pyrazole-3-carboxylic acid piperidin-1-ylamide
[59] *cis*-5-(5-Chloro-thiophen-2-yl)-1-(2,4-dichloro-phenyl)-4-methyl-4,5-dihydro-1H-pyrazole-3-carboxylic acid piperidin-1-ylamide
[60] *trans*-5-(5-Chloro-thiophen-2-yl)-1-(2,4-dichloro-phenyl)-4-methyl-4,5-dihydro-1H-pyrazole-3-carboxylic acid

piperidin-1-ylamide
[61] *cis*-5-(5-Chloro-thiophen-2-yl)-1-(2,4-dichloro-phenyl)-4-ethyl-4,5-dihydro-1H-pyrazole-3-carboxylic acid piperidin-1-ylamide
[62] *trans*-5-(5-Chloro-thiophen-2-yl)-1-(2,4-dichloro-phenyl)-4-ethyl-4,5-dihydro-1H-pyrazole-3-carboxylic acid piperidin-1-ylamide
[63] 5-(5-Chloro-thiophen-2-yl)-1-(2,4-dichloro-phenyl)-4,4-dimethyl-4,5-dihydro-1H-pyrazole-3-carboxylic acid piperidin-1-ylamide
[64] 5-(5-Chloro-thiophen-2-yl)-1-(2,4-dichloro-phenyl)-4-trifluoromethyl-4,5-dihydro-1H-pyrazole-3-carboxylic acid piperidin-1-ylamide
[65] 5-(5-Chloro-thiophen-2-yl)-1-(2,4-dichloro-phenyl)-4-hydroxy-4,5-dihydro-1H-pyrazole-3-carboxylic acid piperidin-1-ylamide
[66] 5-(5-Chloro-thiophen-2-yl)-1-(2,4-dichloro-phenyl)-4-(2-hydroxy-ethyl)-4,5-dihydro-1H-pyrazole-3-carboxylic acid piperidin-1-ylamide
[67] 5-(5-Chloro-thiophen-2-yl)-1-(2,4-dichloro-phenyl)-4-fluoromethyl-4,5-dihydro-1H-pyrazole-3-carboxylic acid piperidin-1-ylamide
[68] 5-(5-Chloro-thiophen-2-yl)-1-(2,4-dichloro-phenyl)-4-formyl-4,5-dihydro-1H-pyrazole-3-carboxylic acid piperidin-1-ylamide
[69] 5-(5-Chloro-thiophen-2-yl)-4-cyano-1-(2,4-dichloro-phenyl)-4,5-dihydro-1H-pyrazole-3-carboxylic acid piperidin-1-ylamide
[70] *cis*-5-(5-Bromo-thiophen-2-yl)-1-(2,4-dichloro-phenyl)-4-methyl-4,5-dihydro-1H-pyrazole-3-carboxylic acid piperidin-1-ylamide
[71] *trans*-5-(5-Bromo-thiophen-2-yl)-1-(2,4-dichloro-phenyl)-4-methyl-4,5-dihydro-1H-pyrazole-3-carboxylic acid piperidin-1-ylamide
[72] *cis*-5-(5-Bromo-thiophen-2-yl)-1-(2,4-dichloro-phenyl)-4-ethyl-4,5-dihydro-1H-pyrazole-3-carboxylic acid piperidin-1-ylamide
[73] *trans*-5-(5-Bromo-thiophen-2-yl)-1-(2,4-dichloro-phenyl)-4-ethyl-4,5-dihydro-1H-pyrazole-3-carboxylic acid piperidin-1-ylamide
[74] 5-(5-Bromo-thiophen-2-yl)-1-(2,4-di-chloro-phenyl)-4,4-dimethyl-4,5-dihydro-1H-pyrazole-3-carboxylic acid piperidin-1-ylamide
[75] *cis*-5-(4-Bromo-thiophen-2-yl)-1-(2,4-dichloro-phenyl)-4-methyl-4,5-dihydro-1H-pyrazole-3-carboxylic acid piperidin-1-ylamide
[76] *trans*-5-(4-Bromo-thiophen-2-yl)-1-(2,4-dichloro-phenyl)-4-methyl-4,5-dihydro-1H-pyrazole-3-carboxylic acid piperidin-1-ylamide
[77] *cis*-5-(4-Bromo-thiophen-2-yl)-1-(2,4-dichloro-phenyl)-4-ethyl-4,5-dihydro-1H-pyrazole-3-carboxylic acid piperidin-1-ylamide
[78] *trans*-5-(4-Bromo-thiophen-2-yl)-1-(2,4-dichloro-phenyl)-4-ethyl-4,5-dihydro-1H-pyrazole-3-carboxylic acid piperidin-1-ylamide
[79] 5-(4-Bromo-thiophen-2-yl)-1-(2,4-dichloro-phenyl)-4,4-dimethyl-4,5-dihydro-1H-pyrazole-3-carboxylic acid piperidin-1-ylamide
[80] 5-(5-Bromo-thiophen-2-yl)-1-(2,4-dichloro-phenyl)-4-trifluoromethyl-4,5-dihydro-1H-pyrazole-3-carboxylic acid piperidin-1-ylamide
[81] 5-(5-Bromo-thiophen-2-yl)-1-(2,4-di-chloro-phenyl)-4-hydroxy-4,5-dihydro-1H-pyrazole-3-carboxylic acid piperidin-1-ylamide
[82] 5-(5-Bromo-thiophen-2-yl)-1-(2,4-di-chloro-phenyl)-4-(2-hydroxy-ethyl)-4,5-dihydro-1H-pyrazole-3-carboxylic acid piperidin-1-ylamide
[83] 5-(5-Bromo-thiophen-2-yl)-1-(2,4-di-chloro-phenyl)-4-fluoromethyl-4,5-dihydro-1H-pyrazole-3-carboxylic acid piperidin-1-ylamide
[84] 5-(5-Bromo-thiophen-2-yl)-1-(2,4-di-chloro-phenyl)-4-formyl-4,5-dihydro-1H-pyrazole-3-carboxylic acid piperidin-1-ylamide
[85] 5-(5-Bromo-thiophen-2-yl)-4-cyano-1-(2,4-dichloro-phenyl)-4,5-dihydro-1H-pyrazole-3-carboxylic acid piperidin-1-ylamide
[86] *cis*-5-(5-Chloro-thiophen-2-yl)-1-(2,4-dichloro-phenyl)-4-methyl-4,5-dihydro-1H-pyrazole-3-carboxylic acid
[87] *trans*-[5-(5-Chloro-thiophen-2-yl)-1-(2,4-dichloro-phenyl)-4-methyl-4,5-dihydro-1H-pyrazol-3-yl]-piperidin-1-yl-methanone
[88] *cis*-5-(5-Bromo-thiophen-2-yl)-1-(2,4-dichloro-phenyl)-4-methyl-4,5-dihydro-1H-pyrazole-3-carboxylic acid
[89] *trans*-5-(5-Bromo-thiophen-2-yl)-1-(2,4-dichloro-phenyl)-4-methyl-4,5-dihydro-1H-pyrazole-3-carboxylic acid
[90] *cis*-5-(4-Bromo-thiophen-2-yl)-1-(2,4-dichloro-phenyl)-4-methyl-4,5-dihydro-1H-pyrazole-3-carboxylic acid
and

[91] *trans*-5-(4-Bromo-thiophen-2-yl)-1-(2,4-dichloro-phenyl)-4-methyl-4,5-dihydro-1H-pyrazole-3-carboxylic acid;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

In another aspect the present invention also provides a process for the preparation of 4-substituted pyrazoline compounds of general formula I given above, wherein $R^5$ represents hydrogen, according to which at least one compound of general formula II,

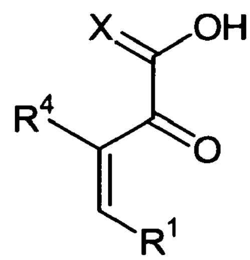

II,

wherein $R^1$, X and $R^4$ have the meaning given above, is reacted with at least one compound of general formula III,

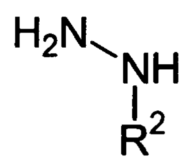

III,

or a corresponding salt thereof, wherein $R^2$ has the meaning given above, in a reaction medium, optionally in an inert atmosphere, optionally in the presence of at least one acid, to yield at least one compound of general formula IV,

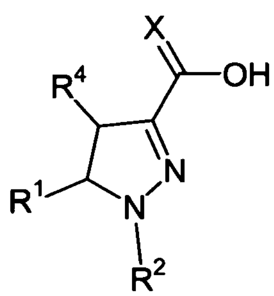

IV,

wherein $R^1$, X, $R^2$ and $R^4$ have the meaning given above, which is optionally isolated and/or purified,
and at least one compound of general formula IV is reacted with an activating agent in a reaction medium, optionally in an inert atmosphere, to yield at least one compound of general formula V,

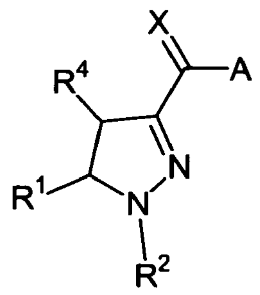

V,

wherein $R^1$, X, $R^2$ and $R^4$ have the meaning according given above and A represents a leaving group, which is optionally purified and/or isolated,

and at least one compound of general formula V is reacted with at least one compound of general formula $R^3$-H, wherein $R^3$ has the meaning given above, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula I, wherein $R^1$, $R^2$, X, $R^3$ and $R^4$ have the meaning given above and $R^5$ represents hydrogen, which is optionally purified and/or isolated;

or at least one compound of general formula IV is reacted with at least one compound of general formula $R^3$-H, wherein $R^3$ represents a —$NR^7R^8$ moiety, whereby $R^7$ and $R^8$ have the meaning given above, in a reaction medium, in the presence of at least one coupling agent, optionally in the presence of at least one base, to yield at least one compound of general formula I, wherein $R^1$, $R^2$, X and $R^4$ have the meaning given above, $R^3$ represents a —$NR^7R^8$ moiety and $R^5$ represents hydrogen, which is optionally purified and/or isolated.

Also preferred is the process for the preparation of a compound of general formula I given above, wherein $R^5$ represents a hydrogen atom, according to which at least one compound of general formula $R^1$-C(=O)-H (general formula VII), wherein $R^1$ has the meaning given above, is reacted with at least one compound of general formula VI,

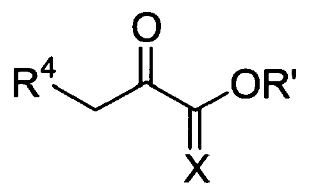

VI,

wherein $R^4$ and X have the meaning given above and R' represents a linear or branched $C_{1-6}$-alkyl radical, preferably an ethyl radical, a potassium cation or a sodium cation, in a reaction medium, optionally in an inert atmosphere, optionally in the presence of at least one base, to yield at least one compound of general formula II,

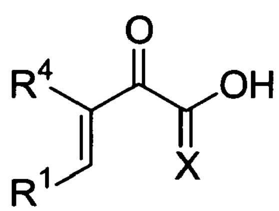

II,

wherein $R^1$, X and $R^4$ have the meaning given above, which is optionally purified and/or isolated,

and at least one compound of general formula II is reacted with an activating agent in a reaction medium, optionally in an inert atmosphere, to yield at least one compound of general formula VIII,

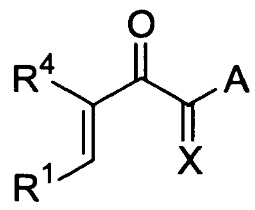

VIII,

wherein $R^1$, X and $R^4$ have the meaning given above and A represents a leaving group, which is optionally purified and/or isolated,
and at least one compound of general formula VIII is reacted with at least one compound of general formula $R^3$-H, wherein $R^3$ has the meaning given above, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula IX,

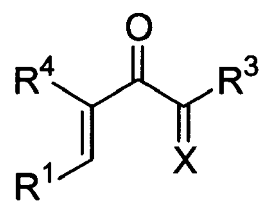

IX,

wherein $R^1$, X, $R^3$ and $R^4$ have the meaning given above, which is optionally purified and/or isolated;
or at least one compound of general formula II is reacted with at least one compound of general formula $R^3$-H, wherein $R^3$ represents a —$NR^7R^8$ moiety, whereby $R^7$ and $R^8$ have the meaning given above, in a reaction medium, in the presence of at least one coupling agent, optionally in the presence of at least one base, to yield at least one compound of general formula IX, wherein $R^3$ represents a —$NR^7R^8$ moiety, which is optionally purified and/or isolated,
and at least one compound of general formula IX is reacted with at least one compound of general formula III,

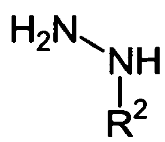

III,

wherein $R^2$ has the meaning given above, in a reaction medium, optionally in an inert atmosphere, optionally in the presence of at least one acid, to yield a compound of general formula I, wherein $R^1$, X, $R^2$, $R^3$ and $R^4$ have the meaning given above and $R^5$ represents hydrogen, which is optionally purified and/or isolated.

The inventive process is also illustrated in scheme I given below:

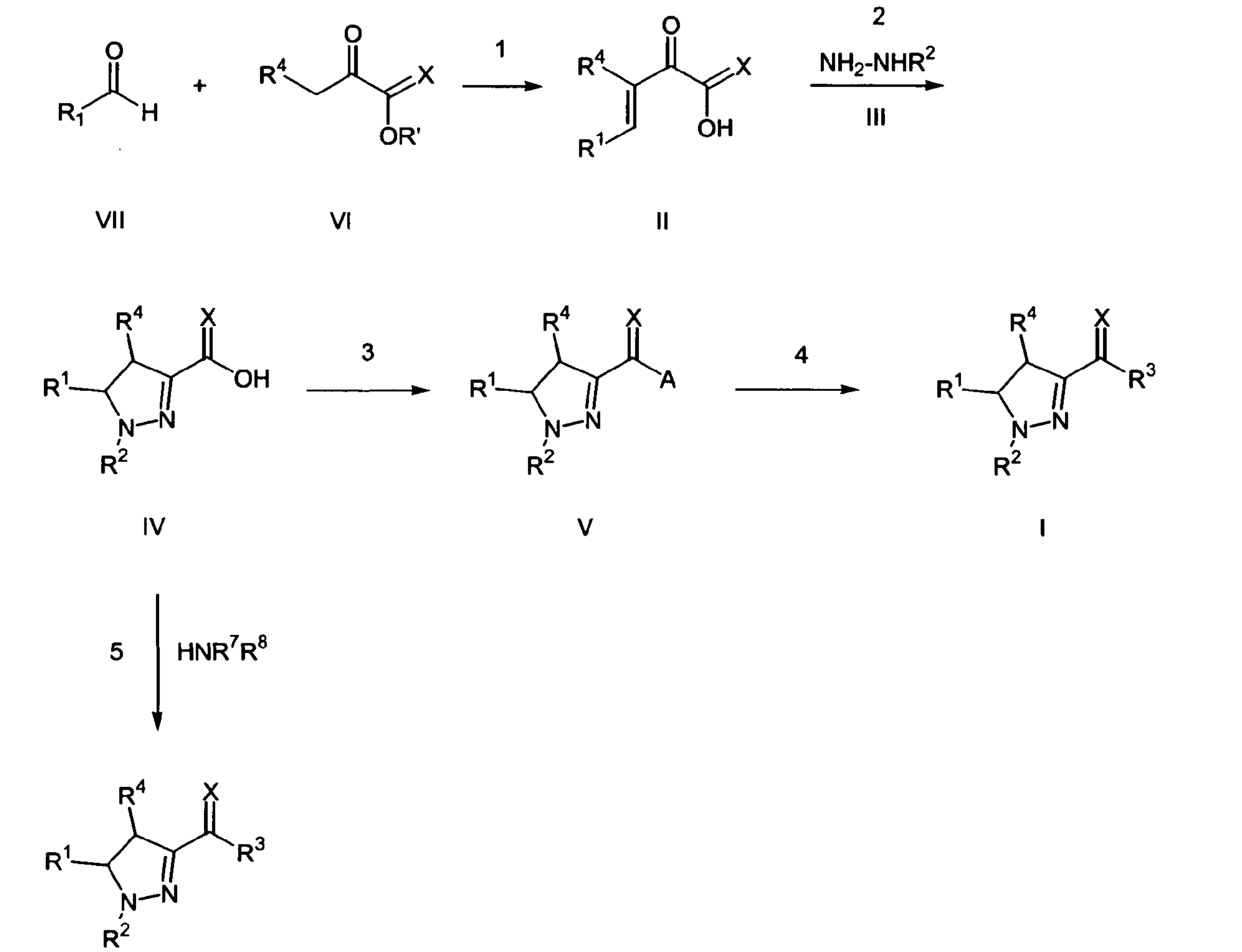


VII VI II

IV V I

I, $R^3$ = $-NR^7R^8$

**Scheme 1.**

In step 1 a compound of general formula VI is reacted with a compound of general formula VII in a protic reaction medium, preferably in a reaction medium selected from the group consisting of methanol, ethanol, isopropanol, n-butanol, water and mixtures thereof, in the presence of at least one base, preferably in the presence of an alkali metal hydroxide such as sodium hydroxide or potassium hydroxide or an alkali metal methoxide such as sodium methoxide, as described, for example, in Synthetic Communications, 26(11), 2229-33, (1996). The respective description is hereby incorporated by reference and forms part of the disclosure. Reaction temperature as well as the duration of the reaction may vary over a broad range. Preferred reaction temperatures range from —10 °C to the boiling point of the reaction medium. Suitable reaction times may vary for example from several minutes to several hours.

Preferably the reaction between a compound of general formula VI and general formula VII can also be carried out under acid catalysed conditions, more preferably by refluxing the above mentioned compounds in dichloromethane in the presence of copper(II)trifluoromethanesulfonate as described, for example, in Synlett, (1), 147-149, 2001. The respective description is hereby incorporated by reference and forms part of the disclosure.

In step 2 a compound of general formula II is reacted with a compound of general formula III in a reaction medium, preferably in a reaction medium selected from the group consisting of methanol, ethanol, isopropanol, n-butanol, diethylether, tert-butyl-methylether, dioxane, tetrahydrofurane or mixtures of at least two of these afore mentioned reaction media. Also preferably, said reaction may be carried out in the presence of an acid, whereby the acid may be organic such as acetic acid and/or inorganic such as hydrochloric acid. Altematively the reaction may also be carried out in the presence of a base such as piperidine, piperazine, sodium hydroxide, potassium hydroxide, sodium methoxide or sodium ethoxide or mixtures of at least two of these bases.

Reaction temperature as well as the duration of the reaction may vary over a broad range. Suitable reaction temperatures range from room temperature, i.e. approximately 25 °C to the boiling point of the reaction medium. Suitable

reaction times may vary for example from several minutes to several hours.

The carboxylic group of the compound of general formula IV may be activated for further reactions by the introduction of a suitable leaving group according to conventional methods well known to those skilled in the art. Preferably the compounds of general formula IV are transferred into an acid chloride, an acid anhydride, a mixed anhydride, a $C_{1-4}$ alkyl ester or an activated ester such as p-nitrophenylester. Suitable activating agent therefore are selected from the group consisting of thionyl chloride, oxalyl chloride and ethylchloroformate.

If said activated compound of general formula V is an acid chloride, wherein A represents a chlorine atom, that compound is preferably prepared by the reaction of the corresponding acid of general formula IV with thionyl chloride or oxalyl chloride, whereby said chlorinating agent is also used as the reaction medium, in the presence of at least one base, preferably in the presence of a base selected from the group consisting of triethylamine, N-methylmorpholine, pyridine, dimethylaminopyridine and diisopropylethylamine. Also preferably an additional reaction medium may be used. Suitable reaction media include hydrocarbons such as benzene, toluene or xylene, halogenated hydrocarbons such as dichloromethane, chloroform or carbon tetrachloride, ethers such as diethyl ether, dioxane, tetrahydrofurane or dimethoxyethane or dimethylformamide and mixtures thereof. More preferably toluene in the presence of a catalytic amount of dimethylformamide is used as reaction medium. Preferred reaction temperature range from 0° C to the boiling point of the solvent and reaction times vary from several minutes to several hours.

If said activated compound of general formula V is a mixed anhydride, wherein A represents —O-C(=O)-O-$C_2H_5$, said anhydride may preferably be prepared, for example, by reaction of the corresponding acid of general formula IV with ethylchloroformate in the presence of a base such as triethylamine, pyridine or diisopropylethylamine, in a suitable solvent such as dichloromethane, optionally in an inert atmosphere, at a temperature between -50 °C and 50 °C.

In step 4 the reaction between a compound of general formula V with a compound of general formula H-$R^3$ to yield a compound of general formula I, wherein $R^3$ represents a —$NR^7R^8$ moiety, is preferably carried out in the presence of a base such as triethylamine in a reaction medium such as methylenchloride. The temperature is preferably in the range from 0 °C to the boiling point of the reaction medium. The reaction time may vary over a broad range, e.g. from several hours to several days.

Altematively the reaction of a compound of general formula V with a compound of general formula H-$R^3$ to yield compounds of general formula I may be carried out according to conventional methods well known to those skilled in the art, e.g. from Pascual, A., J. Prakt Chem., 1999, 341(7), 695-700; Lin, S. et al., Heterocycles, 2001, 55(2), 265-277; Rao, P. et al., J. Org. Chem., 2000, 65(22), 7323-7344, Pearson D.E and Buehler, C.A., Synthesis, 1972, 533-542 and references cited therein. The respective descriptions are hereby incorporated by reference and form part of the present disclosure.

Preferably said reaction is carried out in the presence of a Lewis acid, which is preferably selected from the group consisting of $FeCl_3$, $ZnCl_2$ and $AlCl_3$, in a suitable reaction medium such as toluene, benzene, tetrahydrofurane or similar reaction media. The temperature is preferably in the range from 0° C to the boiling point of the reaction medium, more preferably from 15 to 25 °C. The reaction time may vary over a broad range, e.g. from several minutes to several hours.

In step 5 a compound of general formula IV is reacted with a compound of general formula H-$R^3$, wherein $R^3$ represents a —$NR^7R^8$ moiety, in a reaction medium, preferably in a reaction medium selected from the group consisting of diethylether, tetrahydrofuran, acetonitrile, methanol, ethanol, (1,2)-dichlorethane, dimethylformamide, dichlormethane and mixtures thereof, in the presence of at least one coupling agent, preferably in the presence of a coupling agent selected from the group consisting of 1-benzotriazolyloxy-tris-(dimethylamino)-phosphonium hexafluorophosphate (BOP), dicyclohexylcarbodiimide (DCC), N'-(3-dimethylaminopropyl)-N-ethylcarbodiimide (EDCI), diisoproylcarbodiimide, 1,1'-carbonyl-diimidazole (CDI), N-[(dimethyamino)-1H-1, 2, 3-triazolo[4, 5-b]pyridino-1-ylmethylen]-N-methylmethanaminium hexafluorophosphate N-oxid (HATU), O-(benzotriazol-1-yl)-N,N,N',N'-tetramethyluroniom hexafluorophosphate (HBTU), O-(benzotriazol-1-yl)-N,N,N',N'-tetramethyluronium-tetrafluoroborate (TBTU), 1-hydroxy-benzotriazole (HOBt) and 1-hydroxy-7-azabenzotriazol (HOAt), optionally in the presence of a base, preferably in the presence of a base selected from the group consisting of pyridine, dimethylaminopyridine, N-methylmorpholine, triethylamine and diisopropylethylamine to yield a compound of general formula I, wherein $R^3$ represents a —$NR^7R^8$ moiety.

Preferably said reaction is carried out in the presence of EDCI and HOBt, optionally in the presence of N-methylmorpholine or triethylamine, in an aprotic reaction medium such as dimethylformamide or tetrahydrofurane, at a temperature between 20 °C and 30 °C for 15 to 24 hours as described in Tetrahedron Lett. 2004, 45, 4977. The respective description is hereby incorporated by reference and forms part of the disclosure. Polymer-supported EDCI (P-EDCI) can also suitably be used for this process instead of EDCI as described in Tetrahedron Lett. 1998, 39, 1487 and Tetrahedron Lett. 2002, 43, 7685. The respective descriptions are hereby incorporated by reference and form part of the disclosure.

Altematively said reaction can be carried out by using HBTU in the presence of a base such as diisopropylethylamine in an aprotic solvent, such as acetonitrile, preferably at a temperature between 20 and 30 °C for 15 to 24 hours.

A further inventive process to obtain compounds of general formula IV is illustrated in scheme II given below.

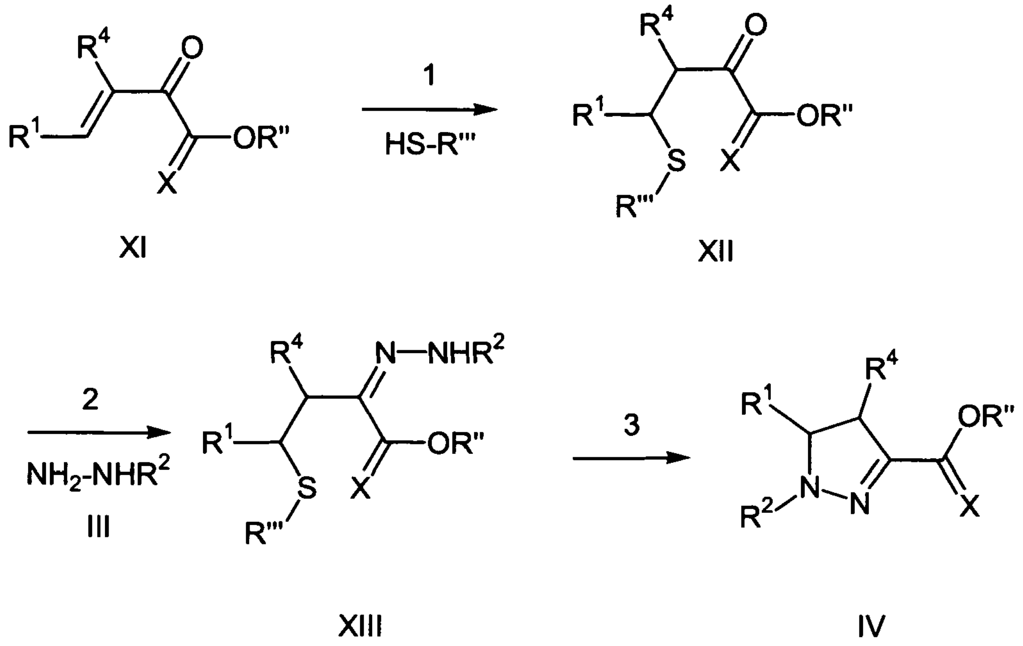


Scheme II

In step 1 a compound of general formula XI, wherein $R^1$, $R^4$ and X have the meaning given above and R" represents a hydrogen atom or a $C_{1-6}$-alkyl radical, is reacted with a compound of general formula HS-R"', wherein R"' represents an unsubstituted or at least mono-substituted phenyl radical, in a reaction medium, preferably in an dry aprotic reaction medium, more preferably in toluene, optionally in the presence of an organic base, preferably in the presence of an organic base selected from the group consisting of triethylamine, pyridine, diisopropylethylamine, dimethylaminopyridine and N-methylmorpholine, preferably at a temperature between - 50 °C and 50 °C, preferably for 4 to 24 hours, to yield a compound of general formula XII, wherein $R^1$, $R^4$, R", R"' and X have the meaning given above.

In step 2 a compound of general formula XII is reacted with a compound of general formula III, wherein $R^2$ has the meaning given above, in a reaction medium, preferably in a protic reaction medium, more preferably in methanol, optionally in the presence of an inorganic base, preferably in the presence of $KHSO_4$, preferably at a temperature between 0 °C and 100 °C, preferably for 4 to 15 hours, to yield a compound of general formula XIII, wherein $R^1$, $R^2$, $R^4$, R", R"' and X have the meaning given above.

In step 3 the compound of general formula XIII is cyclized intramoleculady in a reaction medium, preferably in a dry aprotic reaction medium, more preferably in dimethylformamide, preferably under an inert atmosphere, in the presence of a base, preferably in the presence of a metal hydride salt, more preferably in the presence of sodium hydride and/or potassium hydride to yield a compound of general formula IV. If R" represents a $C_{1-6}$-alkyl radical, the compound of general formula IV, wherein R" represents a hydrogen atom, is obtained after saponification of the cyclized compound according to methods known to those skilled in the art.

The sequence illustrated in scheme 1 is also described in, for example, Tetrahedron 2005, 81, 5235 - 5240 and Tetrahedron Asymmetry 2001, 12, 1923 - 1928. The respective descriptions are hereby incorporated by reference and form part of the disclosure.

A compound of general formula IV can also be obtained as described in scheme III given below.

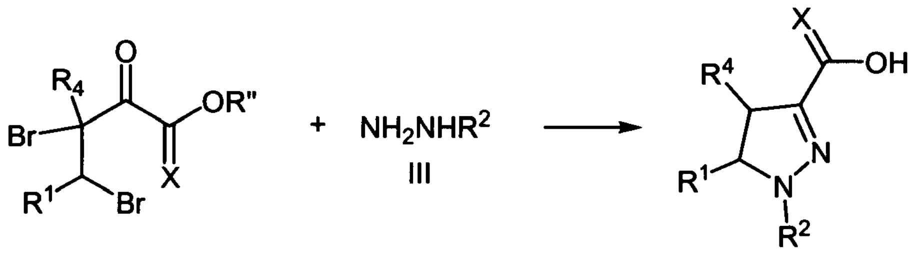

XIV

IV

Scheme III

The compound of general formula XIV, wherein $R^1$, $R^4$ and X have the meaning given above and R" represents a hydrogen atom or a $C_{1-6}$-alkyl radical, is obtained by the bromination of a compound of general formula XI in a reaction medium, preferably in an aprotic reaction medium, more preferably in dichloromethane, with bromine at a temperature between 0 °C and 30 °C for several hours as described in Tetrahedron Lett. 1998, 39 (44), 8163 - 8166; J. Chem. Soc. Perkin Trans 1, 1999, 21, 3069 - 3070; Tetrahedron 1999, 55 (36), 11127 - 11142 and J. Heterocyclic Chem. 1986, 23, 1199. The respective descriptions are hereby incorporated by reference and form part of the disclosure.

The compound of general formula XIV is reacted with a compound of general formula III, wherein $R^2$ has the meaning given above, and cyclized intramolecularly in a reaction medium, preferably in a dry aprotic reaction medium, more preferably in dimethylformamide or in a mixture of dioxane, water and acetic acid, at a temperature between 0 °C and 250 °C to yield a compound of general formula IV as described in Chemistry of Heterocyclic Compounds 1997, 33 (6); Indian J. Chem. 20B, 1981, 1090; Indian J. Chem. 29B, 1990, 887 and J. Indian Chem. Soc. 1997, 74(3), 202 - 205. The respective descriptions are hereby incorporated by reference and form part of the disclosure. If R" represents a $C_{1-6}$-alkyl radical, the compound of general formula IV, wherein R" represents a hydrogen atom, is obtained after saponification of the cyclized compound according to methods known to those skilled in the art.

A compound of general formula IV can also be obtained by the process described in scheme IV.

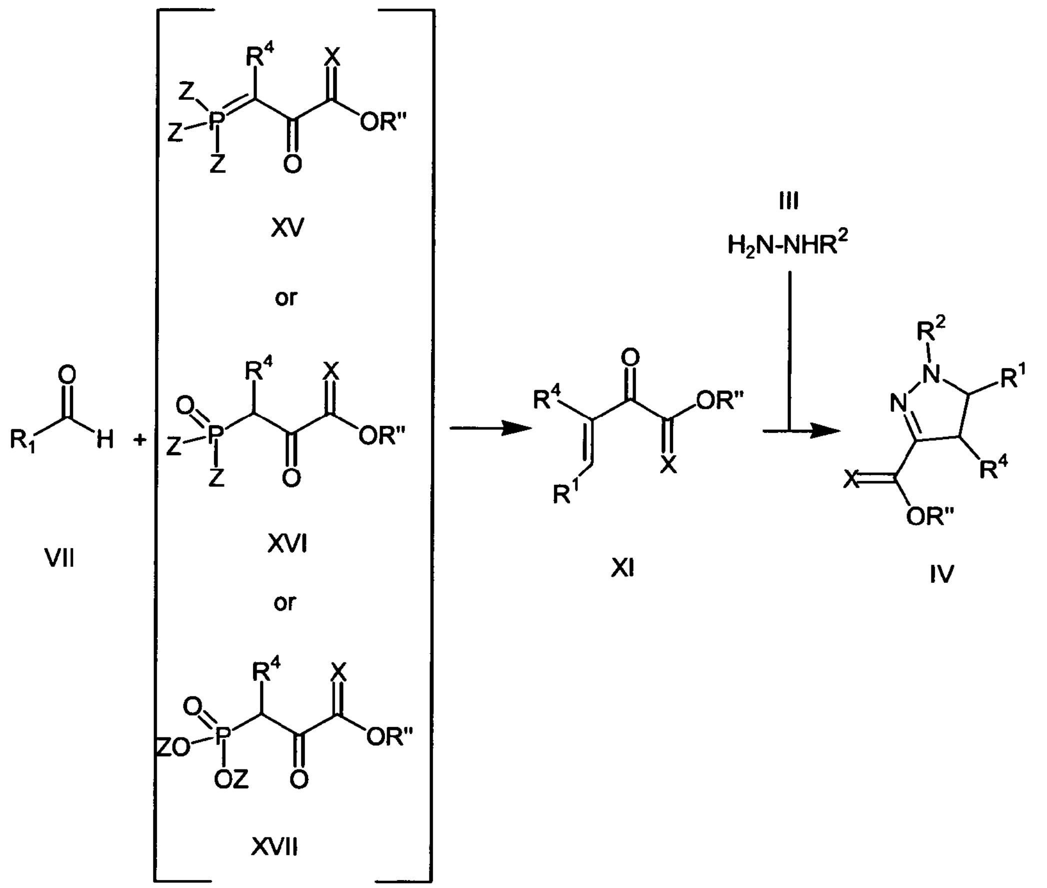

Scheme IV

An aldehyde of general formula VII, wherein R' has the meaning given above, is reacted with either a phosphonium ylide of general formula XV, a phosphine oxide of general formula XVI or a phosphonate of general formula XVII, wherein in each case $R^4$ and X have the meaning given above, R" represents a hydrogen atom or a $C_{1-6}$-alkyl radical and Z represents an unsubstituted or at least mono-substituted phenyl radical, in a reaction medium, preferably in an aprotic reaction medium, more preferably in tetrahydrofuran, optionally in the presence of at least one base, preferably in the presence of a base selected from the group consisting of potassium tert-butylat, n-butyllithium, sodium hydride and lithium diisopropylamide, to yield a compound of general formula XI which is reacted with a compound of general formula III, wherein $R^2$ has the meaning given above, and cyclized intramolecularly to yield a compound of general formula IV as described above. The process is also described in Tetrahedron 1994, 50 (44), 12727 -12742 and Zhumal Obshchei Khimii 1986, 56 (2), 347 - 353. The respective descriptions are hereby incorporated by reference and form part of the disclosure. If R" represents a $C_{1-6}$-alkyl radical, the compound of general formula IV, wherein R" represents a hydrogen atom, is obtained after saponification of the cyclized compound according to methods known to those skilled in the art.

Another method for the preparation of compounds of general formula XI is illustrated in scheme V below.

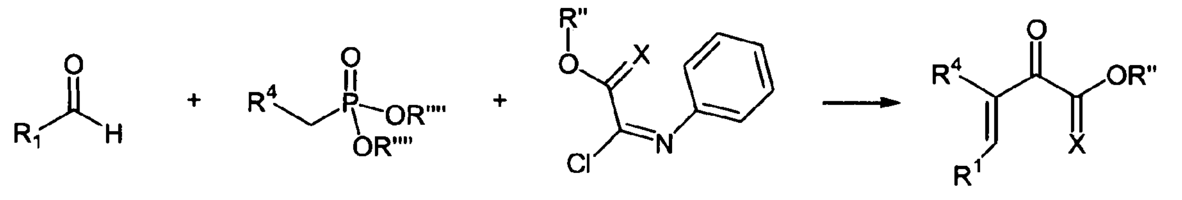

VII XVIII XIX XI

Scheme V

A compound of general formula VII, wherein $R^1$ has the meaning given above, is reacted with a phosphonate of general formula XVIII, wherein $R^4$ has the meaning given above and R'''' represents a $C_{1-6}$-alkyl radical, preferably an ethyl radical, and a compound of general formula XIX, wherein X has the meaning given above and R'' represents a hydrogen atom or a $C_{1-6}$-alkyl radical to yield a compound of general formula XI, wherein $R^1$, $R^4$, X and R'' have the meaning given above. The process is described in J. Chem. Soc. Perkin Trans 1, 1995, 741-742. Preferably the reaction is carried out by the addition of phosphonate of general formula XVIII to a solution of n-butyllithium in a dry reaction medium, preferably in tetrahydrofurane, at a temperature between -100 °C and - 50 °C, followed by the addition of N-phenylalcoxycarbonylacetimidoyl chloride of general formula XIX and aldehyde of general formula VII and stirring at a temperature between 0 °C and 30 °C for several hours. The respective description is hereby incorporated by reference and forms part of the disclosure.

A compound of general formula IV can also be obtained according to the process described in scheme VI.

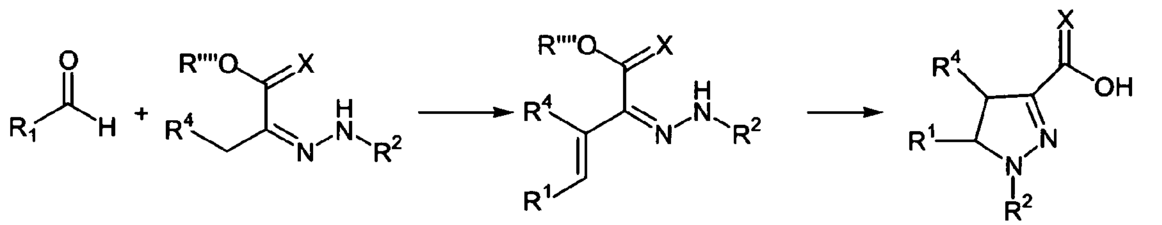

VII XX XXI IV

Scheme VI

A compound of general formula Vil, wherein $R^1$ has the meaning given above, is reacted with a compound of general formula XX, wherein $R^2$, $R^4$ and X have the meaning given above and R'''' represents a $C_{1-6}$-alkyl radical, is reacted in a reaction medium, preferably in ethanol, in the presence of a base, preferably sodium acetate, at a temperature between 30 °C and 90 °C, or in a reaction medium, preferably in ethanol, in the presence of glacial acetic acid at a temperature between 0 °C and 50 °C to yield a compound of general formula XXI, wherein $R^1$, $R^2$, $R^4$ and X have the meaning given above and R'''' represents a $C_{1-6}$-alkyl radical. The compound of general formula XXI is converted into the compound of general formula IV in a reaction medium, preferably in ethanol and/or water, in the presence of an acid, preferably in the presence of hydrochloric acid, at a temperature between 50 °C and 120 °C to yield a compound of general formula IV. The process is described in J. Chem. Engineering Data 1984, 29(2), 225 - 229 and Indian J. Chem. 27B, 1988, 3, 245-249. The respective descriptions are hereby incorporated by reference and form part of the disclosure.

A compound of general formula IV can also be obtained according to the process described in scheme VII.

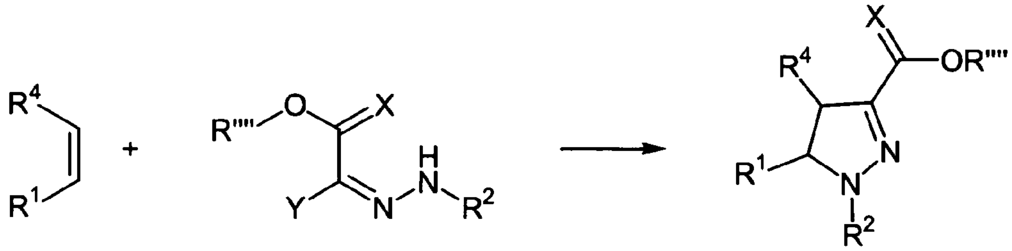

XXII XXIII IV

Scheme VII

A compound of general formula XXII, wherein $R^1$ and $R^4$ have the meaning given above, is reacted with a compound of general formula XXIII, wherein $R^2$ and X have the meaning given above, R'''' represents a $C_{1-6}$-alkyl radical and Y represents a chlorine or bromine atom, in a reaction medium, preferably in an aprotic or protic reaction medium, more preferably in toluene and/or chloroform and/or ethanol, in the presence of a base, preferably an organic base, more preferably an organic base selected from the group consisting of triethylamine, pyridine, diisopropylethylamine, dimethylaminopyridine and N-methylmorpholine, at a temperature between 0 °C and 150 °C to yield a compound of general formula IV. If regioisomers are obtained during the reaction, these regioisomers can be separated by conventional chromatographic techniques. The compound of general formula IV, wherein R'''' represents a $C_{1-6}$-alkyl radical is converted into the corresponding acid by using standard methods which are known to those skilled in the art. The process is disclosed in Bull. Chem. Soc. Japan 1984, 57 (3), 787 - 790 and Chem. Lett. 1982, 543 - 546. The respective description are hereby incorporated by reference and form part of the disclosure.

The compound of general formula XXIII can be prepared according to the processes described in scheme VIII.

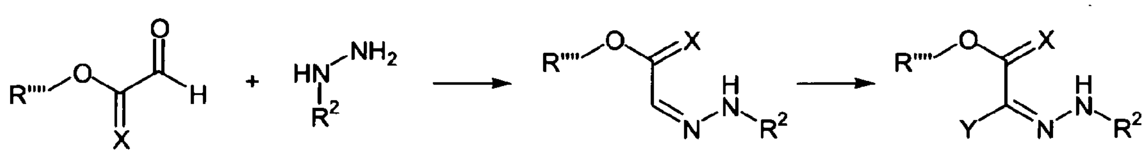

XXIV III XXV XXIII

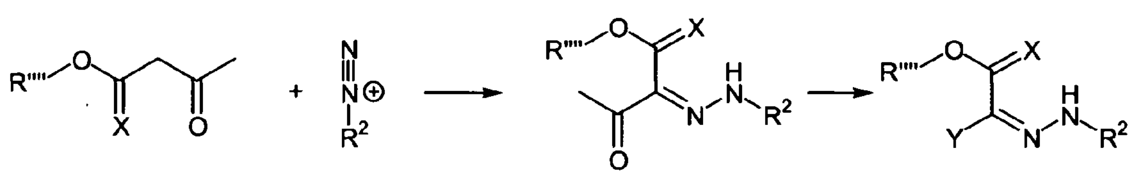

XXVI XXVII XXVIII XXIII

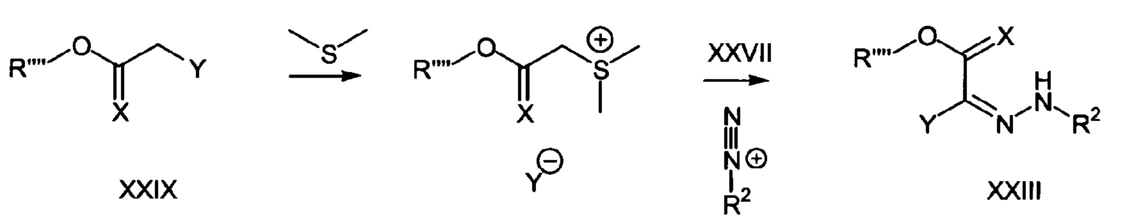

XXIX XXIII

Scheme VIII

The compound of general formula XXIV, wherein X has the meaning given above and R'''' represents a $C_{1-6}$-alkyl

radical, is reacted in a reaction medium, preferably in a mixture of water and ethanol, in the presence of at least one acid, preferably in the presence of acetic acid, at a temperature between 70 °C and 120 °C with a compound of general formula III, wherein $R^2$ has the meaning given above, to yield a compound of general formula XXV, wherein $R^2$ and X have the meaning given above and R'''' represents a $C_{1-6}$-alkyl radical, which is reacted with N-chloro-succinimide or N-bromo-succinimide in a reaction medium, preferably in an aprotic reaction medium, more preferably in dimethylformamide, at a temperature between 0 °C and 30 °C, to yield a compound of general formula XXIII. The process is described in Synth. Commun. 2001, 31(1), 111-115 and Tetrahedron 1994, 50 (25), 7543 - 7556. The respective descriptions are hereby incorporated by reference and form part of the disclosure.

The compound of general formula XXIII can also be prepared by the reaction of a compound of general formula XXVI, wherein X has the meaning given above and R'''' represents a $C_{1-6}$-alkyl radical, with a compound of general formula XXVII, wherein $R^2$ has the meaning given above and subsequent bromination of the resulting compound of general formula XXVIII, wherein $R^2$ and X have the meaning given above and R'''' represents a $C_{1-6}$-alkyl radical, by using bromine in the presence of acetic acid as described in Synthesis 1975, 333 and J. Chem. Soc. Perkin Trans. 1, 1977, 2092. The diazonium salt of general formula XXVII can preferably be obtained by the addition of an aqueous solution of sodium nitrite to a compound of general formula $R^2$-$NH_2$ in aqueous hydrochloride acid, wherein $R^2$ has the meaning given above. Altematively this transformation can also be achieved in the presence of a compound of general formula XXVI by adjusting the pH of the reaction medium to 4 by the addition of sodium acetate at a temperature between 0 °C and 30 °C. The respective descriptions are hereby incorporated by reference and form part of the disclosure.

The compound of general formula XXIII can also be prepared by the reaction of a compound of general formula XXIX, wherein X has the meaning given above, R'''' represents a $C_{1-6}$-alkyl radical and Y represents a chlorine or bromine atom, with dimethylsulfide, in a reaction medium, preferably in ethanol, at a temperature between 70 °C and 120 °C. Optionally the dimethylsulfonium salt is isolated and further reacted with a compound of general formula XXVII, wherein $R^2$ has the meaning given above, in the presence of sodium acetate and acetic acid at a temperature between 0 °C and 30 °C as described in Heterocycles 1991, 32(6), 1101 — 1107.

The respective description is hereby incorporated by reference and forms part of the disclosure.

Another method for the preparation of a compound of general formula IV is described in scheme IX.

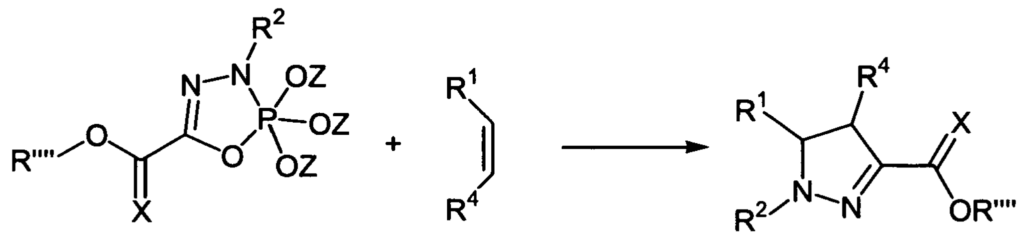

XXX XXII IV

Scheme IX

A compound of general formula XXX, wherein $R^2$ and X have the meaning given above, Z represents an unsubstituted or at least mono-substituted phenyl radical, preferably an unsubstituted phenyl radical, and R'''' represents a $C_{1-6}$-alkyl radical, preferably an ethyl radical, is reacted with a compound of general formula XXII, wherein $R^1$ and $R^4$ have the meaning given above, in a reaction medium, preferably in xylene, at a temperature between 50 °C and 200 °C for 2 to 30 hours to yield a compound of general formula IV. The process is described in Chem. Lett. 1983, 507 - 510 and Bull. Chem. Soc. Japan 1984, 57(9), 2689 - 2690. The respective descriptions are hereby incorporated by reference and form part of the disclosure. The compound of general formula IV, wherein R'''' represents a $C_{1-6}$-alkyl radical, is converted into the corresponding acid by using standard methods which are known to those skilled in the art.

A process for the preparation of a compound of general formula XXX is described in scheme X.

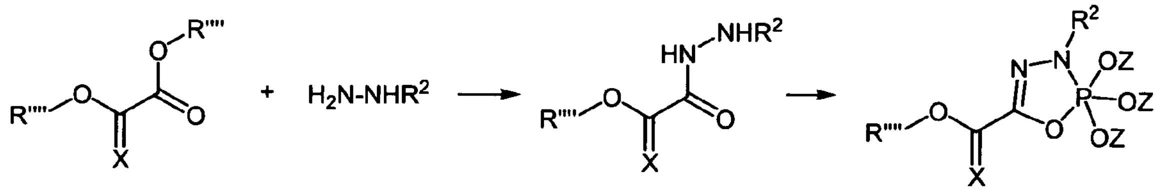

XXXI III XXXII XXX

Scheme X

A compound of general formula XXXI, wherein X has the meaning given above and R'''' is a $C_{1-6}$-alkyl radical, is reacted with a compound of general formula III, wherein $R^2$ has the meaning given above, to yield a compound of general formula XXXII, wherein $R^2$ and X have the meaning given above and R'''' represents a $C_{1-6}$-alkyl radical. Subsequently, the compound of general formula XXXIII is reacted with phosphorous pentachloride or $POCl_3$ in a reaction medium, preferably in toluene, at a temperature between 0 °C and 50 °C, followed by the addition of a phenolic compound, preferably O-trimethylsilyl-p-cresol, in refluxing toluene to yield a compound of general formula XXX.

Another method for the preparation of a compound of general formula IV is described in scheme XI.

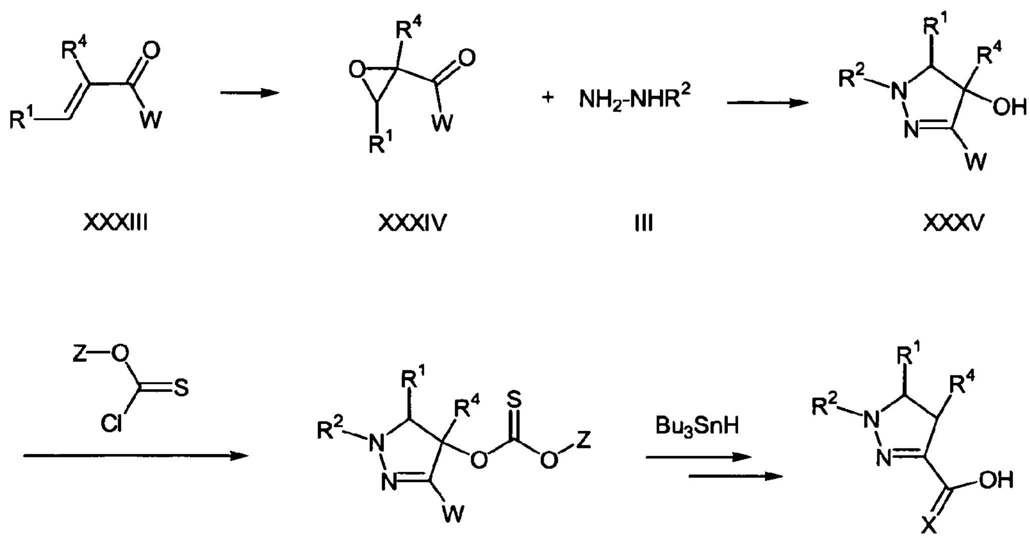


XXXIII XXXIV III XXXV

XXXVI IV

Scheme XI

A compound of general formula XXXIII, wherein $R^1$ and $R^4$ have the meaning given above and W represents -C(=X)-OH, -C(=X)-OR'''' or -CN, whereby X has the meaning given above and R'''' represents a $C_{1-6}$-alkyl radical, is converted to a compound of general formula XXXIV, wherein $R^1$, $R^4$ and W have the meaning given above, by means of epoxidation with a reagent selected from the group consisting of perbenzoic acid, preferably m-chloro-perbenzoic acid, sodium peroxocarbonate, hydrogen peroxide, dioxirane and hydroperoxide. The compound of general formula XXIV is reacted with a compound of general formula III, wherein $R^2$ has the meaning given above, in a reaction medium, preferably in ethanol, to yield a compound of general formula XXXV, wherein $R^1$, $R^2$, W and $R^4$ have the meaning given above. Subsequently, the compound of general formula XXXV is converted into the corresponding xanthate of general formula XXXVI, wherein $R^1$, $R^2$, W and $R^4$ have the meaning given above, by reaction with an unsubstituted or at least mono-substituted phenylthionochloroformate of general formula ZO-C(=S)-Cl, wherein Z represents an unsubstituted or at least mono-substituted phenyl radical. The compound of general formula XXXVI is reacted with tributyltinhydride

optionally followed by saponification and/or hydrolysis to yield a compound of general formula IV, wherein $R^1$, $R^2$, X and $R^4$ have the meaning given above. The process is described in Synlett 1990, 11, 705-706. The respective description is hereby incorporated by reference and forms part of the disclosure.

Yet another method for the preparation of a compound of general formula IV is described in scheme XII.

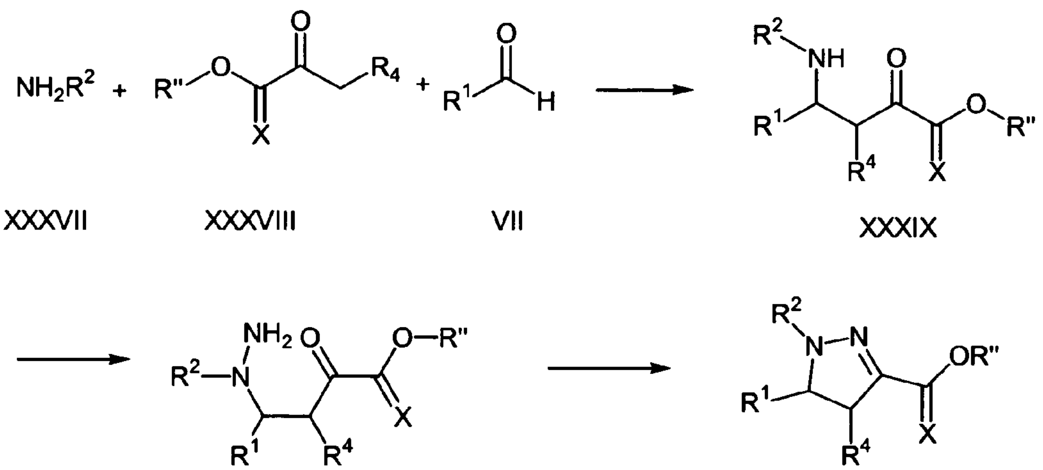

XXXVII XXXVIII VII XXXIX

XXXX IV

Scheme XII

A compound of general formula XXXVII, wherein $R^2$ has the meaning given above, is reacted with a compound of general formula XXXVIII, wherein $R^4$ and X have the meaning given above and R" represents a hydrogen atom or a $C_{1-6}$-alkyl radical, and a compound of general formula VII, wherein $R^1$ has the meaning given above, to yield a compound of general formula XXXIX, wherein $R^1$, $R^2$, $R^4$ and X have the meaning given above and R" represents a hydrogen atom or a $C_{1-6}$-alkyl radical. The compound of general formula XXXIX is converted into the compound of general formula XXXX by using O-substituted hydroxylamines according to the method described in J. Org. Chem. 2002, 67, 6237 - 6239. The respective description is hereby incorporated by reference and forms part of the disclosure. Alternatively, this transformation can be achieved by using nitrites and subsequent reduction according to methods known to those skilled in the art. Upon cyclization of a compound of general formula XXXX according to the methods described above a compound of general formula IV is obtained. If R" represents a $C_{1-6}$-alkyl radical, the compound of general formula IV, wherein R" represents hydrogen, is obtained after saponification of the cyclized compound according to methods known to those skilled in the art.

In case $R^5$ is unlike hydrogen the reaction sequence given in Scheme I is adapted as follows.

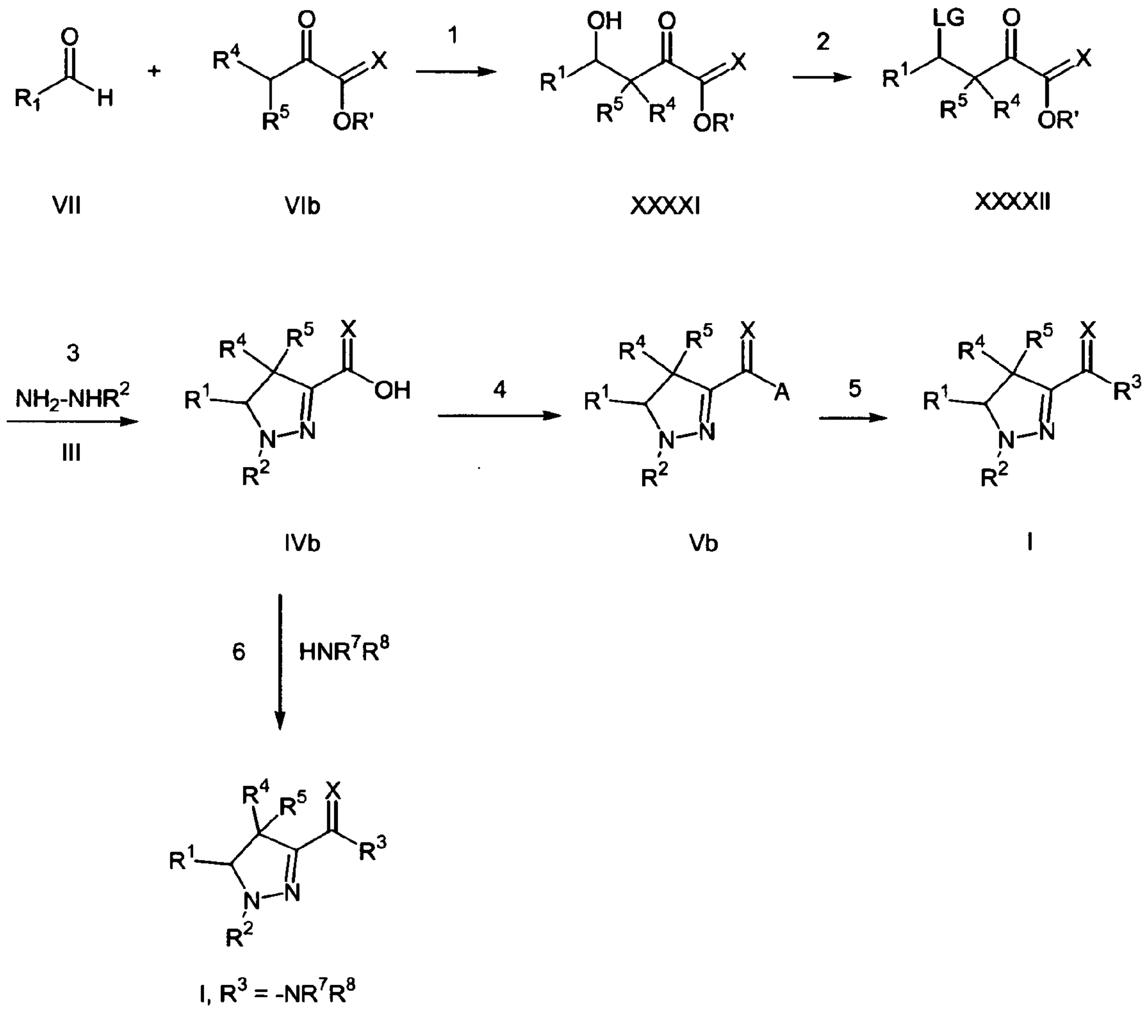


Scheme XIII

In step 1 a compound of general formula VIb is reacted with a compound of general formula VII in a reaction medium , preferably in a protic reaction medium, more preferably in a reaction medium selected from the group consisting of methanol, ethanol, isopropanol, n-butanol, water and mixtures thereof, in the presence of at least one base, preferably in the presence of an alkali metal hydroxide such as sodium hydroxide or potassium hydroxide or an alkali metal methoxide such as sodium methoxide or in the presence of lithium diisopropylamide in an aprotic solvent, preferably in tetrahydrofurane.

In step 2 a compound of general formula XXXXI is transformed into a compound of general formula XXXXII which contains a good living group LG, preferably a leaving group selected from the group consisting of mesyl and tosyl, using conventional methods known to those skilled in the art.

In step 3 a compound of general formula XXXXII is reacted with a compound of general formula III in a reaction medium, preferably in a reaction medium selected from the group consisting of methanol, ethanol, isopropanol, n-butanol, dieethylether, tert-butyl-methylether, dioxane, tetrahydrofurane or mixtures of at least two of these afore mentioned reaction media. Also preferably, said reaction may be carried out in the presence of an acid, whereby the acid may be organic such as acetic acid and/or inorganic such as hydrochloric acid. Altematively the reaction may also be carried out in the presence of a base such as piperidine, piperazine, sodium hydroxide, potassium hydroxide, sodium methoxide or sodium ethoxide or mixtures of at least two of these bases. Reaction temperature as well as the duration of the reaction may vary over a broad range. Suitable reaction temperatures range from room temperature, i.e. approximately 25 °C to the boiling point of the reaction medium. Suitable reaction times may vary for example from several minutes to several hours.

Step 4 can be carried out as described for step 3, scheme I; step 5 can be carried out as described for step 4, scheme I and step 6 can be carried out as described for step 5, scheme 1.
The compounds of general formula I, wherein $R^5$ is unlike hydrogen, can also be obtained by the reaction sequence described in scheme XIV.

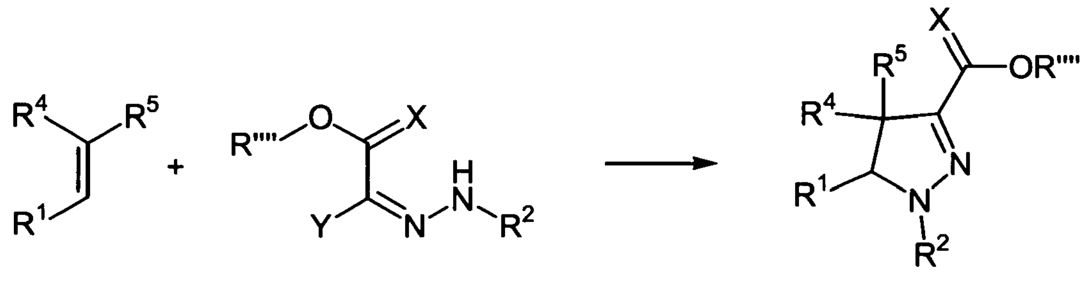

XXIIb XXIII IVb

Scheme XIV

The reaction is carried out as described for the analogues reaction depicted in scheme VII. If a mixture of regioisomers is obtained, said regioisomers can be separated by standard methods known to those skilled in the art, e. g. chromatographic methods or crystallization. The process is disclosed in Bull. Chem. Soc. Japan 1984, 57 (3), 787 - 790. The respective description is hereby incorporated by reference and forms part of the disclosure.

Compounds of general formula I, wherein X represents O, can be transformed in the corresponding compound, wherein X represents S, by reacting the first compound with a dithiaphosphetane, preferably with 2,4-bis(4-methoxyphenyl)-1,3,2,4-dithiadiphosphetan-2,4-disulfide (Lawesson reagent) or phosphorous pentasulfide, in a reaction medium, preferably in a reaction medium selected from the group consisting of toluene, xylene, acetonitrile, dichloromethane and dimethylformamide, at a temperature between 50 °C to 150 °C.

The afore mentioned reactions involving the synthesis of the 4,5-dihydro-pyrazole ring or the reaction of a compound comprising said ring are preferably carried out under an inert atmosphere, preferably under a nitrogen or argon atmosphere, to avoid oxidation of the ring-system.

During some synthetic reactions described above the protection of sensitive or reactive groups may be necessary and/or desirable. This can be performed by using conventional protective groups like those described in Protective groups in Organic Chemistry, ed. J. F. W. McOmie, Plenum Press, 1973; T.W. Greene & P.G.M. Wuts and Protective Groups in Organic Chemistry, John Wiley & sons, 1991. The respective parts of the description is hereby incorporated by reference and forms part of the disclosure. The protective groups may be eliminated when convenient by means well-known to those skilled in the art.

If the 4-substituted pyrazoline compounds of general formula I are obtained in form of a mixture of stereoisomers, particularly enantiomers or diastereomers, said mixtures may be separated by standard procedures known to those skilled in the art, e.g. chromatographic methods or crystallization with chiral reagents. It is also possible to obtain pure stereoisomers via stereoselective synthesis.

In a further aspect the present invention also provides a process for the preparation of salts of 4-substituted pyrazoline compounds of general formula I and stereoisomers thereof, wherein at least one compound of general formula I having at least one basic group is reacted with at least one inorganic and/or organic acid, preferably in the presence of a suitable reaction medium. Suitable reaction media include, for example, any of the ones given above. Suitable inorganic acids include hydrochloric acid, hydrobromic acid, phosphoric acid, sulfuric acid, nitric acid, suitable organic acids are e.g. citric acid, maleic acid, fumaric acid, tartaric acid, or derivatives thereof, p-toluenesulfonic acid, methanesulfonic acid or camphersulfonic acid.

In yet a further aspect the present invention also provides a process for the preparation of salts of 4-substituted pyrazoline compounds of general formula I or stereoisomers thereof, wherein at least one compound of general formula I having at least one acidic group is reacted with one or more suitable bases, preferably in the presence of a suitable reaction medium. Suitable bases are e.g. hydroxides, carbonates or alkoxides, which include suitable cations, derived e.g. from alkaline metals, alkaline earth metals or organic cations, e.g. $[NH_nR_{4-n}]^+$, wherein n is 0, 1, 2, 3 or 4 and R represents a branched or unbranched $C_{1-4}$-alkyl-radical. Suitable reaction media are, for example, any of the ones given above.

In the presence of several acidic or basic groups, mono- or poly-salts may be formed. Compounds of the formula I having an acidic group, for example a free carboxyl group, and a basic group, for example an amino group,

may also be present in the form of inner salts, i.e., in zwitterionic form, or a part of the molecule may be present in the form of an inner salt and another part in the form of a normal salt.

Solvates, preferably hydrates, of the 4-substituted pyrazoline compounds of general formula I, of corresponding stereoisomers, of corresponding N-oxides or of corresponding salts thereof may also be obtained by standard procedures known to those skilled in the art.

4-Substituted pyrazoline compounds of general formula I, which comprise nitrogen-atom containing saturated, unsaturated or aromatic rings may also be obtained in the form of their N-oxides by methods well known to those skilled in the art.

The purification and isolation of the inventive 4-substituted pyrazoline compounds of general formula I, of a corresponding stereoisomer, or salt, or N-oxide, or solvate or any intermediate thereof may, if required, be carried out by conventional methods known to those skilled in the art, e.g. chromatographic methods or recrystallization.

The compounds of general formula I given above may also act as prodrugs, i.e. they represent a drug precusor, which following administration to a patient releases a drug in vivo via some kind of chemical and/or physiological process (e.g., a prodrug on being brought to a physiological pH and/or through an enzyme action is converted to a desired drug form; see, e.g., R. B. Silverman, 1992, "The Organic Chemistry of Drug Design and Drug Action", Academic Press, Chp. 8). In particular, the compounds of general formula I give rise to a compound of general formula I,
wherein $R^3$ represents a —OH moiety, upon administration to a patient.

Prodrugs can be used to alter the biodistribution (e.g., to allow compounds which would not typically enter the reactive site of the protease) or the pharmacokinetics for a particular compound. For example, a hydroxyl group, can be esterified, e.g., with a carboxylic acid group to yield an ester. When the ester is administered to a subject, the ester is cleaved, enzymatically or non-enzymatically, reductively or hydrolytically, to reveal the hydroxyl group.

The 4-substituted pyrazoline compounds of general formula I given above, their stereoisomers, corresponding N-oxides, corresponding salts thereof and corresponding solvates are toxicologically acceptable and are therefore suitable as pharmaceutical active substances for the preparation of medicaments.

It has been found that the 4-substituted pyrazoline compounds of general formula I given above, stereoisomers thereof, N-oxides thereof, corresponding salts and corresponding solvates have a high affinity to cannabinoid receptors, particularly cannabinoid 1 ($CB_1$)-receptors, i.e. they are selective ligands for the ($CB_1$)-receptor and act as modulators, e.g. antagonists, inverse agonists or agonists, on these receptors. In particular, these pyrazoline compounds show little or no development of tolerance during treatment, particularly with respect to food intake, i.e. if the treatment is interrupted for a given period of time and then continued afterwards, the inventively used pyrazoline compounds will again show the desired effect. After ending the treatment with the pyrazoline compounds, the positive influence on the body weight is found to continue.

Furthermore, these 4-substituted pyrazoline compounds show relatively weak Herg channel affinity, thus a low risk of prolongation of the QT-interval is to be expected for these compounds.

In summary, the inventively used 4-subsituted pyrazoline compounds are distinguished by a broad spectrum of beneficial effects, while at the same time showing relatively little undesired effects, i.e. effects which do not positively contribute to or even interfere with the well being of the patient.

Thus, an other aspect of the present invention relates to a medicament comprising at least one 4-substituted pyrazoline compound of general formula I, optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof, and optionally at least one physiologically acceptable auxiliary agent.

Preferably said medicament is suitable for the modulation (regulation) of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferably said medicament is suitable for the prophylaxis and/or treatment of psychosis.

Also particularly preferably said medicament is suitable for the prophylaxis and/or treatment of food intake disorders, preferably bulimia, anorexia, cachexia, obesity and/or type II diabetus mellitus (non-insuline dependent diabetes mellitus), more preferably obesity. The inventive medicament also seems to be active in the prophylaxis and/or treatment of appetency disorders, e.g. the pyrazoline compounds of general formula I also reduce the desire for sweets.

Also particularly preferably said medicament is suitable for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Particularly preferably said medicament is suitable for the prophylaxis and/or treatment of alcohol abuse and/or

alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments and/or drugs, which are frequently the subject of misuse include opioids, barbiturates, cannabis, cocaine, amphetamines, phencyclidine, hallucinogens and benzodiazepines.

The medicament is also suitable for the prophylaxis and/or treatment of one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhoea, asthma, memory disorders, pruritus, pain, or for potentiation of the analgesic effect of narcotic and non-narcotic analgesics, or for influencing intestinal transit.

Another aspect of the present invention is the use of at least one 4-substituted pyrazoline compound of general formula I given above as suitable active substances, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

Particularly preferred is the use of at least one of the respective 4-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of psychosis.

Also particularly preferred is the use of at least one of the respective 4-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of food intake disorders, preferably bulimia, anorexia, cachexia, obesity and/or type II diabetus mellitus (non-insuline dependent diabetes mellitus), more preferably obesity.

Also particularly preferred is the use of at least one of the pyrazoline compounds as defined herein and optionally one or more pharmaceutically acceptable excipients, for the preparation of a medicament for the treatment of metabolic syndrome.

The metabolic syndrome and definitions thereof are described in detail by Eckel et al., The Lancet, Vol. 365 (2005), 1415-1428, included herewith by reference. One of the respective definitions was established by the WHO in 1998 (as described in Alberti et al., Diabet. Med. 1998, 15, pages 539-53, the respective description thereof is herewith incorporated by reference and forms part of the present disclosure). The other, more widely accepted, definition of the metabolic syndrome was established by the Adult Treatment Panel (ATP III) of the US National Cholesterol Education Program (NCEP) in 2001, as described in JAMA 2001; 285; 2486-97, the respective description thereof is herewith incorporated by reference and forms part of the present disclosure.

The metabolic syndrome is characterized by an interaction of several physiological parameters such as triglycerides, lipids, blood pressure, glucose levels and insuline levels.

Even though obesity may play a critical role in the development of metabolic syndrome, many of its aspects are weight independent, especially some lipid parameters. Especially the positive influence on the weight independent aspects of the metabolic syndrome (see e.g. Pagotto and Pasquali, The Lancet, Vol. 365 (2005), 1363, 1364, included herewith by reference) like some blood parameters, especially lipid parameters is one of the major and surprising advantages of the inventively used substituted pyrazoline compounds.

Another aspect of the invention is the use of one or more pyrazoline compounds as defined herein for the manufacture of a medicament for improvent of cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance

Dyslipidemia.

Another aspect of the invention is the use of one or more pyrazoline compounds as defined herein for the manufacture of a medicament for the treatment of the weight independent aspects of metabolic syndrome.

Another aspect of the invention is a method for improving cardiovascular and/or metabolic risk factors, such as one or more of the following factors:

Elevated triglycerides, whereby elevated levels of triglycerides are preferably understood as being > 150 mg/dl,

Low HDL cholesterol, whereby low levels of HDL cholesterol are preferably understood as being < 40 mg/dl in men and < 50 mg/dl in women,

Hypertension, whereby hypertension is preferably understood as being > 130/85 mmHg,

Impaired fasting glucose, whereby impaired fasting glucose levels are preferably understood as being > 110 mg/dl,

Insulin resistance

Dyslipidemia,

in a subject, preferably a human.

Another aspect of the invention is a method for treating of the weight independent aspects of metabolic syndrome.

Also particularly preferred is the use of at least one of the respective 4-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

Also particularly preferred is the use of at least one of the respective 4-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

Medicaments/drugs, which are frequently the subject of misuse include opioids, barbiturates, cannabis, cocaine, amphetamines, phencyclidine, hallucinogens and benzodiazepines.

Also preferred is the use of at least one of the respective 4-substituted pyrazoline compounds, optionally in form of one of the stereoisomers, preferably enantiomers or diastereomers, a racemate or in form of a mixture of at least two of the stereoisomers, preferably enantiomers and/or diastereomers, in any mixing ratio, or a corresponding N-oxide thereof, or a corresponding salt thereof, or a corresponding solvate thereof, and optionally one or more pharmaceutically acceptable excipients, for the preparation of a medicament for the prophylaxis and/or treatment of one or more disorders

selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebella disorders, spinocerebellar disorders, cognitive disorders, cranial trauma, head trauma, stroke, panic attacks, peripheric neuropathy, inflammation, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

The medicament according to the present invention may be in any form suitable for the application to humans and/or animals, preferably humans including infants, children and adults and can be produced by standard procedures known to those skilled in the art. The medicament can be produced by standard procedures known to those skilled in the art, e.g. from the table of contents of "Pharmaceutics: The Science of Dosage Forms", Second Edition, Aulton, M.E. (ED. Churchill Livingstone, Edinburgh (2002); "Encyclopedia of Pharmaceutical Technology", Second Edition, Swarbrick, J. and Boylan J.C. (Eds.), Marcel Dekker, Inc. New York (2002); "Modem Pharmaceutics", Fourth Edition, Banker G.S. and Rhodes C.T. (Eds.) Marcel Dekker, Inc. New York 2002 y "The Theory and Practice of Industrial Pharmacy", Lachman L., Lieberman H. And Kanig J. (Eds.), Lea & Febiger, Philadelphia (1986). The respective descriptions are hereby incorporated by reference and form part of the disclosure. The composition of the medicament may vary depending on the route of administration.

The medicament of the present invention may for example be administered parentally in combination with conventional injectable liquid carriers, such as water or suitable alcohols. Conventional pharmaceutical excipients for injection, such as stabilizing agents, solubilizing agents, and buffers, may be included in such injectable compositions. These medicaments may for example be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, granules, capsules, lozenges, aqueous or oily solutions, suspensions, emulsions, or dry powdered forms suitable for reconstitution with water or other suitable liquid medium before use, for immediate or retarded release. The multiparticulate forms, such as pellets or granules, may e.g. be filled into a capsule, compressed into tablets or suspended in a suitable liquid.

Suitable controlled release formulations, materials and methods for their preparation are known from the prior art, e.g. from the table of contents of "Modified-Release Drug Delivery Technology", Rathbone, M.J. Hadgraft, J. and Roberts, M.S. (Eds.), Marcel Dekker, Inc., New York (2002); "Handbook of Pharmaceutical Controlled Release Technology", Wise, D.L. (Ed.), Marcel Dekker, Inc. New York, (2000); "Controlled Drug Delivery", Vol, I, Basic Concepts, Bruck, S.D. (Ed.), CRD Press Inc., Boca Raton (1983) y de Takada, K. and Yoshikawa, H., "Oral Drug Delivery", Encyclopedia of Controlled Drug Delivery, Mathiowitz, E. (Ed.), John Wiley & Sons, Inc., New York (1999), Vol. 2, 728-742; Fix, J., "Oral drug delivery, small intestine and colon", Encyclopedia of Controlled Drug Delivery, Mathiowitz, E. (Ed.), John Wiley & Sons, Inc., New York (1999), Vol. 2, 698-728. The respective descriptions are hereby incorporated by reference and form part of the disclosure.

Medicaments according to the present invention may also comprise an enteric coating, so that their dissolution is dependent on pH-value. Due to said coating the medicament can pass the stomach undissolved and the respective nitro-subsituted phenyl-piperazine compound is liberated in the intestinal tract. Preferably the enteric coating is soluble at a pH value of 5 to 7.5. Suitable materials and methods for the preparation are known from the prior art.

Typically, the medicaments according to the present invention may contain 1-60 % by weight of one or more 4-substituted pyrazoline compounds as defined herein and 40-99 % by weight of one or more auxiliary substances (additives).

The liquid oral forms for administration may also contain certain additives such as sweeteners, flavoring, preservatives, and emulsifying agents. Non-aqueous liquid compositions for oral administration may also be formulated, containing edible oils. Such liquid compositions may be conveniently encapsulated in e.g., gelatin capsules in a unit dosage amount.

The compositions of the present invention may also be administered topically or via a suppository.

The daily dosage for humans and animals may vary depending on factors that have their basis in the respective species or other factors, such as age, sex, weight or degree of illness and so forth. The daily dosage for humans may preferably be in the range from1 to 2000, preferably 1 to 1500, more preferably 1 to 1000, even more preferably 1 to 150 milligrams of active substance to be administered during one or several intakes per day.

## Pharmacological Methods

### I. In-vitro determination of affinity to CB1/CB2-Receptors

The in-vitro determination of the affinity of the inventive 4-substituted pyrazoline compounds to $CB_1/CB_2$-Rezeptors is carried out as described in the publication of Ruth A. Ross, Heather C. Brockie et al., "Agonist-inverse agonist characterization at CB1 and CB2 cannabinoid receptors of L-759633, L759656 and AM630", British Journal of Pharmacology, 126, 665-672, (1999), whereby the transfected human $CB_1$ and $CB_2$ receptors of Receptor Biology, Inc. are used. The radioligand used for both receptors is [$^3$H]-CP55940. The respective parts of the description is hereby incorporated by reference and forms part of the present disclosure.

### II. In-vivo bioassay system for determination of cannabinoid activity

### Mouse tetrad model

Substances with affinity for cannabinoid receptors are known to produce a wide range of pharmacological effects. It is also known that intravenous administration of a substance with affinity for cannabinoid receptors in mice produces analgesia , hypothermia, sedation and catalepsy. Individually, none of these effects can be considered as proof that a tested substance has affinity for cannabinoid-receptors, since all of these effects are common for various classes of centrally active agents. However, substances, which show all of these effects, i.e. substances that are active in this so-called tetrad model are considered to have affinity for the cannabinoid receptors. It has further been shown that cannabinoid receptor antagonists are higly effective in blocking the effects of a cannabinoid agonist in the mouse tetrad model.

The tetrad model is described, for example, in the publication of A. C. Howlett et al, International Union of Pharmacology XXVII. Classification of Cannabinoid Receptors, Pharmacol Rev 54, 161-202 , 2002 and David R. Compton et al., "In-vivo Characterization of a Specific Cannabinoid Receptor Antagonist (SR141716A) :Inhibition of Tetrahydrocannbinol- induced Responses and Apparent Agonist Activity", J. Pharmacol. Exp. Ther. 277 , 2, 586-594, 1996. The corresponding parts of the description are hereby incorporated by reference.

### Material and Methods

Male NMRI mice with a weight of 20-30 g (Harlan, Barcelona, Spain) are used in all of the following experiments.

Before testing in the behavioral procedures given below, mice are acclimatized to the experimental setting. Pre-Treatment control values are determined for analgesia hot plate latency (in seconds), rectal temperature, sedation and catalepsy.

In order to determine the agonistic activty of the substance to be tested, the mice are injected intravenously with the substance to be tested or the vehicle alone. 15 minutes after injection, latency in hot plate analgesia is measured. Rectal temperature, sedation and catalepsy are measured 20 minutes after injection.

In order to determine the antagonistic activity the identical procedure is used as for the determination of the agonistic effects, but with the difference that the substance to be evaluated for its antagonistic activity is injectected 5 minutes before the intravenous injection of 1.25 mg/kg Win-55,212 a known cannabinoid-receptor agonist.

### Hot plate analgesia

The hot plate analgesia is determined according to the method described in Woolfe D. et al. "The evaluation of analgesic action of pethidine hydrochloride (Demerol)", J. Pharmacol. Exp. Ther. 80, 300-307,1944. The respective description is hereby incorporated by reference and forms part of the present disclosure.

The mice are placed on a hot plate (Harvard Analgesimeter) at 55 $\pm$ 0.5 °C until they show a painful sensation by licking their paws or jumping and the time for these sensations to occur is recorded. This reading is considered the basal value (B). The maximum time limit the mice are allowed to remain on the hot plate in absence of any painful response is 40 seconds in order to prevent skin damage. This period is called the cut-off time (PC).

Fifteen minuts after the administration of the substance to be tested, the mice are again placed on the hot plate and the afore described procedure is repeated. This period is called the post-treatment reading (PT).

The degree of analgesia is calculated from the formula :

$$\% \text{ MPE of Analgesia} = (\text{PT- B}) / (\text{PC-B}) \times 100$$

MPE = Maximum possible effect.

**Determination of sedation and ataxia**

Sedation and ataxia is determined according to the method described in Desmet L. K. C. et al. "Anticonvulsive properties of Cinarizine and Flunarizine in Rats and Mice", Arzneim. -Forsch. (Frug Res) 25, 9, 1975. The respective description is hereby incorporated by reference and forms part of the present disclosure.

The chosen scoring system is

**0**: no ataxia;
**1**: doubful;
**2**: obvious calmness and quiet;
**3** pronounced ataxia;

prior to as well as after treatment.

The percentage of sedation is determined according to the formula:

$$\% \text{ of sedation} = \text{arithmetic mean} / 3 \times 100$$

**Hypothermia:**

Hypothermia is determined according to the method described in David R. Compton et al. "In-vivo Characterization of a Specific Cannabinoid Receptor Antagonist (SR141716A) Inhibition of Tetrahydrocannbinol- induced Responses and Apparent Agonist Activity", J. Pharmacol Exp Ther. 277 , 2, 586-594, 1996. The respective description is hereby incorporated by reference and forms part of the present disclosure.

The base-line rectal temperatures are determined with a thermometer (Yello Springs Instruments Co., Panlabs) and a thermistor probe inserted to 25mm before the administration of the substance to be tested. Rectal temperature is again measured 20 minutes after the administration of the substances to be tested. The temperature difference is calculated for each animal, whereby differences of ≥-2 ˚C are considered to represent activity.

**Catalepsy:**

Catalepsy is determined according to the method described in Alpermann H. G. et al. "Pharmacological effets of Hoe 249: A new potential antidepressant", Drugs Dev. Res. 25, 267-282. 1992. The respective description is hereby incorporated by reference and forms part of the present disclosure.

The cataleptic effect of the substance to be tested is evaluated according to the duration of catalepsy, whereby the animals are placed head downwards with their kinlegs upon the top of the wooden block.

The chosen scoring system is:

Catalepsy for: more than 60 seconds = 6; 50 -60 seconds = 5, 40-50 seconds = 4, 30-40 seconds = 3, 20-30 seconds = 2, 5-10 seconds = 1, and less than 5 seconds =0.

The percentage of catalepsy is determined according ot the following formula:

$$\% \text{ Catalepsy} = \text{arithmetic mean} / 6 \times 100$$

### III. In vivo testing for antiobesic activity

The in-vivo testing for antiobesic activity of the inventive pyrazoline compounds is carried out as described in the publication of G. Colombo et al., "Appetite Suppression and Weight Loss after the Cannabinoid Antagonist SR 141716"; Life Sciences, 63 (8), 113-117, (1998). The respective part of the description is hereby incorporated by reference and forms part of the present disclosure.

### IV. In vivo testing for antidepressant activity

The in-vivo testing for antidepressant activity of the inventive pyrazoline compounds in the water despair test is carried out as described in the publication of E.T. Tzavara et al., "The CB1 receptor antagonist SR141716A selectively increases monoaminergic neurotransmission in the medial prefrontal cortex: implications for therapeutic actions"; Br. J. Pharmacol. 2003, 138(4):544:53. The respective part of the description is hereby incorporated by reference and forms part of the present disclosure.

The present invention is illustrated below with the aid of examples. These illustrations are given solely by way of example and do not limit the general spirit of the present invention.

### Examples:

### Preparation of 4-(4-Chlorophenyl)-3-methyl-2-oxo-but-3-enoic acid

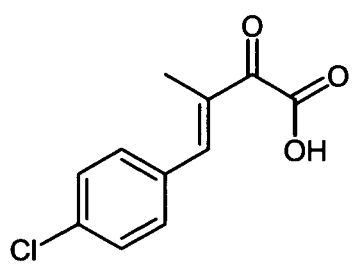

4-Chlorobenzaldehyde (1.4 g, 10 mmol) was dissolved in MeOH (1 mL) and 2-oxobutyric acid (1 g, 9.8 mmol) was subsequently added. A third of a 2 M KOH solution (0.84 g) in MeOH (2.5 mL) was added drop wise at a rate that the temperature of the reaction medium did not rise above 27 °C. The remaining alkaline solution was added quickly causing an increase of the temperature to 42 °C and the yellow colour of the solution turns into orange-red. After approximately five minutes a voluminous and yellow precipitate appeared. After 30 min, water (5 mL) was added and the organic layer was separated. After extraction with *tert*-butyl methyl ether (8 mL), the aqueous phase was acidified with conc. HCl to pH 1 and thoroughly extracted with *tert*-butyl methyl ether (3 x 8 mL). The combined organic layers were washed with aq. NaCl solution, dried with $Na_2SO_4$, and evaporated to dryness to afford a cream-white solid (0.88 g, 40% yield). Recrystallisation of 0.6 g of the crude solid in hexane (40 mL) and ethyl acetate (5 mL) afforded 0.44 g of pure product (80 % recovery).
$^1H$ NMR (400 MHz, $CDCl_3$): $\delta$ 2.17 (3H, s, $CH_3$), 7.44 (4H, d, *J* 3.28 Hz, ArH), 8.41 (1 H, s, CH); $^{13}C$ NMR (100 MHz, $CDCl_3$): $\delta$ 13.2 ($CH_3$), 129.2 (CH), 129.6 (C), 131.9 (CH), 133.4 (C), 136.4 (C), 147.6 (CH), 164.4 (CO), 187.1 (CO)

### Preparation of 5-(4-Chlorophenyl)-1-(2,4-dichlorophenyl)-4-methyl-4,5-dihydro-1H-pyrazole-3-carboxylic acid

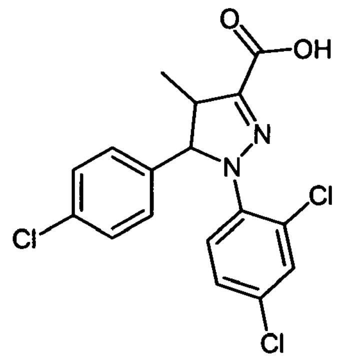

A mixture of 4-(4-chlorophenyl)-3-methyl-2-oxo-but-3-enoic acid (0.72 g, 3.2 mmol) and 2,4-dichlorophenyl hydrazine hydrochloride (0.68 g, 3.2 mmol) in glacial acetic acid (10.6 mL) was heated under nitrogen atmosphere to reflux for 4 h. The crude mixture was allowed to cool down to room temperature and poured into ice. The dark mixture was extracted with dichloromethane, washed with aq. NaCl, dried over $Na_2SO_4$, and evaporated to dryness to yield 1.18 g of crude product (100 % yield) as a mixture of two diastereomers. The ratio of the two diastereomers was determined by NMR spectroscopy to be 80:20.

**Preparation of 5-(4-Chlorophenyl)-1-(2,4-dichlorophenyl)-4-methyl-4,5-dihydro-1H-pyrazole-3-carboxylic acid methyl ester**

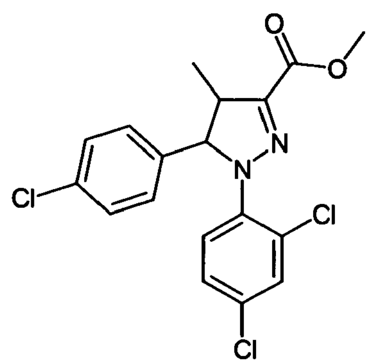

Methyl iodide (0.16 mL, 2.25 mmol) was added drop wise to a mixture of 5-(4-chlorophenyl)-1-(2,4-dichlorophenyl)-4-methyl-4,5-dihydro-1H-pyrazole-3-carboxylic acid (0.47 g, 1.27 mmol) and $KHCO_3$ (0.19 g, 1.9 mmol) in anhydrous dimethylformamide (10 mL) under nitrogen atmosphere. The mixture was stirred at room temperature under nitrogen overnight (ca 16 h). Water was added, the mixture was extracted with ethyl acetate and the combined organic layers were thoroughly washed with aq. NaCl solution. After drying over $Na_2SO_4$ and evaporation under reduced pressure, 0.5 g of the crude product were obtained. Purification by column chromatography ($SiO_2$, 40:1 $SiO_2$, packed with 100% hexane and eluted with a gradient of 1 % to 5% ethyl acetate) affords 0.045 g (9% yield) of the minor isomer (*RS* and *SR* as a racemic mixture, trans isomer) and 0.23 g (48% yield) of the major isomer (*RR* and *SS* as a racemic mixture, cis isomer).

**Example 24: Minor isomer: *trans*-5-(4-Chlorophenyl)-1-(2,4-dichlorophenyl)-4-methyl-4,5-dihydro-1H-pyrazole-3-carboxylic acid methyl ester**

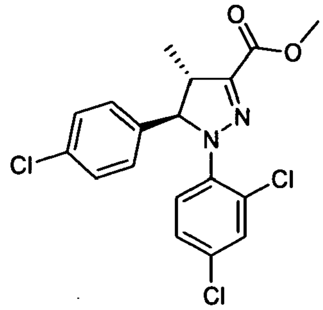

$^1H$ NMR (400 MHz, $CDCl_3$): δ 1.49 (3H, d, *J* 7.6 Hz, $CH_3$), 3.51 (1H, m, CH), 3.90 (3H, s, $OCH_3$), 5.32 (1H, d, *J* 5.6 Hz, CH), 7.03-7.10 (3H, m, ArH), 7.20 (2H, ap d *J* 8.4 Hz, ArH), 7.24-7.28 (2H, m, ArH)
$^{13}C$ NMR (100 MHz, $CDCl_3$): δ 18.4 ($CH_3$), 49.6 (CH), 52.5 ($OCH_3$), 75.5 (CH), 126.2 (CH), 126.7 (C), 127.8 (CH), 128.1 (CH), 129.3 (CH), 130.4 (CH), 134.5 (C), 137.6 (C), 139.1 (C), 145.2 (C), 162.8 (CO)

MS $(M+H)^+$: 398

**Example 23: Major isomer: *cis*-5-(4-Chlorophenyl)-1-(2,4-dichlorophenyl)-4-methyl-4,5-dihydro-1H-pyrazole-3-carboxylic acid methyl ester**

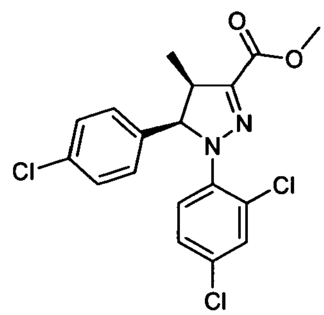

$^1H$ NMR (400 MHz, $CDCl_3$): δ 0.94 (3H, d, *J* 7.2 Hz, $CH_3$), 3.80 (1 H, m, CH), 3.88 (3H, s, $OCH_3$), 5.82 (1 H, d, *J* 11.2 Hz, CH), 7.04-7.10 (3H, m, ArH), 7.22 (2H, m, ArH), 7.24-7.29 (2H, m, ArH)
$^{13}C$ NMR (100 MHz, $CDCl_3$): δ 17.8 ($CH_3$), 49.5 (CH), 52.7 ($OCH_3$), 75.7 (CH), 126.2 (CH), 127.7 (C), 127.9 (CH), 129.4 (CH), 130.4 (CH), 130.8 (C), 134.4 (C), 137.9 (C), 139.0 (C), 144.8 (C), 162.8 (CO)

MS $(M+H)^+$: 398

**Hydrolysis of the racemic mixture of (*RR*)- and (*SS*)-5-(4-Chlorophenyl)-1-(2,4-dichlorophenyl)-4-methyl-4,5-dihydro-1H-pyrazole-3-carboxylic acid methyl ester**

Aq. 2 M NaOH (6.4 mL) was added to a solution of the racemic mixture of (*RR*) and (*SS*)-5-(4-chlorophenyl)-1-(2,4-dichlorophenyl)-4-methyl-4,5-dihydro-1H-pyrazole-3-carboxylic acid methyl ester (0.36 g, 0.9 mmol) in THF (6.4 mL). The mixture was left to stir at room temperature for 3 h (until TLC showed disappearance of the starting material). Water was added and the reaction mixture was concentrated under reduced pressure. The residue was washed once with $Et_2O$ and the aqueous phase acidified to pH 1 with 1 M HCl. Extraction with ethyl acetate, drying with $Na_2SO_4$ and concentration to dryness yielded pure acid (0.23 g, 68% yield).

**Example 19: Major isomer: cis-5-(4-Chloro-phenyl)-1-(2,4-dichloro-phenyl)-4-methyl-4,5-dihydro-1H-pyrazole-3-carboxylic acid**

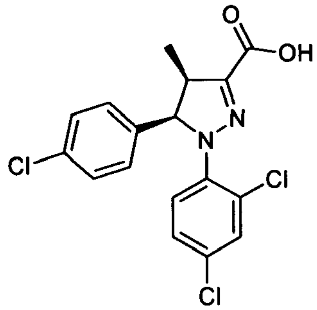

$^{1}$H NMR (400 MHz, CHLOROFORM-*d*) δ ppm 0.96 (d, *J*=7.42 Hz, 3 H) 3.82 (td, *J*=11.72, 7.42 Hz, 1 H) 5.91 (d, *J*=11.72 Hz, 1 H) 7.04 (d, *J*=8.60 Hz, 2 H) 7.11 (dd, *J*=8.60, 2.34 Hz, 1 H) 7.21 - 7.30 (m, 4 H)
$^{13}$C NMR (100 MHz, $CDCl_3$): δ 13.6 ($CH_3$), 43.5 (CH), 72.0 (CH), 124.9 (CH), 127.6 (CH), 129.1 (CH), 129.6 (CH), 130.9 (CH), 131.3 (C), 132.7 (C), 134.5 (C), 138.7 (C), 144.6 (C), 165.9 (CO).

MS $(M+H)^+$: 384

**Hydrolysis of the racemic mixture of (*RS*)- and (*SR*)-5-(4-Chlorophenyl)-1-(2,4-dichlorophenyl)-4-methyl-4,5-dihydro-1H-pyrazole-3-carboxylic acid methyl ester**

The hydrolysis was carried out as described above for the racemic mixture of (*RR*)-and (*SS*)-5-(4-Chlorophenyl)-1-(2,4-dichlorophenyl)-4-methyl-4,5-dihydro-1H-pyrazole-3-carboxylic acid methyl ester

**Example 20: Minor isomer: *trans*-5-(4-Chlorophenyl)-1-(2,4-dichlorophenyl)-4-methyl-4,5-dihydro-1H-pyrazole-3-carboxylic acid**

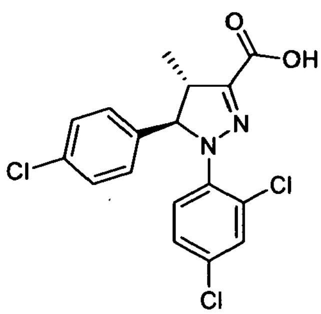

$^{1}$H NMR (400 MHz, CHLOROFORM-*d*) δ ppm 1.52 (d, *J*=7.03 Hz, 3 H) 3.52 (m, 1 H) 5.39 (d, *J*=5.47 Hz, 1 H) 7.08 (ddd, *J*=13.92, 5.62, 5.28 Hz, 3 H) 7.14 - 7.34 (m, 4 H)
$^{13}$C NMR (100 MHz, $CDCl_3$): δ 18.4 ($CH_3$), 48.9 (CH), 76.2 (CH), 126.4 (CH), 126.7 (C), 127.8 (CH), 128.0 (CH), 129.5 (CH), 130.5 (CH), 131.5 (C), 134.7 (C), 137.6 (C), 138.6 (C), 144.2 (C), 166.5 (CO).

MS $(M+H)^+$: 384

**Preparation of 4-(4-Chlorophenyl)-3-methyl-2-oxo-but-3-enoic acid methyl ester**

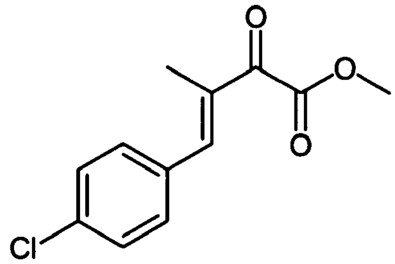

4-(4-Chlorophenyl)-3-methyl-2-oxo-but-3-enoic acid (0.13 g, 0.58 mmol) was dissolved in $Et_2O$ (0.8 mL) and EtOH (1.2 mL) and the solution was cooled down to 0 °C. $TMSCH_2N_2$ (0.43 mL of a 2 M solution in $Et_2O$, 0.87 mmol) was added drop wise and the mixture was stirred at 0 °C for 1 h. A second portion of $TMSCH_2N_2$ (0.2 mL of a 2 M solution in $Et_2O$, 0.40 mmol) was added and the reaction was allowed to reach room temperature. After 30 min, the solvents were evaporated to dryness and the residue was taken up in a mixture of $Et_2O$/hexane 1:1 (10 mL). The organic layer was washed with 2% aq. HCl (10 mL), aq. $NaHCO_3$ (10 mL) and aq. NaCl (10 mL), dried over $Na_2SO_4$ and concentrated to dryness to give pure product (68 mg, 49% yield).

**Preparation of 5-(4-Chlorophenyl)-1-(2,4-dichlorophenyl)-4-methyl-4,5-dihydro-1H-pyrazole-3-carboxylic acid methyl ester**

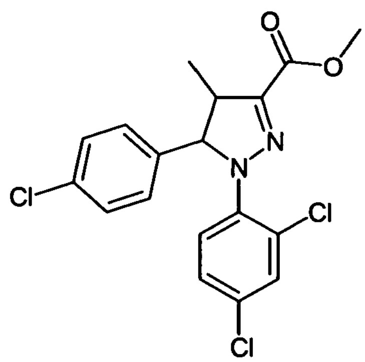

A mixture of 4-(4-chlorophenyl)-3-methyl-2-oxo-but-3-enoic acid methyl ester (0.4 g, 1.68 mmol) and 2,4-dichlorohydrazine hydrochloride (0.356 g, 1.68 mmol) in glacial acetic acid (4 mL) was heated at reflux under nitrogen atmosphere for 4 h. The reaction mixture was then allowed to cool down to room temperature and poured into ice. The dark mixture was extracted with dichloromethane, washed with aq. NaCl, dried over $Na_2SO_4$ and evaporated to dryness to yield the crude product (0.64 g, 95 % yield, as a mixture of two diastereomers). Purification by column chromatography ($SiO_2$, 40:1 $SiO_2$, packed with 100% hexane and eluted with a gradient of 1 % to 5% ethyl acetate) of 0.5 g of crude product afforded 0.051 g of the minor isomer (RS and SR as a racemic mixture) and 0.20 g of the major isomer (RR and SS as racemic mixture).

**Preparation of Example 1: cis-5-(4-Chloro-phenyl)-1-(2,4-dichlorophenyl)-4-methyl-4,5-dihydro-1H-pyrazole-3-carboxylic acid piperidin-1-ylamide** Hydrochloride

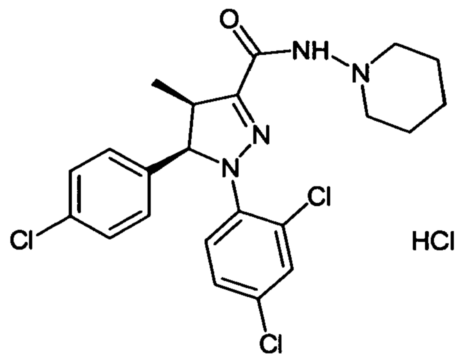

5-(4-Chlorophenyl)-1-(2,4-dichlorophenyl)-4-methyl-4,5-dihydro-1H-pyrazole-3-carboxylic acid (0.160 g, 0.417 mmol) was dissolved in toluene (4 mL) and was partially distilled at atmospheric pressure to eliminate water traces. The resulting solution was cooled to 75-85 °C. DMF was added (2 drops) as a catalyst and thionyl chloride (0.060 mL, 0.5 mmol) was added drop by drop. The solution was stirred until formation of the acid chloride was complete (monitoring by IR). The mixture was cooled to 20 - 25 °C and was added drop wise to a solution of 1-aminopiperidine (0.053 mL, 0.500 mmol), dichloromethane (5 mL) and N,N-diisopropylethylamine (0.170 mL, 1 mmol) under nitrogen at a temperature between 5 - 10 °C. After complete addition the mixture was warmed to 20 - 25 °C and stirred overnight. The organic layer was washed with water (3 x 5 mL), a sat. $NaHCO_3$ solution (3 x 25 mL) and again with water (3 x 5 mL). The combined organic layers were dried with $Na_2SO_4$, filtered and concentrated under vacuum to give a solid (130 mg, 68 % yield).

The crude solid was dissolved in ethyl acetate and a 2.8 M HCl solution in ethanol was added drop wise to form the hydrochloride which was obtained as a beige solid (82 mg, 49 % yield).

$^1$H NMR (400 MHz, METHANOL-$d_4$) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H)

MS $(M+H)^+$: 466

**Example 2: trans-5-(4-Chloro-phenyl)-1-(2,4-dichlorophenyl)-4-methyl-4,5-dihydro-1H-pyrazole-3-carboxylic acid piperidin-1-ylamide Hydrochloride**

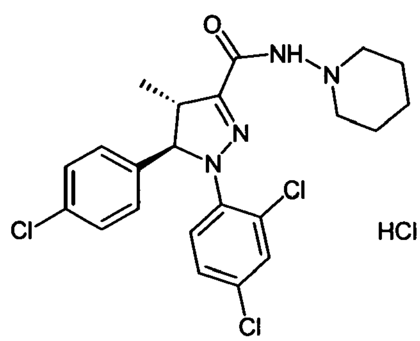

5-(4-Chlorophenyl)-1-(2,4-dichlorophenyl)-4-methyl-4,5-dihydro-1H-pyrazole-3-carboxylic acid (0.190 g, 0.50 mmol) was dissolved in toluene (4 mL) and was partially distilled at atmospheric pressure to eliminate water traces. The resulting solution was cooled to 75-85 °C. DMF was added (2 drops) as a catalyst and thionyl chloride (0.060 mL, 0.5 mmol) was added drop by drop. The solution was stirred until formation of the acid chloride was complete (monitoring by IR). The mixture was cooled to 20 - 25 °C and was added drop wise to a solution of 1-aminopiperidine (0.0623 mL, 0.57 mmol), dichloromethane (5 mL) and N,N-diisopropylethylamine (0.197 mL, 1.15 mmol) under nitrogen at a temperature between 5 - 10 °C. After complete addition the mixture was warmed to 20 - 25 °C and stirred overnight. The organic layer was washed with water (3 x 5 mL), a sat. $NaHCO_3$ solution (3 x 25 mL) and again with water (3 x 5 mL).

The combined organic layers were dried with $Na_2SO_4$, filtered and concentrated under vacuum to give a solid. The crude solid was dissolved in ethyl acetate and a 2.8 M HCl solution in ethanol was added drop wise to form the hydrochloride which was obtained as a beige solid (120 mg, 50 % yield).

$^1$H NMR (400 MHz, DMSO-$d_6$) δ ppm 1.38 (d, *J*=7.03 Hz, 3 H) 1.42 (m, 2 H) 1.71 (m, 4 H) 3.12 (m, 4 H) 3.42 (dt, *J*=13.38, 5.86 Hz, 1 H) 5.36 (d, *J*=5.86 Hz, 1 H) 7.19 (d, J=8.21 Hz, 2 H) 7.31 (d, *J*=8.60 Hz, 3 H) 7.49 (d, *J*=2.34 Hz, 1 H) 7.53 (d, *J*=8.60 Hz, 1 H) 10.49 (s, 1 H)

MS $(M+H)^+$: 466

The following compounds were prepared according to the processes described above. Those skilled in the art are familiar with the starting materials that are needed to obtain said compounds.

| N° | STRUCTURE | Name | $^1$H-NMR | MS $(M+H)^+$ |
|---|---|---|---|---|
| 1 | 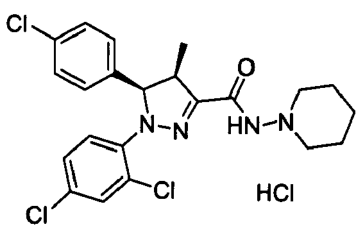 HCl | *cis*-5-(4-Chloro-phenyl)-1-(2,4-dichloro-phenyl)-4-methyl-4,5-dihydro-1H-pyrazole-3-carboxylic acid piperidin-1-ylamide hydro-chloride | 1H NMR (400 MHz, METHANOL-$d_4$) δ ppm 0.94 (d, *J*=7.42 Hz, 3 H) 1.68 (br, 2 H) 1.98 (dt, *J*=11.28, 5.59 Hz, 4 H) 3.53 (br, 4 H) 3.90 (td, *J*=11.53, 7.42 Hz, 1 H) 5.99 (d, *J*=11.53 Hz, 1 H) 7.16 (d, *J*=8.40 Hz, 2 H) 7.20 - 7.29 (m, 3 H) 7.36 - 7.48 (m, 2 H) | 466 |
| 2 | 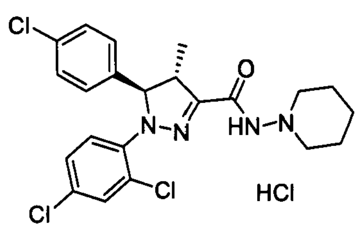 HCl | *trans*-5-(4-Chloro-phenyl)-1-(2,4-dichloro-phenyl)-4-methyl-4,5-dihydro-1H-pyrazole-3-carboxylic acid piperidin-1-ylamide hydro-chloride | $^1$H NMR (400 MHz, DMSO-$d_6$) δ ppm 1.38 (d, *J*=7.03 Hz, 3 H) 1.42 (m, 2 H) 1.71 (m, 4 H) 3.12 (m, 4 H) 3.42 (dt, *J*=13.38, 5.86 Hz, 1 H) 5.36 (d, *J*=5.86 Hz, 1 H) 7.19 (d, *J*=8.21 Hz, 2 H) 7.31 (d, *J*=8.60 Hz, 3 H) 7.49 (d, *J*=2.34 Hz, 1 H) 7.53 (d, *J*=8.60 Hz, 1 H) 10.49 (s, 1 H) | 466 |
| 3 | 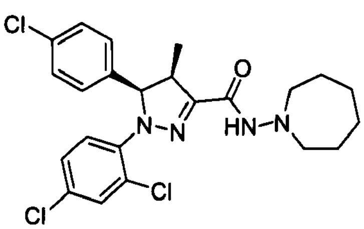 | *cis*-5-(4-Chloro-phenyl)-1-(2,4-dichloro-phenyl)-4-methyl-4,5-dihydro-1H-pyrazole-3-carboxylic acid azepan-1-ylamide | | |
| 4 | 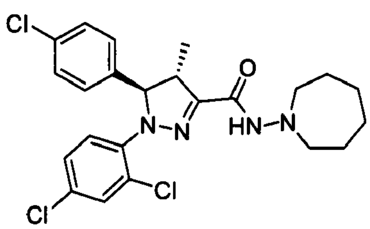 | *trans*-5-(4-Chloro-phenyl)-1-(2,4-dichloro-phenyl)-4-methyl-4,5-dihydro-1H-pyrazole-3-carboxylic acid azepan-1-ylamide | | |

(continued)

| N° | STRUCTURE | Name | $^1$H-NMR | MS (M+H)$^+$ |
|---|---|---|---|---|
| 5 | 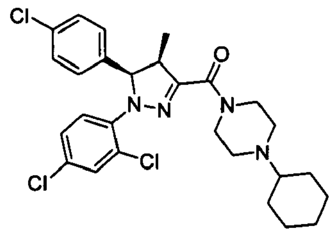 | *cis*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone | | |
| 6 | 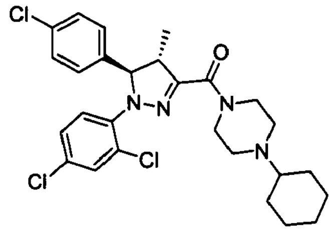 | *trans*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone | | |
| 7 | 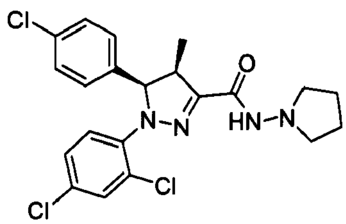 | *cis*-5-(4-Chloro-phenyl)-1-(2,4-dichloro-phenyl)-4-methyl-4,5-dihydro-1H-pyrazole-3-carboxylic acid pyrrolidin-1-ylamide | | |
| 8 | 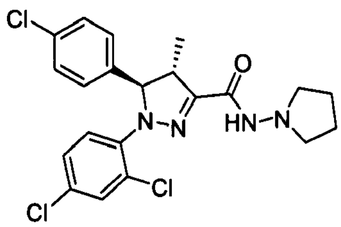 | *trans*-5-(4-Chloro-phenyl)-1-(2,4-dichloro-phenyl)-4-methyl-4,5-dihydro-1H-pyrazole-3-carboxylic acid pyrrolidin-1-ylamide | | |
| 9 | 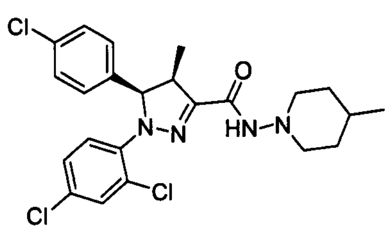 | *cis*-5-(4-Chloro-phenyl)-1-(2,4-dichloro-phenyl)-4-methyl-4,5-dihydro-1H-pyrazole-3-carboxylic acid (4-methyl-piperidin-1-yl)-amide | | |
| 10 | 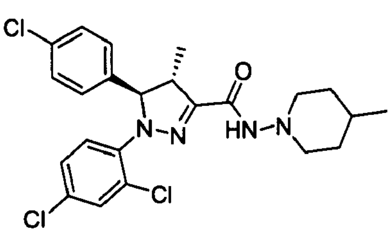 | *trans*-5-(4-Chloro-phenyl)-1-(2,4-dichloro-phenyl)-4-methyl-4,5-dihydro-1H-pyrazole-3-carboxylic acid (4-methyl-piperidin-1-yl)-amide | | |

(continued)

| N° | STRUCTURE | Name | $^1$H-NMR | MS (M+H)$^+$ |
|---|---|---|---|---|
| 11 | 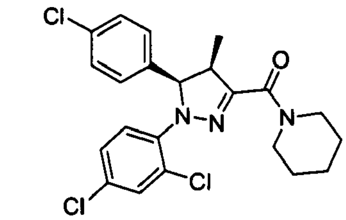 | *cis*-[5-(4-Chloro-phenyl)-1-(2,4-dichloro-phenyl)-4-methyl-4,5-dihydro-1H-pyrazol-3-yl]-piperidin-1-yl-methanone | | |
| 12 | 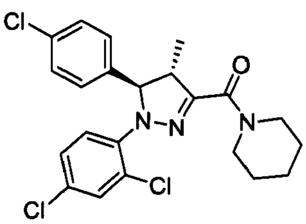 | *trans*-[5-(4-Chloro-phenyl)-1-(2,4-dichloro-phenyl)-4-methyl-4,5-dihydro-1H-pyrazol-3-yl]-piperidin-1-yl-methanone | | |
| 13 | 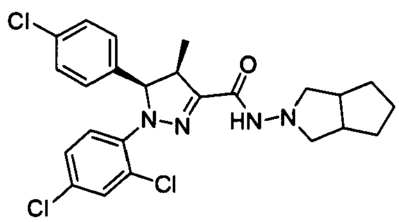 | *cis*-5-(4-Chloro-phenyl)-1-(2,4-dichloro-phenyl)-4-methyl-4,5-dihydro-1H-pyrazole-3-carboxylic acid (hexahydro-cyclopenta[c]pyrrol-2-yl)-amide | | |
| 14 | 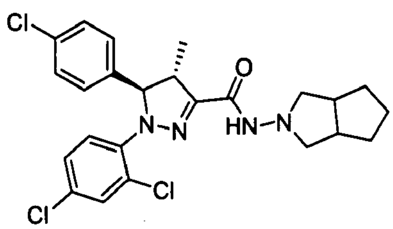 | *trans*-5-(4-Chloro-phenyl)-1-(2,4-dichloro-phenyl)-4-methyl-4,5-dihydro-1H-pyrazole-3-carboxylic acid (hexahydro-cyclopenta-[c]pyrrol-2-yl)-amide | | |
| 15 | 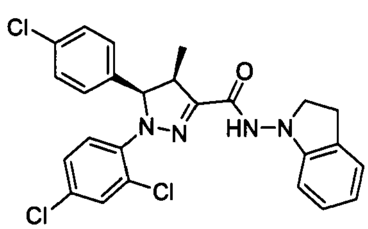 | *cis*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide | | |
| 16 | 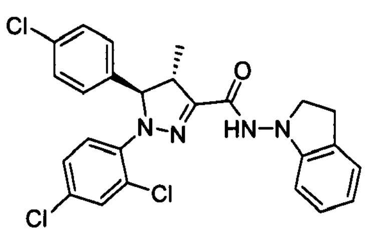 | *trans*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide | | |

(continued)

| N° | STRUCTURE | Name | $^1$H-NMR | MS (M+H)$^+$ |
|---|---|---|---|---|
| 17 | 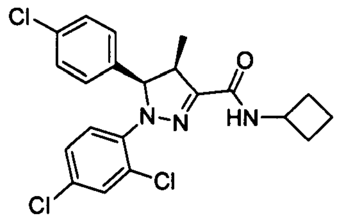 | *cis*-5-(4-Chloro-phenyl)-1-(2,4-dichloro-phenyl)-4-methyl-4,5-dihydro-1H-pyrazole-3-carboxylic acid cyclobutylamide | | |
| 18 | 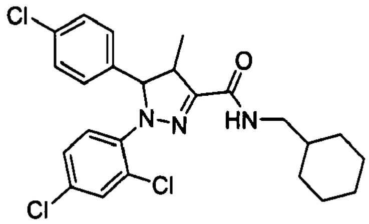 | 5-(4-Chloro-phenyl)-1-(2,4-dichloro-phenyl)-4-methyl-4,5-dihydro-1H-pyrazole-3-carboxylic acid cyclohexyl methyl-amide | | |
| 19 | 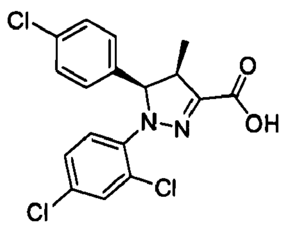 | *cis*-5-(4-Chloro-phenyl)-1-(2,4-dichloro-phenyl)-4-methyl-4,5-dihydro-1H-pyrazole-3-carboxylic acid | 1H NMR (400 MHz, CHLOROFORM-*d*) δ ppm 0.96 (d, *J*=7.42 Hz, 3H) 3.82 (td, *J*=11.72, 7.42 Hz, 1 H) 5.91 (d, *J*=11.72 Hz, 1 H) 7.04 (d, *J*=8.60 Hz, 2 H) 7.11 (dd, *J*=8.60,2.34 Hz, 1 H) 7.21 - 7.30 (m, 4 H) | 384 |
| 20 | 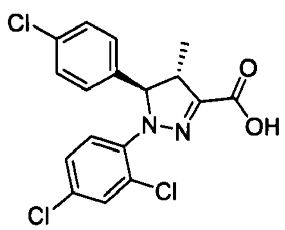 | *trans*-5-(4-Chloro-phenyl)-1-(2,4-dichloro-phenyl)-4-methyl-4,5-dihydro-1H-pyrazole-3-carboxylic acid | 1 H NMR (400 MHz, CHLOROFORM-*d*) δ ppm 1.52 (d, *J*=7.03 Hz, 3 H) 3.52 (m, 1 H) 5.39 (d, *J*=5.47 Hz, 1 H) 7.08 (ddd, *J*=13.92, 5.62, 5.28 Hz, 3 H) 7.14 - 7.34 (m, 4 H) | 384 |
| 21 | 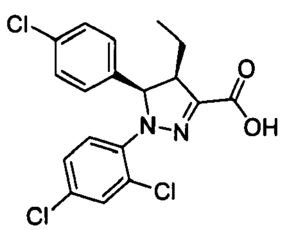 | *cis*-5-(4-Chloro-phenyl)-1-(2,4-dichloro-phenyl)-4-ethyl-4,5-dihydro-1H-pyrazole-3-carboxylic acid | | |
| 22 | 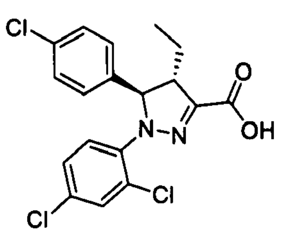 | *trans*-5-(4-Chloro-phenyl)-1-(2,4-dichloro-phenyl)-4-ethyl-4,5-dihydro-1H-pyrazole-3-carboxylic acid piperidin-1-ylamide | | |

(continued)

| N° | STRUCTURE | Name | $^1$H-NMR | MS (M+H)$^+$ |
|---|---|---|---|---|
| 23 | 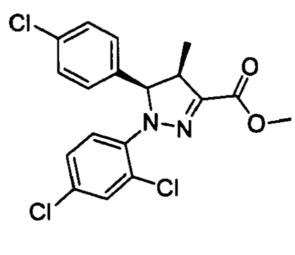 | *cis*-5-(4-Chloro-phenyl)-1-(2,4-dichloro-phenyl)-4-methyl-4,5- dihydro-1H-pyrazole-3-carboxylic acid methyl ester | 1H NMR (400 MHz, CHLOROFORM-*d*) δ ppm 0.94 (d, *J*=7.32 Hz, 3 H) 3.80 (td, *J*=11.48, 7.32 Hz, 1H) 3.88 (s, 3 H) 5.82 (d, *J*=11.48 Hz, 1H) 7.04 (m, 2 H) 7.10 (dd, *J*=8.67, 2.32 Hz, 1H) 7.20 - 7.24 (m, 3 H) 7.27 (m, 1 H) | 398 |
| 24 | 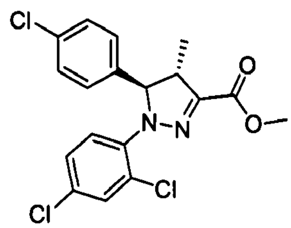 | *trans*-5-(4-Chloro-phenyl)-1-(2,4-dichloro-phenyl)-4-methyl-4,5-dihydro-1H-pyrazole-3-carboxylic acid methyl ester | 1H NMR (400 MHz, CHLOROFORM-*d*) δ ppm 1.49 (d, *J*=7.32 Hz, 3 H) 3.50 (td, *J*=7.32 Hz, 5.60 1H) 3.90 (s, 3 H) 5.35 (d, *J*=5.60 Hz, 1 H) 7.04 (m, 2 H) 7.10 (m, 1 H) 7.20-7.27 (m, 4 H) | 398 |
| 25 | 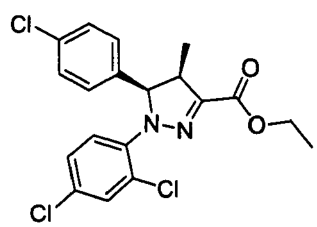 | *cis*-5-(4-Chloro-phenyl)-1-(2,4-dichloro-phenyl)-4-methyl-4,5-dihydro-1H-pyrazole-3-carboxylic acid ethyl ester | | |
| 26 | 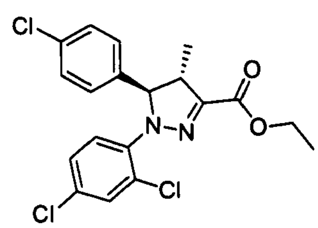 | *trans*-5-(4-Chloro-phenyl)-1-(2,4-dichloro-phenyl)-4-methyl-4,5-dihydro-1H-pyrazole-3-carboxylic acid ethyl ester | | |
| 27 | 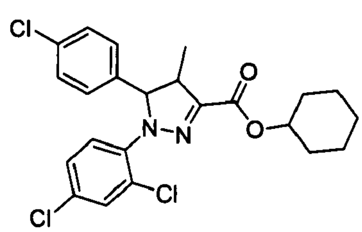 | 5-(4-Chloro-phenyl)-1-(2,4-dichloro-phenyl)-4-methyl-4,5-dihydro-1H-pyrazole-3-carboxylic acid cyclohexyl ester | | |
| 28 | 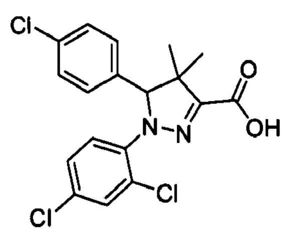 | 5-(4-Chloro-phenyl)-1-(2,4-dichloro-phenyl)-4,4-dimethyl-4,5-dihydro-1H-pyrazole-3-carboxylic acid | | |

(continued)

| N° | STRUCTURE | Name | $^1$H-NMR | MS (M+H)$^+$ |
|---|---|---|---|---|
| 29 | 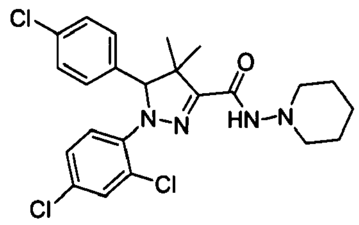 | 5-(4-Chloro-phenyl)-1-(2,4-dichloro-phenyl)-4,4-dimethyl-4,5-dihydro-1H-pyrazole-3-carboxylic acid piperidin-1-ylamide | | |
| 30 | 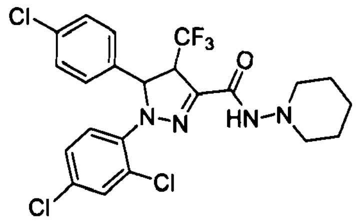 | 5-(4-Chloro-phenyl)-1-(2,4-dichloro-phenyl)-4-trifluoromethyl-4,5-dihydro-1 H-pyrazole-3-carboxylic acid piperidin-1-ylamide | | |
| 31 | 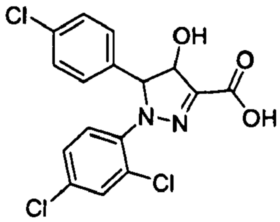 | 5-(4-Chloro-phenyl)-1-(2,4-dichloro-phenyl)-4-hydroxy-4,5-dihydro-1H-pyrazole-3-carboxylic acid | | |
| 32 | 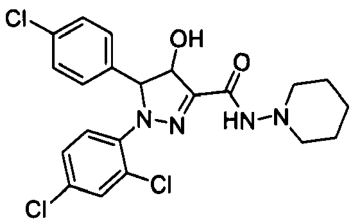 | 5-(4-Chloro-phenyl)-1-(2,4-dichloro-phenyl)-4-hydroxy-4,5-dihydro-1H-pyrazole-3-carboxylic acid piperidin-1-ylamide | | |
| 33 | 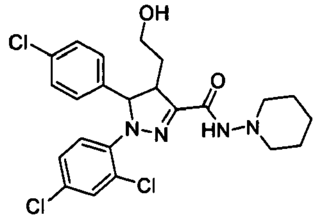 | 5-(4-Chloro-phenyl)-1-(2,4-dichloro-phenyl)-4-(2-hydroxy-ethyl)-4,5-di-hydro-1H-pyrazole-3-carboxylic acid piperidin-1-ylamide | | |
| 34 | 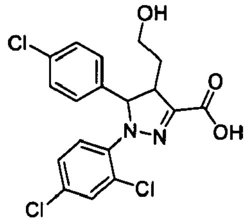 | 5-(4-Chloro-phenyl)-1-(2,4-dichloro-phenyl)-4-(2-hydroxy-ethyl)-4,5-di-hydro-1H-pyrazole-3-carboxylic acid | | |
| 35 | 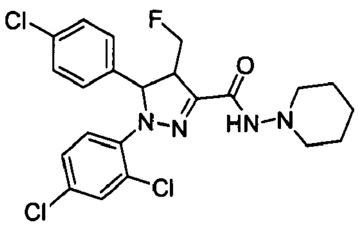 | 5-(4-Chloro-phenyl)-1-(2,4-dichloro-phenyl)-4-fluoromethyl-4,5-dihydro-1H-pyrazole-3-carboxylic acid piperidin-1-ylamide | | |

(continued)

| N° | STRUCTURE | Name | $^1$H-NMR | MS (M+H)$^+$ |
|---|---|---|---|---|
| 36 | 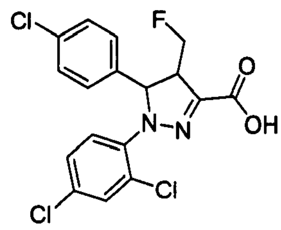 | 5-(4-Chloro-phenyl)-1-(2,4-dichloro-phenyl)-4-fluoromethyl-4,5-dihydro-1H-pyrazole-3-carboxylic acid | | |
| 37 | 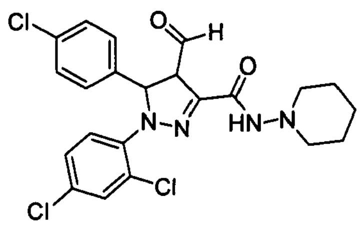 | 5-(4-Chloro-phenyl)-1-(2,4-dichloro-phenyl)-4-formyl-4,5-dihydro-1H-pyrazole-3-carboxylic acid piperidin-1-ylamide | | |
| 38 | 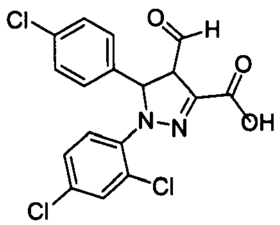 | 5-(4-Chloro-phenyl)-1-(2,4-dichloro-phenyl)-4-formyl-4,5-dihydro-1H-pyrazole-3-carboxylic acid | | |
| 39 | 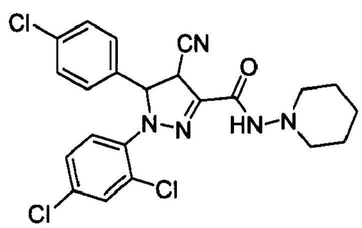 | 1-(2,4-Dichloro-phenyl)-5-(4-fluoro-phenyl)-4-methyl-4,5-dihydro-1H-pyrazole-3-carboxylic acid piperidin-1-ylamide | | |
| 40 | 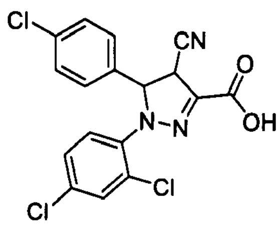 | 5-(4-Chloro-phenyl)-4-cyano-1-(2,4-dichloro-phenyl)-4,5-dihydro-1H-pyrazole-3-carboxylic acid | | |
| 41 | 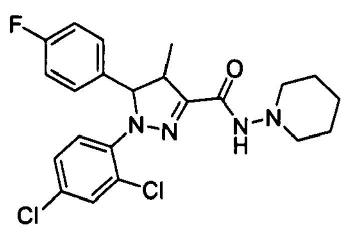 | 1-(2,4-Dichloro-phenyl)-5-(4-fluoro-phenyl)-4-methyl-4,5-dihydro-1H-pyrazole-3-carboxylic acid piperidin-1-ylamide | | |
| 42 | 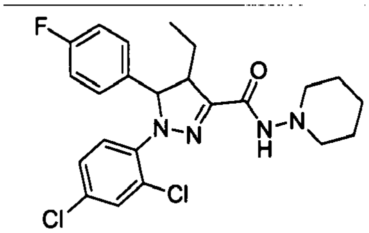 | 1-(2,4-Dichloro-phenyl)-4-ethyl-5-(4-fluoro-phenyl)-4,5-dihydro-1H-pyrazole-3-carboxylic acid piperidin-1-ylamide | | |

(continued)

| N° | STRUCTURE | Name | 1H-NMR | MS (M+H)+ |
|---|---|---|---|---|
| 43 | 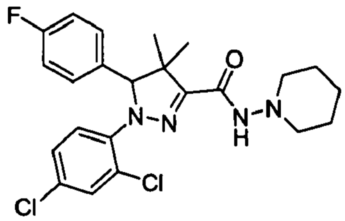 | 1-(2,4-Dichloro-phenyl)-5-(4-fluoro-phenyl)-4,4-dimethyl-4,5-dihydro-1H-pyrazole-3-carboxylic acid piperidin-1-ylamide | | |
| 44 | 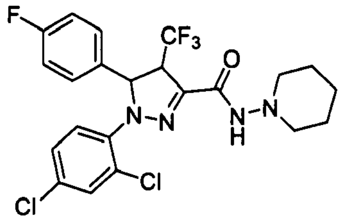 | 1-(2,4-Dichloro-phenyl)-5-(4-fluoro-phenyl)-4-trifluoromethyl-4,5-dihydro-1H-pyrazole-3-carboxylic acid piperidin-1-ylamide | | |
| 45 | 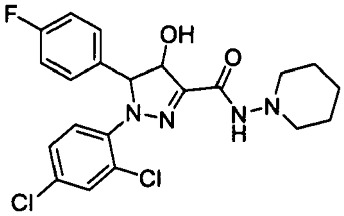 | 1-(2,4-Dichloro-phenyl)-5-(4-fluoro-phenyl)-4-hydroxy-4,5-dihydro-1H-pyrazole-3-carboxylic acid piperidin-1-ylamide | | |
| 46 | 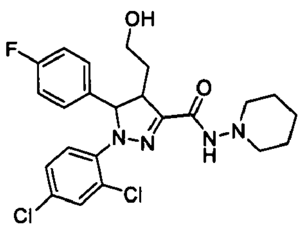 | 1-(2,4-Dichloro-phenyl)-5-(4-fluoro-phenyl)-4-(2-hydroxy-ethyl)-4,5-di-hydro-1H-pyrazole-3-carboxylic acid piperidin-1-ylamide | | |
| 47 | 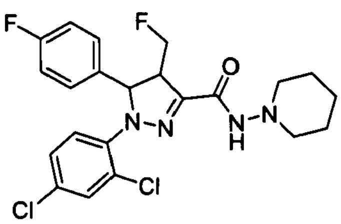 | 1-(2,4-Dichloro-phenyl)-4-fluoromethyl-5-(4-fluoro-phenyl)-4,5-dihydro-1H-pyrazole-3-carboxylic acid piperidin-1-ylamide | | |
| 48 | 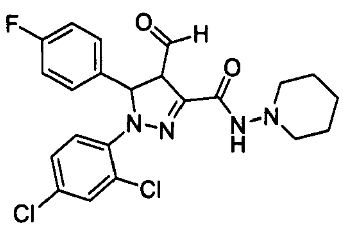 | 1-(2,4-Dichloro-phenyl)-5-(4-fluoro-phenyl)-4-formyl-4,5-dihydro-1H-pyrazole-3-carboxylic acid piperidin-1-ylamide | | |

(continued)

| N° | STRUCTURE | Name | $^1$H-NMR | MS (M+H)$^+$ |
|---|---|---|---|---|
| 49 | 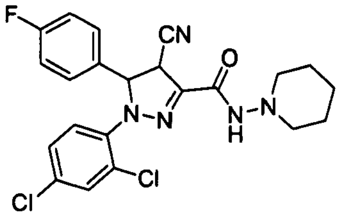 | 4-Cyano-1-(2,4-dichloro-phenyl)-5-(4-fluoro-phenyl)-4,5-dihydro-1H-pyrazole-3-carboxylic acid piperidin-1-ylamide | | |
| 50 | 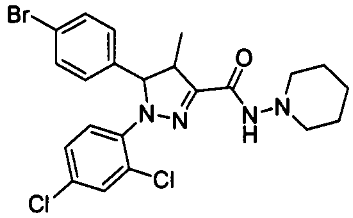 | 5-(4-Bromo-phenyl)-1-(2,4-dichloro-phenyl)-4-methyl-4,5-dihydro-1H-pyrazole-3-carboxylic acid piperidin-1-ylamide | | |
| 51 | 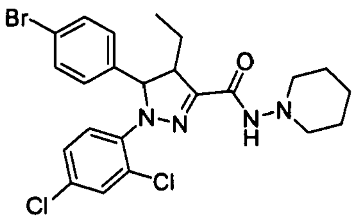 | 5-(4-Bromo-phenyl)-1-(2,4-dichloro-phenyl)-4-ethyl-4,5-dihydro-1H-pyrazole-3-carboxylic acid piperidin-1-ylamide | | |
| 52 | 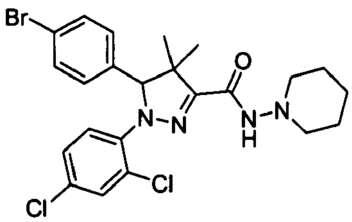 | 5-(4-Bromo-phenyl)-1-(2,4-dichloro-phenyl)-4,4-dimethyl-4,5-dihydro-1 H-pyrazole-3-carboxylic acid piperidin-1-ylamide | | |
| 53 | 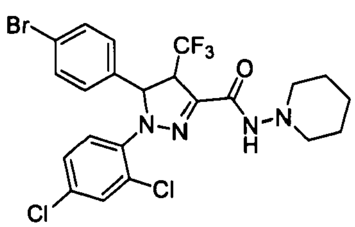 | 5-(4-Bromo-phenyl)-1-(2,4-dichloro-phenyl)-4-trifluoromethyl-4,5-dihydro-1H-pyrazole-3-carboxylic acid piperidin-1-ylamide | | |
| 54 | 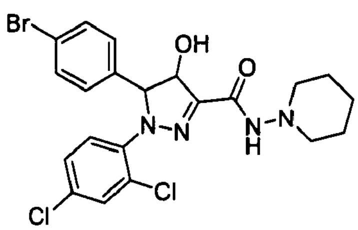 | 5-(4-Bromo-phenyl)-1-(2,4-dichloro-phenyl)-4-hydroxy-4,5-dihydro-1H-pyrazole-3-carboxylic acid piperidin-1-ylamide | | |
| 55 | 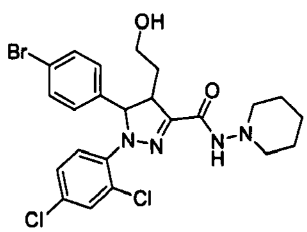 | 5-(4-Bromo-phenyl)-1-(2,4-dichloro-phenyl)-4-(2-hydroxy-ethyl)-4,5-dihydro-1H-pyrazole-3-carboxylic acid piperidin-1-ylamide | | |

(continued)

| N° | STRUCTURE | Name | $^1$H-NMR | MS (M+H)$^+$ |
|---|---|---|---|---|
| 56 | 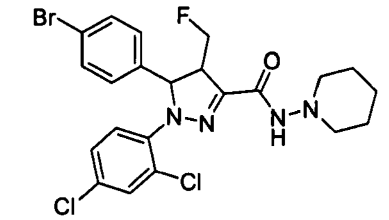 | 5-(4-Bromo-phenyl)-1-(2,4-dichloro-phenyl)-4-fluoromethyl-4,5-dihydro-1H-pyrazole-3-carboxylic acid piperidin-1-ylamide | | |
| 57 | 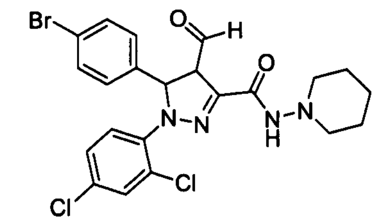 | 5-(4-Bromo-phenyl)-1-(2,4-dichloro-phenyl)-4-formyl-4,5-dihydro-1H-pyrazole-3-carboxylic acid piperidin-1-ylamide | | |
| 58 | 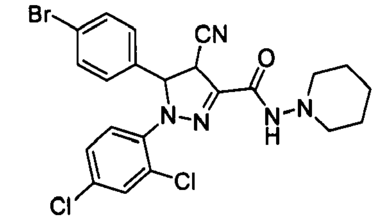 | 5-(4-Bromo-phenyl)-4-cyano-1-(2,4-dichloro-phenyl)-4,5-dihydro-1H-pyrazole-3-carboxylic acid piperidin-1-ylamide | | |
| 59 | 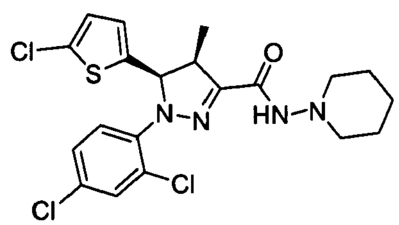 | *cis*-5-(5-Chloro-thiophen-2-yl)-1-(2,4-dichloro-phenyl)-4-methyl-4,5-dihydro-1H-pyrazole-3-carboxylic acid piperidin-1-ylamide | | |
| 60 | 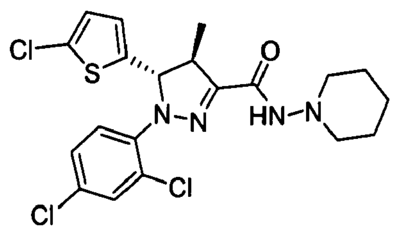 | *trans*-5-(5-Chloro-thiophen-2-yl)-1-(2,4-dichloro-phenyl)-4-methyl-4,5-dihydro-1H-pyrazole-3-carboxylic acid piperidin-1-ylamide | | |
| 61 | 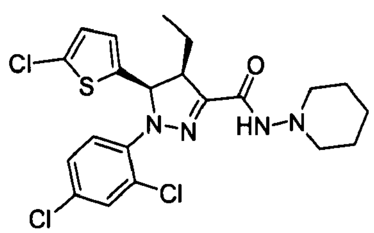 | *cis*-5-(5-Chloro-thiophen-2-yl)-1-(2,4-dichloro-phenyl)-4-ethyl-4,5-dihydro-1H-pyrazole-3-carboxylic acid piperidin-1-yiamide | | |

(continued)

| N° | STRUCTURE | Name | 1H-NMR | MS (M+H)+ |
|---|---|---|---|---|
| 62 | 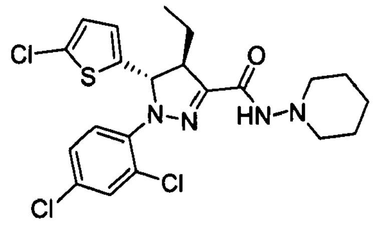 | *trans*-5-(5-Chloro-thiophen-2-yl)-1-(2,4-dichloro-phenyl)-4-ethyl-4,5-dihydro-1H-pyrazole-3-carboxylic acid piperidin-1-ylamide | | |
| 63 | 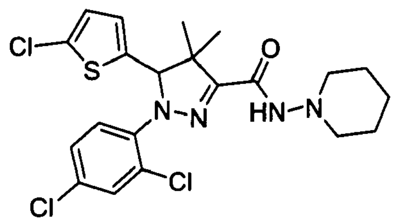 | 5-(5-Chloro-thiophen-2-yl)-1-(2,4-d ichloro-phenyl)-4,4-dimethyl-4,5-di hydro-1H-pyrazole-3-carboxylic acid piperidin-1-ylamide | | |
| 64 | 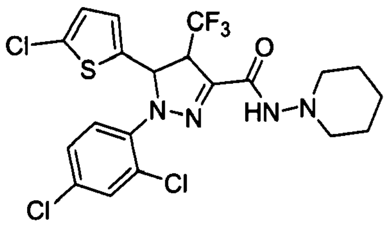 | 5-(5-Chloro-thiophen-2-yl)-1-(2,4-dichloro-phenyl)-4-trifluoromethyl-4,5-dihydro-1H-pyrazole-3-carboxylic acid piperidin-1-ylamide | | |
| 65 | 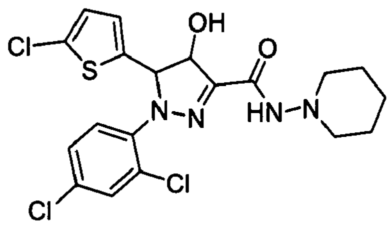 | 5-(5-Chloro-thiophen-2-yl)-1-(2,4-dichloro-phenyl)-4-hydroxy-4,5-dihydro-1H-pyrazole-3-carboxylic acid piperidin-1-yiamide | | |
| 66 | 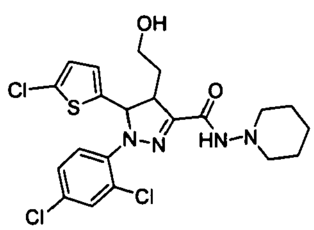 | 5-(5-Chloro-thiophen-2-yl)-1-(2,4-dichloro-phenyl)-4-(2-hydroxy-ethyl)-4,5-dihydro-1H-pyrazole-3-carboxylic acid piperidin-1-ylamide | | |
| 67 | 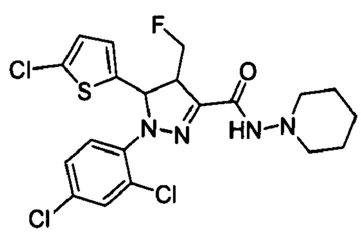 | 5-(5-Chloro-thiophen-2-yl)-1-(2,4-dichloro-phenyl)-4-fluoromethyl-4,5-dihydro-1H-pyrazole-3-carboxylic acid piperidin-1-ylamide | | |

(continued)

| N° | STRUCTURE | Name | $^1$H-NMR | MS (M+H)$^+$ |
|---|---|---|---|---|
| 68 | 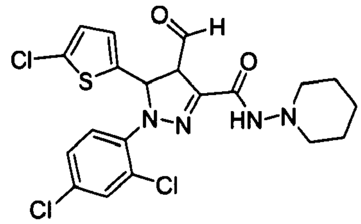 | 5-(5-Chloro-thiophen-2-yl)-1-(2,4-dichloro-phenyl)-4-formyl-4,5-dihydro-1H-pyrazole-3-carboxylic acid piperidin-1-ylamide | | |
| 69 | 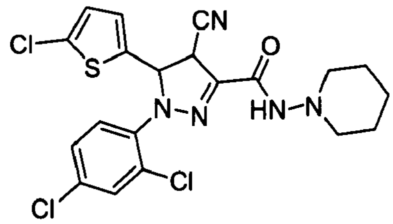 | 5-(5-Chloro-thiophen-2-yl)-4-cyano-1-(2,4-dichloro-phenyl)-4,5-dihydro-1H-pyrazole-3-carboxylic acid piperidin-1-ylamide | | |
| 70 | 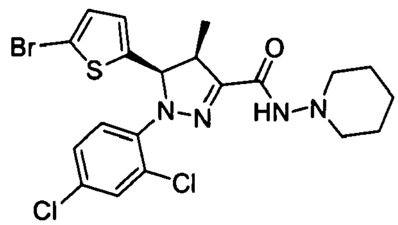 | *cis*-5-(5-Bromo-thiophen-2-yl)-1-(2,4-dichloro-phenyl)-4-methyl-4,5-dihydro-1H-pyrazole-3-carboxylic acid piperidin-1-ylamide | | |
| 71 | 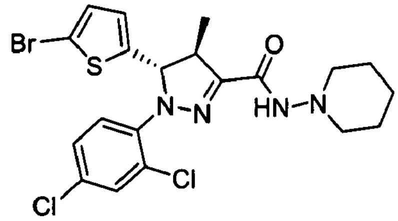 | *trans*-5-(5-Bromo-thiophen-2-yl)-1-(2,4-dichloro-phenyl)-4-methyl-4,5-dihydro-1H-pyrazole-3-carboxylic acid piperidin-1-ylamide | | |
| 72 | 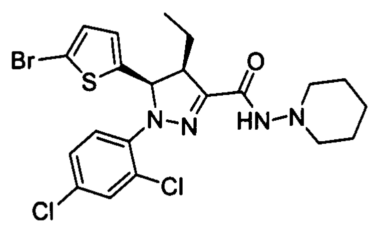 | *cis*-5-(5-Bromo-thiophen-2-yl)-1-(2,4-dichloro-phenyl)-4-ethyl-4,5-dihydro-1H-pyrazole-3-carboxylic acid piperidin-1-yiamide | | |
| 73 | 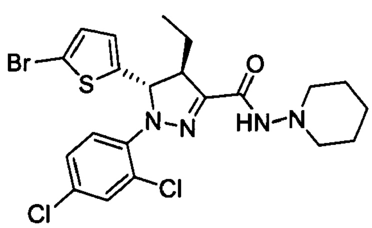 | *trans*-5-(5-Bromo-thiophen-2-yl)-1-(2,4-dichloro-phenyl)-4-ethyl-4,5-dihydro-1H-pyrazole-3-carboxylic acid piperidin-1-ylamide | | |
| 74 | 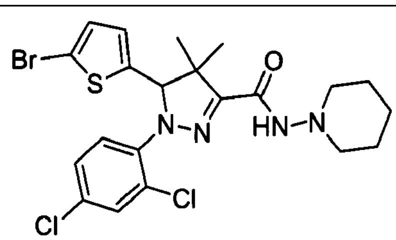 | 5-(5-Bromo-thiophen-2-yl)-1-(2,4-di-chloro-phenyl)-4,4-dimethyl-4,5-di-hydro-1H-pyrazole-3-carboxylic acid piperidin-1-ylamide | | |

(continued)

| N° | STRUCTURE | Name | $^1$H-NMR | MS (M+H)$^+$ |
|---|---|---|---|---|
| 75 | 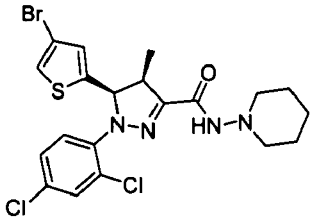 | *cis*-5-(4-Bromo-thiophen-2-yl)-1-(2,4-dichloro-phenyl)-4-methyl-4,5-dihydro-1H-pyrazole-3-carboxylic acid piperidin-1-ylamide | | |
| 76 | 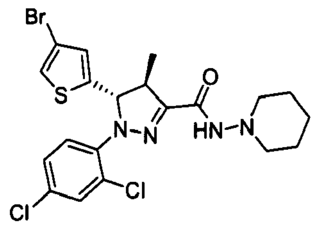 | *trans*-5-(4-Bromo-thiophen-2-yl)-1-(2,4-dichloro-phenyl)-4-methyl-4,5-dihydro-1H-pyrazole-3-carboxylic acid piperidin-1-ylamide | | |
| 77 | 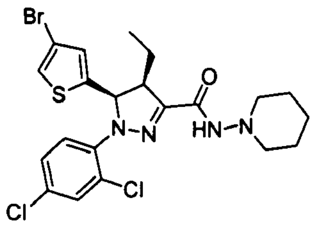 | *cis*-5-(4-Bromo-thiophen-2-yl)-1-(2,4-dichloro-phenyl)-4-ethyl-4,5-dihydro-1H-pyrazole-3-carboxylic acid piperidin-1-yiamide | | |
| 78 | 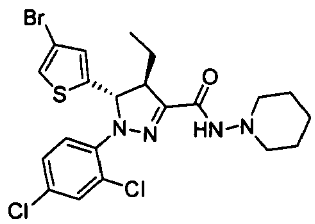 | *trans*-5-(4-Bromo-thiophen-2-yl)-1-(2,4-dichloro-phenyl)-4-ethyl-4,5-dihydro-1H-pyrazole-3-carboxylic acid piperidin-1-ylamide | | |
| 79 | 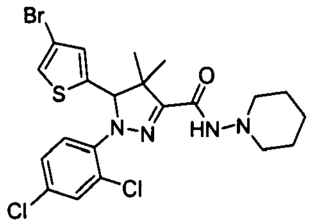 | 5-(4-Bromo-thiophen-2-yl)-1-(2,4-di chloro-phenyl)-4,4-dimethyl-4,5-dih ydro-1H-pyrazole-3-carboxylic acid piperidin-1-ylamide | | |
| 80 | 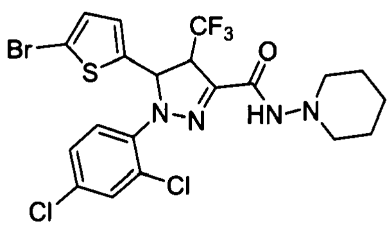 | 5-(5-Bromo-thiophen-2-yl)-1-(2,4-dichloro-phenyl)-4-trifluoromethyl-4,5-dihydro-1H-pyrazole-3-carboxylic acid piperidin-1-yiamide | | |
| 81 | 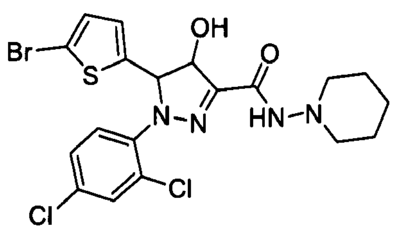 | 5-(5-Bromo-thiophen-2-yl)-1-(2,4-di-chloro-phenyl)-4-hydroxy-4,5-dihydro-1H-pyrazole-3-carboxylic acid piperidin-1-ylamide | | |

(continued)

| N° | STRUCTURE | Name | $^1$H-NMR | MS (M+H)$^+$ |
|---|---|---|---|---|
| 82 | 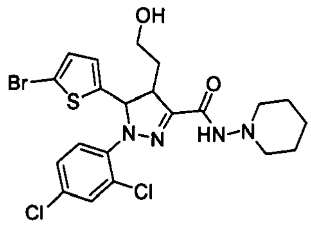 | 5-(5-Bromo-thiophen-2-yl)-1-(2,4-di-chloro-phenyl)-4-(2-hydroxy-ethyl)-4,5-dihydro-1H-pyrazole-3-carboxylic acid piperidin-1-ylamide | | |
| 83 | 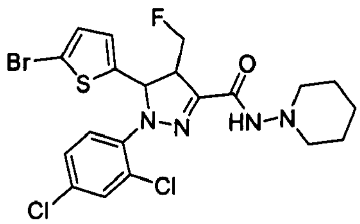 | 5-(5-Bromo-thiophen-2-yl)-1-(2,4-di-chloro-phenyl)-4-fluoromethyl-4,5-dihydro-1H-pyrazole-3-carboxylic acid piperidin-1-ylamide | | |
| 84 | 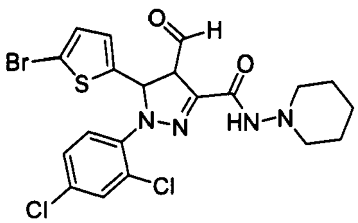 | 5-(5-Bromo-thiophen-2-yl)-1-(2,4-di-chloro-phenyl)-4-formyl-4,5-dihydro-1H-pyrazole-3-carboxylic acid piperidin-1-ylamide | | |
| 85 | 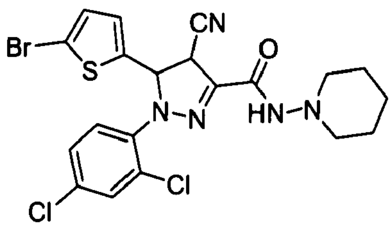 | 5-(5-Bromo-thiophen-2-yl)-4-cyano-1-(2,4-dichloro-phenyl)-4,5-dihydro-1H-pyrazole-3-carboxylic acid piperidin-1-ylamide | | |
| 86 | 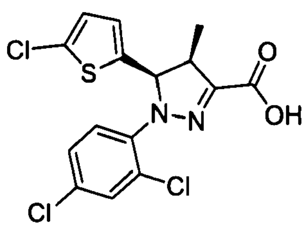 | *cis*-5-(5-Chloro-thiophen-2-yl)-1-(2,4-dichloro-phenyl)-4-methyl-4,5-dihydro-1H-pyrazole-3-carboxylic acid | | |
| 87 | 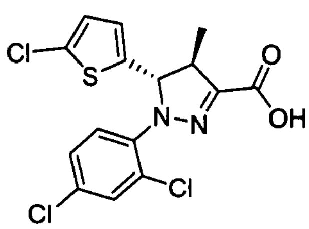 | *trans*-[5-(5-Chloro-thiophen-2-yl)-1-(2,4-dichloro-phenyl)-4-methyl-4,5-dihydro-1H-pyrazol-3-yl]-piperidin-1-yl-methanone | | |
| 88 | 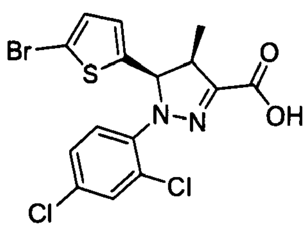 | *cis*-5-(5-Bromo-thiophen-2-yl)-1-(2,4-dichloro-phenyl)-4-methyl-4,5-dihydro-1H-pyrazole-3-carboxylic acid | | |

(continued)

| N° | STRUCTURE | Name | $^1$H-NMR | MS $(M+H)^+$ |
|---|---|---|---|---|
| 89 | 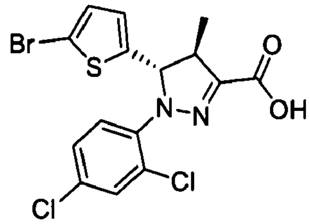 | *trans*-5-(5-Bromo-thiophen-2-yl)-1-(2,4-dichloro-phenyl)-4-methyl-4,5-dihydro-1H-pyrazole-3-carboxylic acid | | |
| 90 | 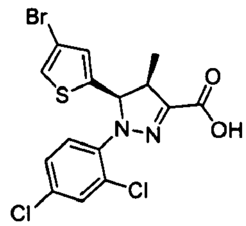 | *cis*-5-(4-Bromo-thiophen-2-yl)-1-(2,4-dichloro-phenyl)-4-methyl-4,5-dihydro-1H-pyrazole-3-carboxylic acid | | |
| 91 | 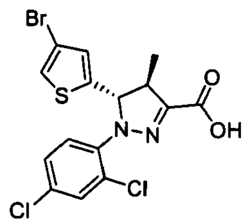 | *trans*-5-(4-Bromo-thiophen-2-yl)-1-(2,4-dichloro-phenyl)-4-methyl-4,5-dihydro-1H-pyrazole-3-carboxylic acid | | |

## Claims

**1.** A 4-substituted pyrazoline compound of general formula I,

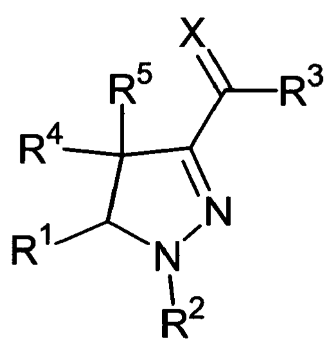

I

wherein

X is O or S;

$R^1$ and $R^2$, independently of one another, in each case represent an unsubstituted or at least mono-substituted aryl or heteroaryl radical, which may be condensed with an unsubstituted or at least mono-substituted mono- or polycyclic ring system;

or a saturated or unsaturated, unsubstituted or at least mono-substituted, optionally at least one heteroatom as a ring member containing cycloaliphatic radical, which may be condensed with an unsubstituted or at least

mono-substituted mono- or polycyclic ring system and/or may be bridged by at least one unsubstituted or at least mono-substituted alkylene group;

$R^3$ an unsubstituted or at least mono-substituted aryl or heteroaryl radical, which may be condensed with an unsubstituted or at least mono-substituted mono- or polycyclic ring system;

a saturated or unsaturated, unsubstituted or at least mono-substituted,

optionally at least one heteroatom as a ring member containing cycloaliphatic radical, which may be condensed with an unsubstituted or at least mono-substituted mono- or polycyclic ring system and/or may be bridged by at least one unsubstituted or at least mono-substituted alkylene group;

a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;

a —O-$R^6$ moiety; a —$NR^7R^8$ moiety or a —$NR^9$-O-$R^{10}$ moiety;

$R^4$ represents F; Cl; Br; I; -CN; -$NO_2$; -NC; -OH; -$NH_2$; -SH; -C(=O)-H;

a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

an unsubstituted or at least mono-substituted aryl or heteroaryl radical, which may be condensed with an unsubstituted or at least mono-substituted mono- or polycyclic ring system and/or may be bonded via an optionally at least mono-substituted alkylene group, alkenylene group or alkinylene group;

a —O-$R^{11}$ moiety; a —S-$R^{12}$ moiety; a —NH-$R^{13}$ moiety or a —$NR^{14}R^{15}$ moiety;

$R^5$ represents H; F; Cl; Br; I; -CN; -$NO_2$; -NC; -OH; -$NH_2$; -SH; -C(=O)-H;

a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

a —O-$R^{11}$ moiety; a —S-$R^{12}$ moiety; a —NH-$R^{13}$ moiety or a —$NR^{14}R^{15}$ moiety;

$R^6$ represents a hydrogen atom;

a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

a —P(=O)($OR^{16}$)$_2$ moiety; a —C(=O)-$OR^{17}$ moiety; a —C(=O)-NH-$R^{18}$ moiety or a —C(=O)-$R^{19}$ moiety;

$R^7$ and $R^8$, independently of one another, in each case represent a hydrogen atom;

a saturated or unsaturated, unsubstituted or at least mono-substituted aliphatic radical;

a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;

a saturated or unsaturated, unsubstituted or at least mono-substituted, optionally at least one heteroatom as a ring member containing cycloaliphatic radical which together with a saturated or unsaturated, unsubstituted or

at least mono-substituted, optionally at least one heteroatom as a ring member containing cycloaliphatic radical forms a
saturated or unsaturated, unsubstituted or at least mono-substituted, optionally at least one heteroatom as a ring member containing spirocyclic residue via a common ring atom;
an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;
a —P(=O)($OR^{16}$)$_2$ moiety; a —C(=O)-$OR^{17}$ moiety; a —C(=O)-NH-$R^{18}$ moiety; a —C(=O)-$R^{19}$ moiety; a —S(=O)$_2$-$R^{20}$ moiety; or a -$NR^{21}R^{22}$ moiety;
$R^9$ represents hydrogen or a saturated or unsaturated, unsubstituted or at least mono-substituted aliphatic radical;
$R^{10}$, $R^{11}$, $R^{12}$, $R^{13}$, $R^{14}$, $R^{15}$ and $R^{20}$, independently of one another, in each case represent a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group and/or may be bridged by at least one unsubstituted or at least mono-substituted alkylene group;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene
group, alkenylene group or alkinylene group;
$R^{16}$, $R^{17}$, $R^{18}$ and $R^{19}$, independently of one another, in each case represent a saturated or unsaturated, unsubstituted or at least mono-substituted aliphatic radical;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

and

$R^{21}$ and $R^{22}$, independently of one another, in each case represent a hydrogen atom;
a saturated or unsaturated, unsubstituted or at least mono-substituted aliphatic radical;
a saturated or unsaturated, unsubstituted or at least mono-substituted, optionally at least one heteroatom as a ring member containing cycloaliphatic radical, which may be condensed with an unsubstituted or at least mono-substituted mono- or polycyclic ring system and/or may be bonded via an unsubstituted or at least mono-substituted alkylene group, alkenylene group or alkinylene group;
a saturated or unsaturated, unsubstituted or at least mono-substituted, optionally at least one heteroatom as a ring member containing cycloaliphatic radical which together with a saturated or unsaturated, unsubstituted or at least mono-substituted, optionally at least one heteroatom as a ring member containing cycloaliphatic radical forms a saturated or unsaturated, unsubstituted or at least mono-substituted, optionally at least one heteroatom as a ring member containing spirocyclic residue via a common ring atom;
or an unsubstituted or at least mono-substituted aryl or heteroaryl radical, which may be condensed with an unsubstituted or at least mono-substituted mono- or polycyclic ring system and/or may be bonded via an unsubstituted or at least mono-substituted alkylene group, alkenylene group or alkinylene group;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**2.** A compound according to claim 1, **characterized in that**

X is O or S;
$R^1$ and $R^2$, independently of one another, in each case represent an unsubstituted or at least mono-substituted 6- or 10-membered aryl radical, which may be condensed with an unsubstituted or at least mono-substituted mono- or polycyclic ring system;
an unsubstituted or at least mono-substituted 5- to 14-membered heteroaryl radical, which may be condensed with an unsubstituted or at least mono-substituted mono- or polycyclic ring system;
an unsubstituted or at least mono-substituted $C_{3-16}$ cycloalkyl radical or $C_{4-16}$ cycloalkenyl radical, which in

each case may be condensed with an unsubstituted or at least mono-substituted mono- or polycyclic ring system and/or may be bridged by at least one unsubstituted or at least mono-substituted $C_{1-5}$ alkylene group;
or an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;
$R^3$ represents an unsubstituted or at least mono-substituted 6- or 10-membered aryl radical, which may be condensed with an unsubstituted or at least mono-substituted mono- or polycyclic ring system;
an unsubstituted or at least mono-substituted 5- to 14-membered heteroaryl radical, which may be condensed with an unsubstituted or at least mono-substituted mono- or polycyclic ring system;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group;
an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an unsubstituted or at least mono-substituted spirocyclic residue via a common ring atom;
a -O-$R^6$ moiety; a —$NR^7R^8$ moiety or a —$NR^9$-O-$R^{10}$ moiety;
$R^4$ represents F; Cl; Br; I; -CN; -$NO_2$; -NC; -OH; -$NH_2$; -SH; -C(=O)-H;
an unsubstituted or at least mono-substituted $C_{1-16}$ alkyl radical, $C_{2-16}$ alkenyl radical or $C_{2-16}$ alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
a —O-$R^{11}$ moiety; a —S-$R^{12}$ moiety; a —NH-$R^{13}$ moiety or a —$NR^{14}R^{15}$ moiety;
$R^5$ represents H; F; Cl; Br; I; -CN; -$NO_2$; -NC; -OH; -$NH_2$; -SH; -C(=O)-H;
an unsubstituted or at least mono-substituted $C_{1-16}$ alkyl radical, $C_{2-16}$ alkenyl radical or $C_{2-16}$ alkinyl radical;
an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;
an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group and/or may be bridged by at least one unsubstituted or at least mono-substituted $C_{1-5}$ alkylene group;
an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;
or an unsubstituted or at least mono-substituted 5- to 14-membered heteroaryl radical, which may be condensed

with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

—O-$R^{11}$ moiety; a -S-$R^{12}$ moiety; a -NH-$R^{13}$ moiety or a —N$R^{14}R^{15}$ moiety;

$R^6$ represents a hydrogen atom;

an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group; a —P(=O)(O$R^{16}$)$_2$ moiety; a —C(=O)-O$R^{17}$ moiety; a —C(=O)-NH-$R^{18}$ moiety or a —C(=O)-$R^{19}$ moiety;

$R^7$ and $R^8$, independently of one another, in each case represent a hydrogen atom;

an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an unsubstituted or at least mono-substituted spirocyclic residue via a common ring atom;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

a —P(=O)(O$R^{16}$)$_2$ moiety; a —C(=O)-O$R^{17}$ moiety; a -C(=O)-NH-$R^{18}$ moiety; a —C(=O)-$R^{19}$ moiety; a —S(=O)$_2$-$R^{20}$ moiety; or a —N$R^{21}R^{22}$ moiety;

$R^9$ represents a hydrogen atom or an unsubstituted or at least mono-substituted $C_{1-16}$ alkyl radical, $C_{2-16}$ alkenyl radical or $C_{2-16}$ alkinyl radical;

$R^{10}$, $R^{11}$, $R^{12}$, $R^{13}$, $R^{14}$, $R^{15}$ and $R^{20}$, independently of one another, in each case represent an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bridged by at least one unsubstituted or at least mono-substituted $C_{1-5}$ alkylene group and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

$R^{16}$, $R^{17}$, $R^{18}$ and $R^{19}$, independently of one another, in each case represent an unsubstituted or at least mono-substituted $C_{1-16}$ alkyl radical, $C_{2-16}$ alkenyl radical or $C_{2-16}$ alkinyl radical;

or an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group; and

$R^{21}$ and $R^{22}$, independently of one another, in each case represent a hydrogen atom;

an unsubstituted or at least mono-substituted $C_{1-16}$ alkyl radical, $C_{2-16}$ alkenyl radical or $C_{2-16}$ alkinyl radical;

an unsubstituted or at least mono-substituted $C_{3-16}$ cycloalkyl radical or $C_{4-16}$ cycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{4-16}$ heterocycloalkyl radical or $C_{5-16}$ heterocycloalkenyl radical, which in each case may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical which together with an unsubstituted or at least mono-substituted $C_{3-16}$ cycloalkyl radical, $C_{4-16}$ cycloalkenyl radical, $C_{4-16}$ heterocycloalkyl radical or $C_{5-16}$ heterocycloalkenyl radical forms an

unsubstituted or at least mono-substituted spirocyclic residue via a common ring atom;

an unsubstituted or at least mono-substituted 6- or 10-membered aryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

or an unsubstituted or at least mono-substituted 5- to 14-membered heteroaryl radical, which may be condensed with an unsubstituted or at least mono-substituted mono- or polycyclic ring system and/or may be bonded via an unsubstituted or at least mono-substituted $C_{1-5}$ alkylene group, $C_{2-5}$ alkenylene group or $C_{2-5}$ alkinylene group;

whereby

the rings of the aforementioned ring system are in each case independently of one another 5- 6- or 7-membered and may in each case independently of one another optionally contain 1, 2 or 3 heteroatom(s) independently selected from the group consisting of nitrogen, oxygen and sulfur;

the aforementioned heteroaryl radicals in each case optionally contain 1, 2, 3 or 4 heteroatom(s) independently selected from the group consisting of nitrogen, oxygen and sulfur as ring member(s);

the aforementioned heterocycloalkyl radicals and heterocycloalkenyl radicals in each case optionally contain 1, 2, 3 or 4 heteroatom(s) independently selected from the group consisting of nitrogen, oxygen and sulfur as ring member (s);

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

3. A compound according to claim 1 or 2, **characterized in that** $R^1$ and $R^2$, independently of one another, in each

case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl, imidazo[2,1-b]thiazolyl, 2H-chromenyl, pyranyl, indazolyl, quinazolinyl, [1,3]-benzodioxolyl, [1,4]-benzodioxanyl, [1,2,3,4]-tetrahydronaphthyl, (2,3)-dihydro-1H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl, which in each case is optionally bonded to the pyrazoline compound of general formula I via the aromatic or heteroaromatic part of the aforementioned radicals and may optionally be substituted with 1, 2, 3, 4 or 5 substituent(s) independently selected from the group consisting of —$CF_3$, -$CH_2$-Cl, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-C($CH_3$)$_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-$C_2H_5$, -N($CH_3$)$_2$, -N($C_2H_5$)$_2$, phenoxy and thiophenyl, whereby the thiophenyl radical can be substituted with 1, 2 or 3 substituents independently selected from the group consisting of F, Cl, Br, methyl, ethyl and n-propyl; or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl, (1,2,3,4)-tetrahydroquinoxazlinyl, adamantyl, bicyclo[2.2.1]heptyl, bicyclo[3.1.1]heptyl and norbomenyl, which in each case is optionally bonded to the pyrazoline compound of general formula I via the (hetero) cycloaliphatic part of the aforementioned radicals and
may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-C($CH_3$)$_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-$C_2H_5$, -N($CH_3$)$_2$ and —N($C_2H_5$)$_2$.

4. A compound according to one or more of claims 1 to 3, **characterized in that**
$R^3$ represents a radical selected from the group consisting of (2,3)-dihydro-1 H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1 H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, (1,2,3,4)-tetrahydroquinoxazlinyl, adamantyl, bicyclo[2.2.1]heptyl, bicyclo[3.1.1]heptyl, norbomenyl, 8-aza-bicyclo[3.2.1]octyl and 8-aza-spiro[4.5]decanyl, which may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -O-$CH_2$-O-$CH_3$, -O-$CH_2$-$CH_2$-O-$CH_3$,

$-O-CH_2-O-C_2H_5$, $-C(OCH_3)(C_2H_5)_2$, $-C(OCH_3)(CH_3)_2$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C(=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$,- $C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $—N(C_2H_5)_2$, cyclopentyl, cyclohexyl, pyrrolidinyl and piperidinyl;
a $—O-R^6$ moiety; a $—NR^7R^8$ moiety or a $—NR^9-O-R^{10}$ moiety.

5. A compound according to one or more of claims 1 to 4, **characterized in that** $R^4$ represents F; Cl; Br, I; -CN; $-NO_2$; -NC; -OH; $-NH_2$; -SH; -C(=O)-H;
a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4, 5, 6, 7, 8 or 9 substituent(s) independently selected from the group consisting of —OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $-N(C_2H_5)_2$, -CN and $-NO_2$;
a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $—(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of $—CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$,- $S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;
a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)2$;
a $—O-R^{11}$ moiety; a $—S-R^{12}$ moiety, a $—NH-R^{13}$ moiety or a $—NR^{14}R^{15}$ moiety.

6. A compound according to one or more of claims 1 to 5, **characterized in that** $R^5$ represents H, F; Cl; Br; I; -CN; $-NO_2$; -NC; -OH; $-NH_2$; -SH; -C(=O)-H;
a radical selected from the group consisting of methyl, $-CF_3$, $-CH_2F$, $-CF_2H$, $-C_2F_5$, ethyl, $-CH_2-CN$, $-CH_2-OH$, n-propyl, isopropyl, $-CH_2-CH_2-CN$, $-CH_2-CH_2-OH$, n-butyl, $-CH_2-CH_2-CH_2-CN$, $-CH_2-CH_2-CH_2-OH$, isobutyl, sec-butyl, tert-butyl, n-pentyl, 2-pentyl, 3-pentyl, neo-pentyl, n-hexyl, 2-hexyl and 3-hexyl;
a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl,
a radical selected from the group consisting of phenyl, naphthyl, pyridinyl, furyl (furanyl), thienyl (thiophenyl) and pyrrolyl, which may be bonded via a $—(CH_2)$-group and/or may optionally be substituted with 1, 2, 3, 4 or 5 substituent (s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, F, Cl and Br;
a $—O-R^{11}$ moiety; a $-S-R^{12}$ moiety, a $-NH-R^{13}$ moiety or a $—NR^{14}R^{15}$ moiety.

7. A compound according to one or more of claims 1 to 6, **characterized in that** $R^6$ represents a hydrogen atom; a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected

from the group consisting of —OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $-N(C_2H_5)_2$, -CN, -NO, $-NH-C(=O)-CH_3$, $-NH-C(=O)-C_2H_5$, $-NH-C(=O)-C(CH_3)_3$, $-NH-C(=O)-O-CH_3$, $-NH-C(=O)-O-C_2H_5$, $-NH-C(=O)-O-C(CH_3)_3$, $-C(=O)-NH-CH_3$, $-C(=O)-NH-C_2H_5$, $-C(=O)-NH-C(CH_3)_3$, $-C(=O)-N(CH_3)_2$, $-C(=O)-N(C_2H_5)_2$, -C(=O)-OH, $-C(=O)-O-CH_3$, $-C(=O)-O-C_2H_5$, $-C(=O)-O-C(CH_3)_3$, $-C(=O)-CH_3$, $-C(=O)-C_2H_5$ and $-C(=O)-C(CH_3)_3$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-aza-bicyclo[3.2.1]octyl and diazepanyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of $—CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-0-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, - $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=0)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, $-O-C(=O)-CH_3$, $-O-C(=O)-C_2H_5$, $-O-C(=O)-CH(CH_3)_2$, $-O-C(=O)-CH_2-CH_2-CH_3$, $-CH_2-N(CH_3)_2$, $-(CH_2)-N(C_2H_5)_2$, $-CH_2-N(C_3H_7)_2$, $-CH_2-N(C_4H_9)_2$, $-CH_2-N(CH_3)(C_2H_5)$ and $-(CH_2)$-morpholinyl;

a $—P(=O)(OR^{16})_2$ moiety; a $—C(=O)-OR^{17}$ moiety; a $—C(=O)-NH-R^{18}$ moiety or a— $C(=O)-R^{19}$ moiety.

**8.** A compound according to one or more of claims 1 to 7, **characterized in that** $R^7$ and $R^8$, independently of another, in each case represent a hydrogen atom;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $—N(C_2H_5)_2$, -CN and $-NO_2$;

a radical selected from the group consisting of (1,2,3,4)-tetrahydronaphthyl, (2,3)-dihydro-1H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, (1,2,3,4)-tetrahydroquinoxazlinyl, adamantyl, bicyclo[2.2.1]heptyl, bicyclo[3.1.1]heptyl, norbomenyl, 8-aza-bicyclo[3.2.1]octyl and 8-aza-spiro[4.5]decanyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, -C

$(OCH_3)(CH_3)_2$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C (=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, -C (=O)-NH-$C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, -S $(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, -S-$CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$,- $S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

a $-P(=O)(OR^{16})_2$ moiety; a $-C(=O)-OR^{17}$ moiety; a $-C(=O)-NH-R^{18}$ moiety; a $-C(=O)-R^{19}$ moiety; a $-S(=O)_2-R^{20}$ moiety; or a $-NR^{21}R^{22}$ moiety.

**9.** A compound according to one or more of claims 1 to 8, **characterized in that** $R^9$ represents hydrogen or an alkyl radical selected from the group consisting of methyl, ethyl and n-propyl.

**10.** A compound according to one or more of claims 1 to 9, **characterized in that** $R^{10}$, $R^{11}$, $R^{12}$, $R^{13}$, $R^{14}$, $R^{15}$ and $R^{20}$, independently of another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, -N $(CH_3)_2$, $-N(C_2H_5)_2$, -CN and $-NO_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-aza-bicyclo[3.2.1]octyl and diazepanyl, which may be bonded via a $-(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, -O-$CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, -S-CH $(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, -S $(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, -S-$CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$.

**11.** A compound according to one or more of claims 1 to 10, **characterized in that** $R^{16}$, $R^{17}$, $R^{18}$ and $R^{19}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of $NH_2$, $-NH-CH_3$, $-NH-C_2H_5$, $-N(CH_3)_2$, $-N(C_2H_5)_2$,- $NH-C(=O)-CH_3$, $-NH-C(=O)-C_2H_5$, $-NH-C(=O)-C(CH_3)_3$, $-NH-C(=O)-O-CH_3$, $-NH-C(=O)-O-C_2H_5$, $-NH-C(=O)-O-C(CH_3)_3$, $-C(=O)-NH-CH_3$, $-C(=O)-NH-C_2H_5$, $-C(=O)-NH-C(CH_3)_3$, $-C(=O)-N(CH_3)_2$, $-C(=O)-N(C_2H_5)_2$, -OH, -C(=O)-OH, $-C(=O)-O-CH_3$, $-C(=O)-O-C_2H_5$, $-C(=O)-O-C(CH_3)_3$, $-C(=O)-CH_3$, $-C(=O)-C_2H_5$ and $-C(=O)-C(CH_3)_3$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or $-CH=CH-$ group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, $-O-C(=O)-CH_3$, $-O-C(=O)-C_2H_5$, $-O-C(=O)-CH(CH_3)_2$, $-O-C(=O)-CH_2-CH_2-CH_3$, $-CH_2-N(CH_3)_2$, $-(CH_2)-N(C_2H_5)_2$, $-CH_2-N(C_3H_7)_2$, $-CH_2-N(C_4H_9)_2$, $-CH_2-N(CH_3)(C_2H_5)$ and $-(CH_2)$-morpholinyl.

**12.** A compound according to one or more of claims 1 to 11, **characterized in that** $R^{21}$ and $R^{22}$, independently of another, in each case represent hydrogen;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of -OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $-N(C_2H_5)_2$, -CN and $-NO_2$;

a radical selected from the group consisting of (2,3)-dihydro-1 H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl, (1 ,2,3,4)-tetrahydroquinoxazlinyl, adamantyl, bicyclo[2.2.1]heptyl, bicyclo[3.1.1]heptyl, norbornenyl, 8-aza-bicyclo[3.2.1]octyl and 8-aza-spiro[4.5]decanyl, which may be bonded via a $-(CH_2)-$, $-(CH_2)-(CH_2)-$, $-(CH_2)-(CH_2)-(CH_2)-$ or $-CH=CH-$group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-S-CH_3$, $-S-C_2H_5$, $-S-CH_2-CH_2-CH_3$, $-S-CH(CH_3)_2$,- $S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C(=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or $-CH=CH-$ group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$,

-OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$.

**13.** A compound according to one or more of claims 1 to 12, **characterized in that**

X is S or O;

$R^1$ and $R^2$, independently of one another, in each case represent a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl, imidazo[2,1-b]thiazolyl, 2H-chromenyl, pyranyl, indazolyl, quinazolinyl, [1,3]-benzodioxolyl, [1,4]-benzodioxanyl, [1,2,3,4]-tetrahydronaphthyl, (2,3)-dihydro-1 H-cyclopenta[b]indolyl, [1,2,3,4]-tetrahydroquinolinyl, [1,2,3,4]-tetrahydroisoquinolinyl, [1,2,3,4]-tetrahydroquinazolinyl and [3,4]-dihydro-2H-benzo[1,4]oxazinyl, which in each case is optionally bonded to the pyrazoline compound of general formula I via the aromatic or heteroaromatic part of the aforementioned radicals and may optionally be substituted with 1, 2, 3, 4 or 5 substituent(s) independently selected from the group consisting of $—CF_3$, -CH, -Cl, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $—N(C_2H_5)_2$, phenoxy and thiophenyl, whereby the thiophenyl radical can be substituted with 1, 2 or 3 substituents independently selected from the group consisting of F, Cl, Br, methyl, ethyl and n-propyl;

or a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, (1,2,3,4)-tetrahydroquinoxazlinyl, adamantyl, bicyclo[2.2.1]heptyl, bicyclo[3.1.1]heptyl and norbomenyl, which in each case is optionally bonded to the pyrazoline compound of general formula I via the (hetero)cycloaliphatic part of the aforementioned radicals and may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$;

$R^3$ represents a radical selected from the group consisting of (2,3)-dihydro-1H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1 H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, (1 ,2,3,4)-tetrahydroquinoxazlinyl, adamantyl, bicyclo[2.2.1]heptyl, bicyclo [3.1.1]heptyl, norbomenyl, 8-aza-bicyclo[3.2.1]octyl and 8-aza-spiro[4.5]decanyl, which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C(=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;

a —O-$R^6$ moiety; a —$NR^7R^8$ moiety or a —$NR^9$-O-$R^{10}$ moiety;

$R^4$ represents F; Cl; Br; I; -CN; $-NO_2$; -NC; -OH; $-NH_2$; -SH; -C(=O)-H;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl and 4-octyl, which may optionally be substituted with 1, 2, 3, 4, 5, 6, 7, 8 or 9 substituent (s) independently selected from the group consisting of —OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, —$N(C_2H_5)_2$, -CN and $-NO_2$;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a —$(CH_2)$-, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and —$N(C_2H_5)_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and —$N(C_2H_5)_2$;

a —O-$R^{11}$ moiety; a —S-$R^{12}$ moiety, a —NH-$R^{13}$ moiety or a —$NR^{14}R^{15}$ moiety;

$R^5$ represents H, F; Cl; Br; I; -CN; $-NO_2$; -NC; -OH; $-NH_2$; -SH; -C(=O)-H;

a radical selected from the group consisting of methyl, $-CF_3$, $-CH_2F$, $-CF_2H$, $-C_2F_5$, ethyl, $-CH_2-CN$, $-CH_2-OH$, n-propyl, isopropyl, $-CH_2-CH_2-CN$, $-CH_2-CH_2-OH$, n-butyl, $-CH_2-CH_2-CH_2-CN$, $-CH_2-CH_2-CH_2-OH$, isobutyl, sec-butyl, tert-butyl, n-pentyl, 2-pentyl, 3-pentyl, neo-pentyl, n-hexyl, 2-hexyl and 3-hexyl;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl;

a radical selected from the group consisting of phenyl, naphthyl, pyridinyl, furyl (furanyl), thienyl (thiophenyl) and pyrrolyl, which may be bonded via a —$(CH_2)$-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, F, Cl and Br;

a —O-$R^{11}$ moiety; a —S-$R^{12}$ moiety, a —NH-$R^{13}$ moiety or a —N$R^{14}R^{15}$ moiety;

$R^6$ represents a hydrogen atom; a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $—N(C_2H_5)_2$, -CN, -NO, $-NH-C(=O)-CH_3$, $-NH-C(=O)-C_2H_5$, $-NH-C(=O)-C(CH_3)_3$, $-NH-C(=O)-O-CH_3$, $-NH-C(=O)-O-C_2H_5$, $-NH-C(=O)-O-C(CH_3)_3$, $-C(=O)-NH-CH_3$, $-C(=O)-NH-C_2H_5$, $-C(=O)-NH-C(CH_3)_3$, $-C(=O)-N(CH_3)_2$, $-C(=O)-N(C_2H_5)_2$, -C(=O)-OH, $-C(=O)-O-CH_3$, $-C(=O)-O-C_2H_5$, $-C(=O)-O-C(CH_3)_3$, $-C(=O)-CH_3$, $-C(=O)-C_2H_5$ and $-C(=O)-C(CH_3)_3$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-azabicyclo[3.2.1]octyl and diazepanyl, which may be bonded via a $-(CH_2)-$,- $(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $—N(C_2H_5)_2$;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $—N(C_2H_5)_2$, $-O-C(=O)-CH_3$, $-O-C(=O)-C_2H_5$, $-O-C(=O)-CH(CH_3)_2$, $-O-C(=O)-CH_2-CH_2-CH_3$, $-CH_2-N(CH_3)_2$, $-(CH_2)-N(C_2H_5)_2$, $-CH_2-N(C_3H_7)_2$, $-CH_2-N(C_4H_9)_2$, $-CH_2-N(CH_3)(C_2H_5)$ and $—(CH_2)$-morpholinyl;

a $—P(=O)(OR^{16})_2$ moiety; a $—C(=O)-OR^{17}$ moiety; a $—C(=O)-NH-R^{18}$ moiety or a $—C(=O)-R^{19}$ moiety;

$R^7$ and $R^8$, independently of another, in each case represent a hydrogen atom;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent (s) independently selected from the group consisting of -OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $—N(C_2H_5)_2$, -CN and $-NO_2$;

a radical selected from the group consisting of (1,2,3,4)-tetrahydronaphthyl, (2,3)-dihydro-1 H-cyclopenta[b] indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1 H-pyrrolyl, (2,3)-dihydro-1 H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl, decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1 H-benzo[de]isoquinolinyl, (1 ,2,3,4)-tetrahydroquinoxazlinyl, adamantyl, bicyclo [2.2.1]heptyl, bicyclo[3.1.1]heptyl, norbornenyl, 8-aza-bicyclo[3.2.1]octyl and 8-aza-spiro[4.5]decanyl, which may be bonded via a -($CH_2$)-, -($CH_2$)-($CH_2$)-, -($CH_2$)-($CH_2$)-($CH_2$)- or -CH=CH-group and/or may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -O-$CH_2$-O-$CH_3$, -O-$CH_2$-$CH_2$-O-$CH_3$, -O-$CH_2$-O-$C_2H_5$, -C($OCH_3$)($C_2H_5$)$_2$, -C($OCH_3$)($CH_3$)$_2$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-C($CH_3$)$_3$, -C(=O)-OH, -C(=O)-O-$CH_3$, -C(=O)-O-$C_2H_5$, -C(=O)-O-C($CH_3$)$_3$, -$CF_2H$, -$CFH_2$, -C(=O)-$NH_2$, -C(=O)-NH-$CH_3$, -C(=O)-NH-$C_2H_5$, -C(=0)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-$C_2H_5$, -N($CH_3$)$_2$, —N($C_2H_5$)$_2$, cyclopentyl, cyclohexyl, pyrrolidinyl and piperidinyl;

a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a -($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, O-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-C($CH_3$)$_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-$C_2H_5$, -N($CH_3$)$_2$ and —N($C_2H_5$)$_2$;

a —P(=O)($OR^{16}$)$_2$ moiety; a —C(=O)-$OR^{17}$ moiety; a —C(=O)-NH-$R^{18}$ moiety; a -C(=O)-$R^{19}$ moiety; a —S(=O)$_2$-$R^{20}$ moiety; or a —$NR^{21}R^{22}$ moiety;

$R^9$ represents hydrogen or an alkyl radical selected from the group consisting of methyl, ethyl and n-propyl.

$R^{10}$, $R^{11}$, $R^{12}$, $R^{13}$, $R^{14}$, $R^{15}$ and $R^{20}$, independently of another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of —OH, F, Cl, Br, I, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -$NH_2$, -NH-$CH_3$, -NH-$C_2H_5$, -N($CH_3$)$_2$, -N($C_2H_5$)$_2$, -CN and -$NO_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-azabicyclo[3.2.1]octyl and diazepanyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of oxo (=O), thioxo (=S), -$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-C($CH_3$)$_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-$C_2H_5$, -N($CH_3$)$_2$ and —N($C_2H_5$)$_2$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a -($CH_2$)-, -($CH_2$)-($CH_2$)-, -($CH_2$)-($CH_2$)-($CH_2$)- or -CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-CH($CH_3$)$_2$, -O-$CH_2$-$CH_2$-$CH_2$-$CH_3$, -O-C($CH_3$)$_3$, -S-$CH_3$, -S-$C_2H_5$, -S-$CH_2$-$CH_2$-$CH_3$, -S-CH($CH_3$)$_2$, -S-$CH_2$-$CH_2$-$CH_2$-$CH_3$, -S-C($CH_3$)$_3$, F, Cl, Br, I, -CN, -$OCF_3$, -$SCF_3$, -$SCF_2H$, -$SCFH_2$, -OH, -SH, -$NO_2$, -CHO, -C(=O)-$CH_3$, -C(=O)-$C_2H_5$, -C(=O)-C($CH_3$)$_3$, -$CF_2H$, -$CFH_2$, -C(=O)-$NH_2$, -C(=O)-NH-$CH_3$, -C(=O)-NH-$C_2H_5$, -C(=O)-NH-$C_3H_7$, -C(=O)-N($CH_3$)$_2$, -C(=O)-N($C_2H_5$)$_2$, -S(=O)-$CH_3$, -S(=O)-$C_2H_5$, -S(=O)-$C_3H_7$, -S(=O)$_2$-$CH_3$, -S(=O)$_2$-$C_2H_5$, -S(=O)$_2$-$C_3H_7$, -$NH_2$, -NH-$CH_3$, -NH-

$C_2H_5$, $-N(CH_3)_2$ and $-N(C_2H_5)_2$;

$R^{16}$, $R^{17}$, $R^{18}$ and $R^{19}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of $NH_2$, $-NH-CH_3$, $-NH-C_2H_5$, $-N(CH_3)_2$, $-N(C_2H_5)_2$, $-NH-C(=O)-CH_3$, $-NH-C(=O)-C_2H_5$, $-NH-C(=O)-C(CH_3)_3$, $-NH-C(=O)-O-CH_3$, $-NH-C(=O)-O-C_2H_5$, $-NH-C(=O)-O-C(CH_3)_3$, $-C(=O)-NH-CH_3$, $-C(=O)-NH-C_2H_5$, $-C(=O)-NH-C(CH_3)_3$, $-C(=O)-N(CH_3)_2$, $-C(=O)-N(C_2H_5)_2$, -OH, -C(=O)-OH, $-C(=O)-O-CH_3$, $-C(=O)-O-C_2H_5$, $-C(=O)-O-C(CH_3)_3$, $-C(=O)-CH_3$, $-C(=O)-C_2H_5$ and $-C(=O)-C(CH_3)_3$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or $-CH=CH-$group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, $-O-C(=O)-CH_3$, $-O-C(=O)-C_2H_5$, $-O-C(=O)-CH(CH_3)_2$, $-O-C(=O)-CH_2-CH_2-CH_3$, $-CH_2-N(CH_3)_2$, $-(CH_2)-N(C_2H_5)_2$, $-CH_2-N(C_3H_7)_2$, $CH_2-N(C_4H_9)_2$, $-CH_2-N(CH_3)(C_2H_5)$ and $-(CH_2)$-morpholinyl;

and

$R^{21}$ and $R^{22}$, independently of another, in each case represent hydrogen;

a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent (s) independently selected from the group consisting of -OH, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $-N(C_2H_5)_2$, -CN and $-NO_2$;

a radical selected from the group consisting of (2,3)-dihydro-1H-cyclopenta[b]indolyl, cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, diazepanyl, azocanyl, (2,5)-dihydrofuranyl, (2,5)-dihydrothiophenyl, (2,3)-dihydrofuranyl, (2,3)-dihydrofuranyl, (2,5)-dihydro-1H-pyrrolyl, (2,3)-dihydro-1H-pyrrolyl, tetrahydrothiopyranyl, tetrahydropyranyl, (3,4)-dihydro-2H-pyranyl, (3,4)-dihydro-2H-thiopyranyl, (1,2,3,6)-tetrahydropyridinyl, (1,2,3,4)-tetrahydropyridinyl, (1,2,5,6)-tetrahydropyridinyl, [1,3]-oxazinanyl, hexahydropyrimidinyl, (5,6)-dihydro-4H-pyrimidinyl, oxazolidinyl, (1,3)-dioxanyl, (1,4)-dioxanyl, (1,3)-dioxolanyl, indolinyl, isoindolinyl,

decahydronaphthyl, (1,2,3,4)-tetrahydroquinolinyl, (1,2,3,4)-tetrahydroisoquinolinyl, octahydro-cyclopenta[c]pyrrolyl, (1,3,4,7,9a)-hexahydro-2H-quinolizinyl, (1,2,3,5,6,8a)-hexahydro-indolizinyl, decahydroquinolinyl, dodecahydro-carbazolyl, 9H-carbazolyl, decahydroisoquinolinyl, (6,7)-dihydro-4H-thieno[3,2-c]pyridinyl, (2,3)-dihydro-1H-benzo[de]isoquinolinyl, (1,2,3,4)-tetrahydroquinoxazlinyl, adamantyl, bicyclo[2.2.1]heptyl, bicyclo[3.1.1]heptyl, norbornenyl, 8-aza-bicyclo[3.2.1]octyl and 8-aza-spiro[4.5]decanyl, which may be bonded via a $-(CH_2)-$, $-(CH_2)-(CH_2)-$, $-(CH_2)-(CH_2)-(CH_2)-$ or $-CH=CH-$group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-O-CH_2-O-CH_3$, $-O-CH_2-CH_2-O-CH_3$, $-O-CH_2-O-C_2H_5$, $-C(OCH_3)(C_2H_5)_2$, $-C(OCH_3)(CH_3)_2$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, -C(=O)-OH, $-C(=O)-O-CH_3$, $-C(=O)-O-C_2H_5$, $-C(=O)-O-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, -S

$(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, cyclopentyl, cyclohexyl, pyrrolidinyl and piperidinyl;
or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or $-CH=CH-$ group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**14.** A compound according to one or more of claims 1 to 13, **characterized in that**

X is O or S;
$R^1$ represents a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of $-CF_3$, $-CH_2-Cl$, methyl, ethyl, n-propyl, isopropyl, n-butyl, isobutyl, 2-butyl, tert-butyl, n-pentyl, 2-pentyl, n-hexyl, $-NO_2$, F, Cl, Br, I, -CN, $-OCF_3$, $-SCF_3$, $-SCF_2H$, $-SCFH_2$, phenoxy and thiophenyl, whereby the thiophenyl radical can be substituted with 1, 2 or 3 substituents independently selected from the group consisting of F, Cl, Br, methyl, ethyl and n-propyl;
or a radical selected from the group consisting of cyclopropyl, cyclobutyl, cyclopentyl, cyclohexyl, cycloheptyl, cyclooctyl, cyclononyl, cycfopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, F, Cl, Br, I, -CN, $-OCF_3$, $-SCF_3$, $-SCF_2H$ and $-SCFH_2$;
$R^2$ represents a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl, isoindolyl, pyrimidinyl, pyrazinyl, quinolinyl, isoquinolinyl, benzo[b]furanyl, benzo[b]thiophenyl, benzo[2,1,3]thiadiazolyl, [1,2,3]-benzothiadiazolyl, [2,1,3]-benzoxadiazolyl, [1,2,3]-benzoxadiazolyl, benzoxazolyl, benzthiazolyl, benzisoxazolyl, benzisothiazolyl and imidazo[2,1-b]thiazolyl, which may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, F, Cl, Br, I, -CN, $-OCF_3$, $-SCF_3$, $-SCF_2H$ and $-SCFH_2$;
or a radical selected from the group consisting of cyclopropyl, cyclobutyl, cyclopentyl, cyclohexyl, cycfoheptyl, cyclooctyl, cyclononyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl and diazepanyl, which may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, F, Cl, Br, I, -CN, $-OCF_3$, $-SCF_3$, $-SCF_2H$ and $-SCFH_2$;
$R^3$ represents a radical selected from the group consisting of

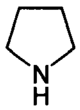, 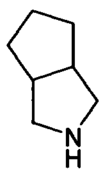, 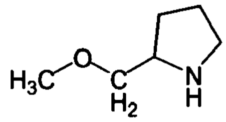, 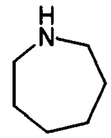, 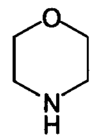, 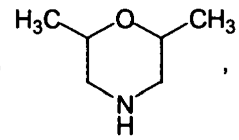,

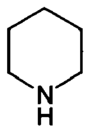, 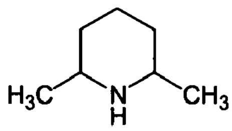, 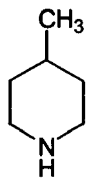, 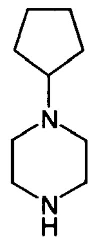, 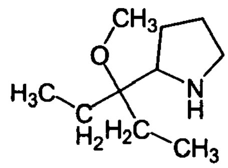, 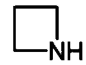,

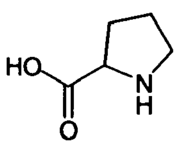, 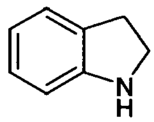, 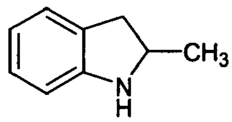, 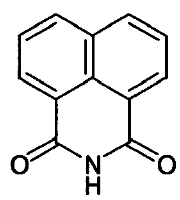, 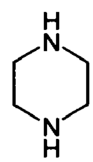,

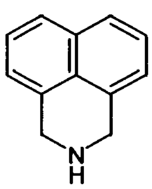, 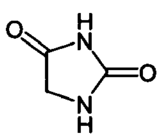, 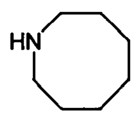, 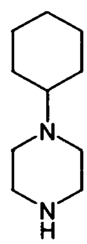, 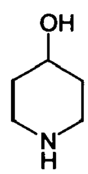, 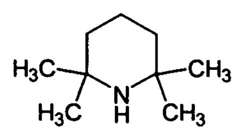,

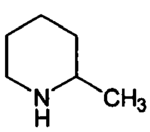, 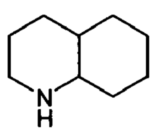, 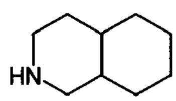, 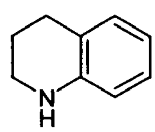, 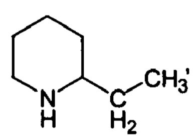,

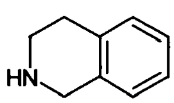, 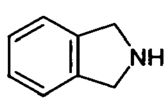, 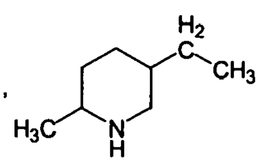, 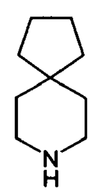 and 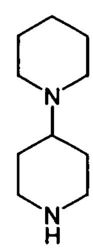 ;

which is in each case bonded to the pyrazoline compound of general formula I in any position of the cyclic part of the aforementioned radicals including the NH-groups, preferably said radicals are bonded to the pyrazoline

compound of general formula I at the nitrogen atom of the cyclic part of the aforementioned radicals;
a —O-$R^6$ moiety, a-$NR^7R^8$ moiety or a —$NR^9$-O-$R^{10}$ moiety;
$R^4$ represents F; Cl; Br; I; -CN; -$NO_2$; -NC; -OH; -$NH_2$; -SH; -C(=O)-H;
a radical selected from the group consisting of methyl, -$CF_3$, -$CH_2F$, -$CF_2H$, -$C_2F_5$, ethyl, -$CH_2$-CN, -$CH_2$-OH, n-propyl, isopropyl, -$CH_2$-$CH_2$-CN, -$CH_2$-$CH_2$-OH, n-butyl, -$CH_2$-$CH_2$-$CH_2$-CN, -$CH_2$-$CH_2$-$CH_2$-OH, isobutyl, sec-butyl, tert-butyl, n-pentyl, 2-pentyl, 3-pentyl, neo-pentyl, n-hexyl, 2-hexyl and 3-hexyl;
a radical selected from the group consisting of phenyl, naphthyl, pyridinyl, furyl (furanyl), thienyl (thiophenyl) and pyrrolyl, which may be bonded via a —($CH_2$)-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, F, Cl and Br;
a —O-$R^{11}$ moiety; a -S-$R^{12}$ moiety, a -NH-$R^{13}$ moiety or a -$NR^{14}R^{15}$ moiety;
$R^5$ represents H or an alkyl radical selected from the group consisting of methyl, ethyl, n-propyl, isopropyl and n-butyl;
$R^6$ represents a hydrogen atom; a radical selected from the group consisting of methyl, -$CF_3$, -$CH_2F$, -$CF_2H$, -$CH_2$-O-$CH_3$, -$C_2F_5$, -$CH_2$-$CH_2$-F, ethyl, -$CH_2$-CN, -$CH_2$-OH, n-propyl, isopropyl, -$CH_2$-$CH_2$-CN, -$CH_2$-$CH_2$-OH, -$CH_2$-$CH_2$-$OCH_3$, n-butyl, -$CH_2$-$CH_2$-$CH_2$-CN, -$CH_2$-$CH_2$-$CH_2$-OH,- $CH_2$-$CH_2$-$CH_2$-O-$CH_3$, isobutyl, sec-butyl, tert-butyl, n-pentyl, -$CH_2$-$CH_2$-$CH_2$-$CH_2$-O-$CH_3$, 2-pentyl, 3-pentyl, neo-pentyl, n-hexyl, 2-hexyl, 3-hexyl, n-heptyl and n-octyl;
a radical selected from the group consisting of cyclopropyl, cyclobutyl, cyclopentyl, cyclohexyl, cycloheptyl, pyrrolidinyl, piperidinyl, piperazinyl, homopiperazinyl, morpholinyl, imidazolidinyl, azepanyl, 8-azabicyclo[3.2.1] octyl and diazepanyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of oxo (=O), thioxo (=S), methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$ and -O-$C(CH_3)_3$;
a radical selected from the group consisting of pyridinyl, furyl (furanyl), thienyl (thiophenyl), pyrrolyl, oxazolyl, isoxazolyl, thiazolyl, isothiazolyl and imidazolyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of methyl, ethyl, n-propyl, isopropyl, n-butyl, isobutyl, 2-butyl, tert-butyl, n-pentyl, 2-pentyl, n-hexyl, -O-$CH_3$, -O-$C_2H_5$, -O-$CH_2$-$CH_2$-$CH_3$, -O-$CH(CH_3)_2$, -O-$CH_2$-$CH_2$-$CH_2$-$CH_3$, -O-$C(CH_3)_3$, F, Cl, Br, I, -O-C(=O)-$CH_3$, -O-C(=O)-$C_2H_5$, -O-C(=O)-$CH(CH_3)_2$, -O-C (=O)-$CH_2$-$CH_2$-$CH_3$, -$CH_2$-$N(CH_3)_2$, -($CH_2$)-$N(C_2H_5)_2$, -$CH_2$-$N(C_3H_7)_2$, -$CH_2$-$N(C_4H_9)_2$, -$CH_2$-$N(CH_3)(C_2H_5)$ and —($CH_2$)-morpholinyl;
a —$P(=O)(OR^{16})_2$ moiety; a —C(=O)-$OR^{17}$ moiety; a —C(=O)-NH-$R^{18}$ moiety or a —C(=O)-$R^{19}$ moiety.
$R^7$ and $R^8$, independently of another, in each case represent a hydrogen atom;
a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, -$CH_2$-$NH_2$, -$CH_2$-$N(CH_3)_2$, -$CH_2$-CH-$NH_2$, -$CH_2$-$CH_2$-$N(CH_3)_2$, -$CH_2$-$CH_2$-$N(C_2H_5)_2$, -$CH_2$-$CH_2$-$CH_2$-$NH_2$, -$CH_2$-$CH_2$-$CH_2$-$N(CH_3)_2$ and —$CH_2$-$CH_2$-$CH_2$-$N(C_2H_5)_2$;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl, [1,2,3,4]-tetrahydronaphthyl and bicyclo[2.2.1]heptyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of -OH, methyl, ethyl, n-propyl, isopropyl, n-butyl, isobutyl, 2-butyl, tert-butyl, n-pentyl, 2-pentyl and n-hexyl;
a radical selected from the group consisting of phenyl, naphthyl, pyridinyl, furyl (furanyl), thienyl (thiophenyl) and triazolyl, which may be bonded via a —($CH_2$)-, -($CH_2$)-($CH_2$)-, -($CH_2$)-($CH_2$)-($CH_2$)- or —CH=CH-group and/or may optionally be substituted with 1, 2, 3, 4 or 5 substituent(s) independently selected from the group consisting of —$CF_3$, methyl, ethyl, n-propyl, isopropyl, n-butyl, isobutyl, 2-butyl, tert-butyl, n-pentyl, 2-pentyl, n-hexyl, F, Cl and Br;
a —$S(=O)_2$-$R^{20}$ moiety;
a —$NR^{21}R^{22}$ moiety;
a radical selected from the group consisting of

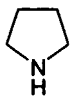 , 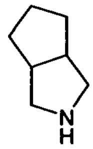 , 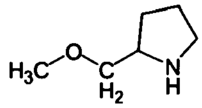 , 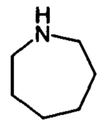 , 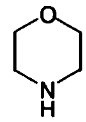 , 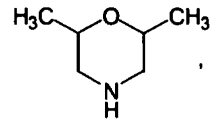 ,

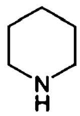 , 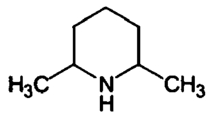 , 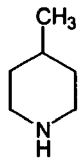 , 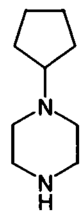 , 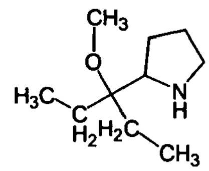 , 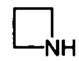 ,

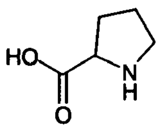 , 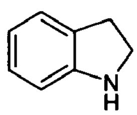 , 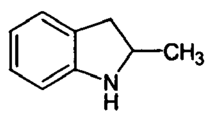 , 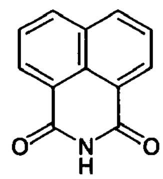 , 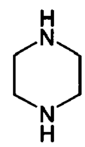 ,

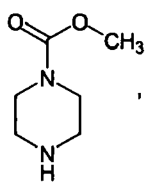 , 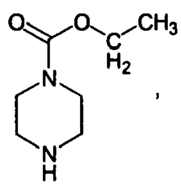 , 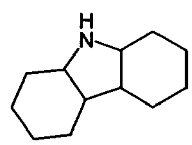 , 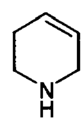 , 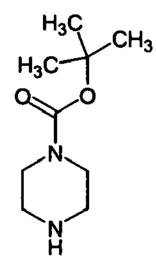 ,

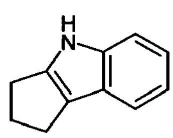 , 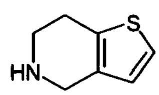 , 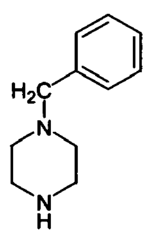 , 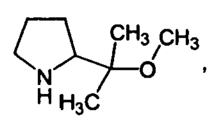 , 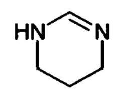 ,

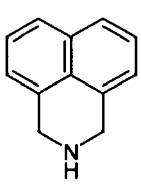 , 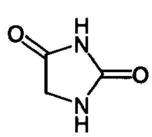 , 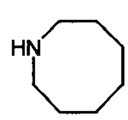 , 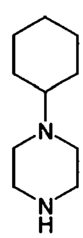 , 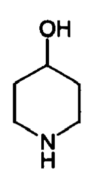 , 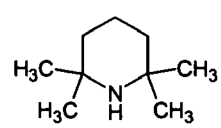 ,

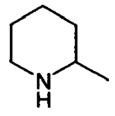, 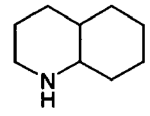, 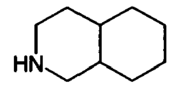, 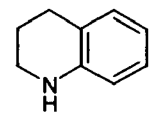, 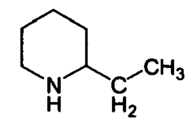,

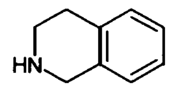, 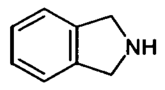, 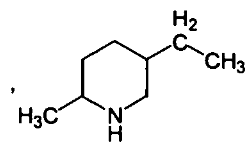, 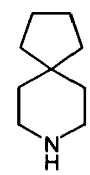 and 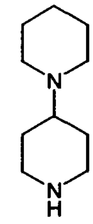 ;

which is in each case bonded to the pyrazoline compound of general formula I in any position of the cyclic part of the aforementioned radicals including the NH-groups, optionally via a $-(CH_2)-$ or $-(CH_2)-(CH_2)-$group, preferably said radicals are bonded to the pyrazoline compound of general formula I at the nitrogen atom of the cyclic part of the aforementioned radicals;

$R^9$ represents hydrogen;

$R^{10}$, $R^{11}$, $R^{12}$, $R^{13}$, $R^{14}$, $R^{15}$ and $R^{20}$, independently of another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, vinyl, n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of $-OH$, F, Cl, Br, I, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-NH_2$, $-NH-CH_3$, $-NH-C_2H_5$, $-N(CH_3)_2$, $-N(C_2H_5)_2$, -CN and $-NO_2$;

a radical selected from the group consisting of cyclopropyl, cyclobutyl, cyclopentyl, cyclohexyl, cycloheptyl, cyclooctyl, cyclononyl, cyclodecyl, cyclopentenyl, cyclohexenyl, cycloheptenyl, cyclooctenyl, pyrrolidinyl, piperidinyl, piperazinyl, homopiperazinyl, morpholinyl, aziridinyl, azetidinyl, imidazolidinyl, thiomorpholinyl, pyrazolidinyl, tetrahydrofuranyl, tetrahydrothiophenyl, azepanyl, 8-azabicyclo[3.2.1]octyl and diazepanyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of oxo (=O), thioxo (=S), $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $-(CH_2)-$, $-(CH_2)-(CH_2)-$, $-(CH_2)-(CH_2)-(CH_2)-$ or $-CH=CH-$group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$ and $-N(C_2H_5)_2$;

$R^{16}$, $R^{17}$, $R^{18}$ and $R^{19}$, independently of one another, in each case represent a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, vinyl,

n-propenyl, n-butenyl, n-pentenyl, n-hexenyl, ethinyl, propinyl, n-butinyl, n-pentinyl and n-hexinyl, which may optionally be substituted with 1, 2, 3, 4, 5, 6, 7, 8 or 9 substituent(s) independently selected from the group consisting of $NH_2$, $-NH-CH_3$, $-NH-C_2H_5$, $-N(CH_3)_2$, $—N(C_2H_5)_2$, $-NH-C(=O)-CH_3$, $-NH-C(=O)-C_2H_5$, $-NH-C(=O)-C(CH_3)_3$, $-NH-C(=O)-O-CH_3$, $-NH-C(=O)-O-C_2H_5$, $-NH-C(=O)-O-C(CH_3)_3$, $-C(=O)-NH-CH_3$, $-C(=O)-NH-C_2H_5$, $-C(=O)-NH-C(CH_3)_3$, $-C(=O)-N(CH_3)_2$, $-C(=O)-N(C_2H_5)_2$, -OH, -C(=O)-OH, $-C(=O)-O-CH_3$, $-C(=O)-O-C_2H_5$, $-C(=O)-O-C(CH_3)_3$, $-C(=O)-CH_3$, $-C(=O)-C_2H_5$ and $-C(=O)-C(CH_3)_3$;
or a radical selected from the group consisting of phenyl, naphthyl, pyridinyl, furyl (furanyl), thienyl (thiophenyl), pyrrolyl, oxazolyl, isoxazolyl, thiazolyl, isothiazolyl, imidazolyl, pyrazolyl, oxadiazolyl, thiadiazolyl, triazolyl, pyridazinyl, indolyl and isoindolyl, which may be bonded via a $—(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of $—CF_3$, methyl, ethyl, n-propyl, isopropyl, n-butyl, isobutyl, 2-butyl, tert-butyl, n-pentyl, 2-pentyl, n-hexyl, $-O-CH_3$, $-O-C_2H_5$, $-O-CH_2-CH_2-CH_3$, $-O-CH(CH_3)_2$, $-O-CH_2-CH_2-CH_2-CH_3$, $-O-C(CH_3)_3$, $-S-CH_3$, $-S-C_2H_5$, $-S-CH_2-CH_2-CH_3$, $-S-CH(CH_3)_2$, $-S-CH_2-CH_2-CH_2-CH_3$, $-S-C(CH_3)_3$, F, Cl, Br, I, -CN, $-OCF_3$, $-SCF_3$, $-SCF_2H$, $-SCFH_2$, -OH, -SH, $-NO_2$, -CHO, $-C(=O)-CH_3$, $-C(=O)-C_2H_5$, $-C(=O)-C(CH_3)_3$, $-CF_2H$, $-CFH_2$, $-C(=O)-NH_2$, $-C(=O)-NH-CH_3$, $-C(=O)-NH-C_2H_5$, $-C(=O)-NH-C_3H_7$, $-C(=O)-N(CH_3)_2$, $-C(=O)-N(C_2H_5)_2$, $-S(=O)-CH_3$, $-S(=O)-C_2H_5$, $-S(=O)-C_3H_7$, $-S(=O)_2-CH_3$, $-S(=O)_2-C_2H_5$, $-S(=O)_2-C_3H_7$, $-NH_2$, $-NH-CH_3$, $-NH-C_2H_5$, $-N(CH_3)_2$, $-N(C_2H_5)_2$, $-O-C(=O)-CH_3$, $-O-C(=O)-C_2H_5$, $-O-C(=O)-CH(CH_3)_2$, $-O-C(=O)-CH_2-CH_2-CH_3$, $-CH_2-N(CH_3)_2$, $-(CH_2)-N(C_2H_5)_2$, $-CH_2-N(C_3H_7)_2$, $-CH_2-N(C_4H_9)_2$, $-CH_2-N(CH_3)(C_2H_5)$ and $—(CH_2)$-morpholinyl;

and

$R^{21}$ and $R^{22}$, independently of another, in each case represent hydrogen;
a radical selected from the group consisting of methyl, ethyl, n-propyl, isopropyl, n-butyl, isobutyl, sec-butyl, tert-butyl, n-pentyl, 2-pentyl, 3-pentyl, neo-pentyl, n-hexyl, 2-hexyl, 3-hexyl, n-heptyl, 2-heptyl, 3-heptyl, 4-heptyl, n-octyl, 2-octyl, 3-octyl, 4-octyl, 2-(6-methyl)-heptyl, 2-(5-methyl)-heptyl, 2-(5-methyl)-hexyl, 2-(4-methyl)-hexyl, 2-(7-methyl)-octyl; 2-(6-methyl)-octyl, $-CH_2-NH_2$, $-CH-N(CH_3)_2$, $-CH_2-CH-NH_2$, $-CH_2-CH_2-N(CH_3)_2$, $-CH_2-CH_2-N(C_2H_5)_2$, $-CH_2-CH_2-CH_2-NH_2$, $-CH_2-CH_2-CH_2-N(CH_3)_2$ and $—CH_2-CH_2-CH_2-N(C_2H_5)_2$;
a radical selected from the group consisting of cyclopropyl, cyclobutyl, cyclopentyl, cyclohexyl, cycloheptyl, cyclooctyl, cyclononyl, cyclodecyl, cycloundecyl, cyclododecyl, cyclotridecyl, cyclotetradecyl and bicyclo[2.2.1]heptyl, which may be bonded via a $—(CH_2)-$, $-(CH_2)-(CH_2)-$, $-(CH_2)-(CH_2)-(CH_2)-$ or —CH=CH-group and/or may optionally be substituted with 1, 2, 3, 4 or 5 substituent(s) independently selected from the group consisting of —OH, methyl, ethyl, n-propyl, isopropyl, n-butyl, isobutyl, 2-butyl, tert-butyl, n-pentyl, 2-pentyl and n-hexyl;
a radical selected from the group consisting of phenyl, naphthyl, pyridinyl, furyl (furanyl), thienyl (thiophenyl) and triazolyl, which may be bonded via a $-(CH_2)-$, $-(CH_2)-(CH_2)-$, $-(CH_2)-(CH_2)-(CH_2)-$ or -CH=CH-group and/or may optionally be substituted with 1, 2, 3, 4 or 5 substituent(s) independently selected from the group consisting of $-CF_3$, methyl, ethyl, n-propyl, isopropyl, n-butyl, isobutyl, 2-butyl, tert-butyl, n-pentyl, 2-pentyl, n-hexyl, F, Cl and Br;
or a radical selected from the group consisting of

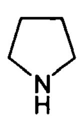 , 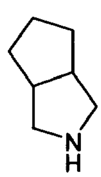 , 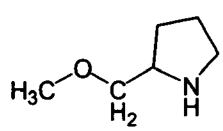 , 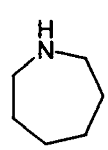 , 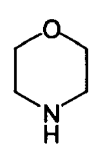 , 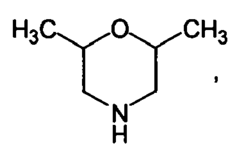 ,

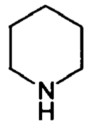 , 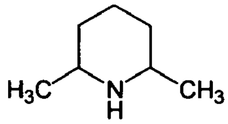 , 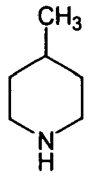 , 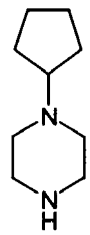 , 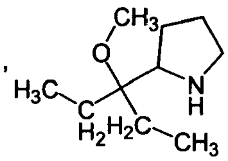 , 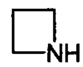 ,

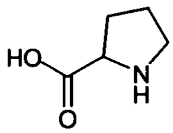 , 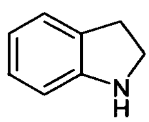 , 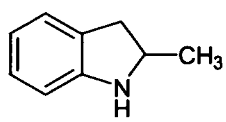 , 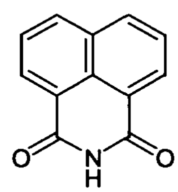 , 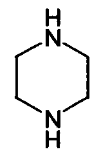 ,

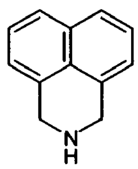 , 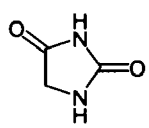 , 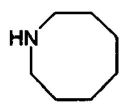 , 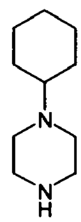 , 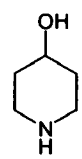 , 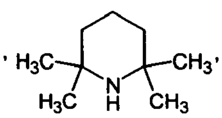 ,

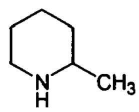 , 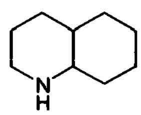 , 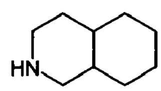 , 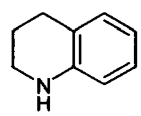 , 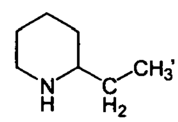 ,

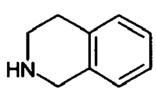 , 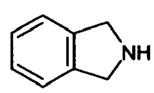 , 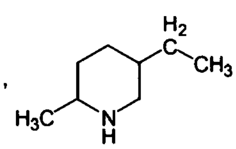 , 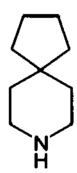 and 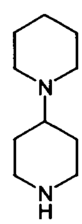 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**15.** A compound according to one or more of claims 1 to 14, **characterized in that**

X is O
$R^1$ represents a radical selected from the group consisting of phenyl and thienyl (thiophenyl), which may optionally be substituted with 1, 2, 3, 4 or 5 substituent(s) independently selected from the group consisting of F, Cl, Br and I;
$R^2$ represents a phenyl radical, which may be substituted with 1, 2, 3, 4 or 5 substituent(s) independently selected from the group consisting of F, Cl, Br and I;
$R^3$ a radical selected from the group consisting of

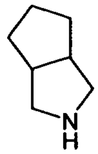 , 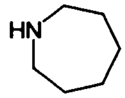 , 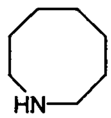 , 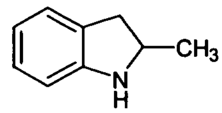 ,

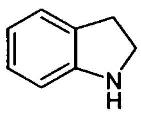 , 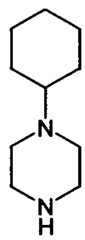 , 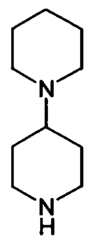 , 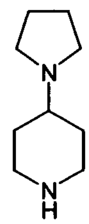 ,

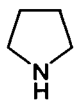 , 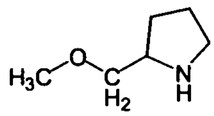 , 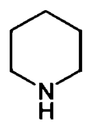 , 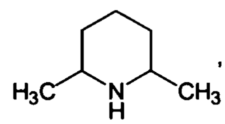 , 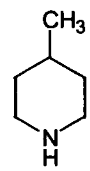 ,

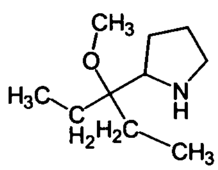 , 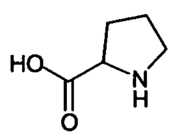 , 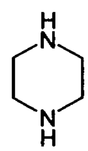 , 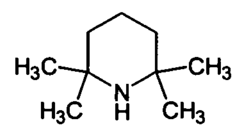 ,

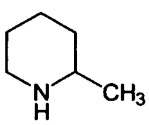 , 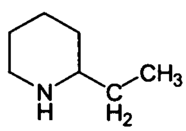 , 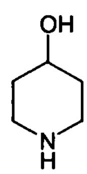 and 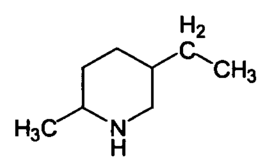 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals;
a —$OR^6$-moiety or a -$NR^7R^8$ moiety;
$R^4$ represents F; Cl; Br; I; -OH, -CN; -C(=O)-H;
a radical selected from the group consisting of methyl, $-CF_3$, $-CH_2F$, $-CF_2H$, $-C_2F_5$, ethyl, $-CH_2-CN$, $-CH_2-OH$, n-propyl, isopropyl, $-CH_2-CH_2-CN$, $-CH_2-CH_2-OH$, n-butyl, $-CH_2-CH_2-CH_2-CN$, $-CH_2-CH_2-CH_2-OH$, isobutyl, sec-butyl, tert-butyl, n-pentyl, 2-pentyl, 3-pentyl, neo-pentyl, n-hexyl, 2-hexyl and 3-hexyl;
a benzyl radical or a —O-$R^{11}$ moiety;
$R^5$ represents H or a radical selected from the group consisting of methyl, ethyl, n-propyl, iso-propyl and n-butyl;
$R^6$ represents a hydrogen atom, a radical selected from the group consisting of methyl, ethyl, n-propyl, isopropyl, n-butyl and tert-butyl or a radical selected from the group consisting of cyclopropyl, cyclobutyl, cyclopentyl,

cyclohexyl and cycloheptyl;
$R^7$ represents a hydrogen atom;
$R^8$ represents a radical selected from the group consisting of cyclopropyl, cyclobutyl, cyclopentyl, cyclohexyl and cycloheptyl, which may be bonded via a $-(CH_2)-$, $-(CH_2)-(CH_2)-$ or $-(CH_2)-(CH_2)-(CH_2)-$group; or a radical selected from the group consisting of

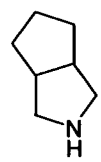 , 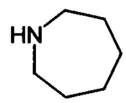 , 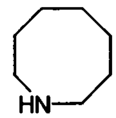 , 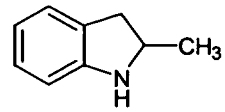 ,

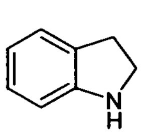 , 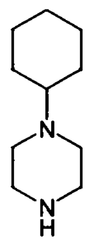 , 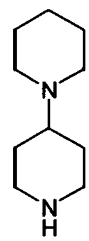 , 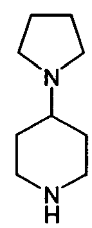 ,

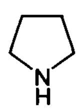 , 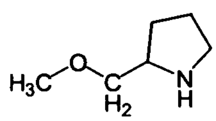 , 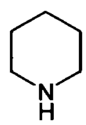 , 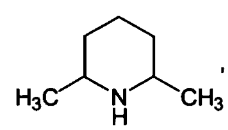 , 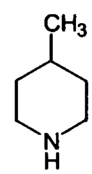 ,

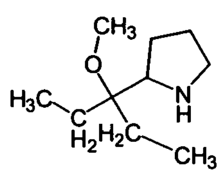 , 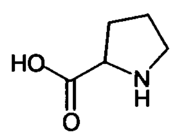 , 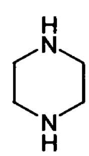 , 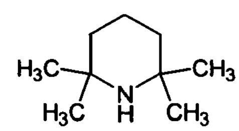 ,

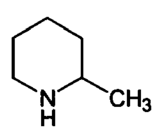 , 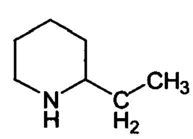 , 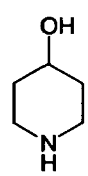 and 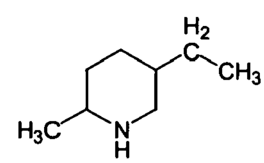 ;

which is in each case bonded to the pyrazoline compound of general formula I in any position of the cyclic part of the aforementioned radicals including the NH-groups, preferably said radicals are bonded to the pyrazoline compound of general formula I at the nitrogen atom of the cyclic part of the aforementioned radicals; and
$R^{11}$ represents a radical selected from the group consisting of methyl, ethyl, n-propyl, isopropyl, n-butyl, isobutyl, sec-butyl, tert-butyl, n-pentyl, 2-pentyl, 3-pentyl and neo-pentyl;

optionally in form of one of its stereoisomers, preferably enantiomers or diastereomers, a racemate or in form of a mixture of at least two of its stereoisomers, preferably enantiomers and/or diastereomers, in any mixing ratio, or a corresponding N-oxide thereof, or a physiologically acceptable salt thereof, or a corresponding solvate thereof.

**16.** A compound or salt according to one or more of claims 1 to 15 selected from the group consisting of

[1] *cis*-5-(4-Chloro-phenyl)-1-(2,4-dichloro-phenyl)-4-methyl-4,5-dihydro-1H-pyrazole-3-carboxylic acid piperidin-1-ylamide hydro-chloride
[2] *trans*-5-(4-Chloro-phenyl)-1-(2,4-dichloro-phenyl)-4-methyl-4,5-dihydro-1H-pyrazole-3-carboxylic acid piperidin-1-ylamide hydro-chloride
[3] *cis*-5-(4-Chloro-phenyl)-1-(2,4-dichloro-phenyl)-4-methyl-4,5-dihydro-1H-pyrazole-3-carboxylic acid azepan-1-ylamide
[4] *trans*-5-(4-Chloro-phenyl)-1-(2,4-dichloro-phenyl)-4-methyl-4,5-dihydro-1H-pyrazole-3-carboxylic acid azepan-1-ylamide
[5] *cis*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone
[6] *trans*-[5-(4-Chloro-phenyl)-1-(2,4-dichloro-phenyl)-4-methyl-4,5-dihydro-1H-pyrazol-3-yl]-(4-cyclohexyl-piperazin-1-yl)-methanone
[7] *cis*-5-(4-Chloro-phenyl)-1-(2,4-dichloro-phenyl)-4-methyl-4,5-dihydro-1H-pyrazole-3-carboxylic acid pyrrolidin-1-ylamide
[8] *trans*-5-(4-Chloro-phenyl)-1-(2,4-dichloro-phenyl)-4-methyl-4,5-dihydro-1H-pyrazole-3-carboxylic acid pyrrolidin-1-ylamide
[9] *cis*-5-(4-Chloro-phenyl)-1-(2,4-dichloro-phenyl)-4-methyl-4,5-dihydro-1H-pyrazole-3-carboxylic acid (4-methyl-piperidin-1-yl)-amide
[10] *trans*-5-(4-Chloro-phenyl)-1-(2,4-dichloro-phenyl)-4-methyl-4,5-dihydro-1H-pyrazole-3-carboxylic acid (4-methyl-piperidin-1-yl)-amide
[11] *cis*-[5-(4-Chloro-phenyl)-1-(2,4-dichloro-phenyl)-4-methyl-4,5-dihydro-1H-pyrazol-3-yl]-piperidin-1-yl-methanone
[12] *trans*-[5-(4-Chloro-phenyl)-1-(2,4-dichloro-phenyl)-4-methyl-4,5-dihydro-1H-pyrazol-3-yl]-piperidin-1-yl-methanone
[13] *cis*-5-(4-Chloro-phenyl)-1-(2,4-dichloro-phenyl)-4-methyl-4,5-dihydro-1H-pyrazole-3-carboxylic acid (hexahydro-cyclopenta[c]pyrrol-2-yl)-amide
[14] *trans*-5-(4-Chloro-phenyl)-1-(2,4-dichloro-phenyl)-4-methyl-4,5-dihydro-1H-pyrazole-3-carboxylic acid (hexahydro-cyclopenta-[c]py-rrol-2-yl)-amide
[15] *cis*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide
[16] *trans*-5-(4-Chloro-phenyl)-1-(2,4-dichloro-phenyl)-4-methyl-4,5-dihydro-1H-pyrazole-3-carboxylic acid (2,3-dihydro-indol-1-yl)-amide
[17] *cis*-5-(4-Chloro-phenyl)-1-(2,4-dichloro-phenyl)-4-methyl-4,5-dihydro-1H-pyrazole-3-carboxylic acid cyclobutylamide
[18] 5-(4-Chloro-phenyl)-1-(2,4-dichloro-phenyl)-4-methyl-4,5-dihydro-1H-pyrazole-3-carboxylic acid cyclohexyl methyl-amide
[19] *cis*-5-(4-Chloro-phenyl)-1-(2,4-dichloro-phenyl)-4-methyl-4,5-dihydro-1H-py-razole-3-carboxylic acid
[20] *trans*-5-(4-Chloro-phenyl)-1-(2,4-dichloro-phenyl)-4-methyl-4,5-dihydro-1H-py-razole-3-carboxylic acid
[21] *cis*-5-(4-Chloro-phenyl)-1-(2,4-dichloro-phenyl)-4-ethyl-4,5-dihydro-1H-pyrazole-3-carboxylic acid
[22] *trans*-5-(4-Chloro-phenyl)-1-(2,4-dichloro-phenyl)-4-ethyl-4,5-dihydro-1H-pyrazole-3-carboxylic acid piperidin-1-ylamide
[23] *cis*-5-(4-Chloro-phenyl)-1-(2,4-dichloro-phenyl)-4-methyl-4,5-dihydro-1H-pyrazole-3-carboxylic acid methyl ester
[24] *trans*-5-(4-Chloro-phenyl)-1-(2,4-dichloro-phenyl)-4-methyl-4,5-dihydro-1H-pyrazole-3-carboxylic acid methyl ester
[25] *cis*-5-(4-Chloro-phenyl)-1-(2,4-dichloro-phenyl)-4-methyl-4,5-dihydro-1H-pyrazole-3-carboxylic acid ethyl ester
[26] *trans*-5-(4-Chloro-phenyl)-1-(2,4-dichloro-phenyl)-4-methyl-4,5-dihydro-1H-py-razole-3-carboxylic acid ethyl ester
[27] 5-(4-Chloro-phenyl)-1-(2,4-dichloro-phenyl)-4-methyl-4,5-dihydro-1H-pyrazole-3-carboxylic acid cyclohexyl ester
[28] 5-(4-Chloro-phenyl)-1-(2,4-dichloro-phenyl)-4,4-dimethyl-4,5-dihydro-1H-pyrazole-3-carboxylic acid
[29] 5-(4-Chloro-phenyl)-1-(2,4-dichloro-phenyl)-4,4-dimethyl-4,5-dihydro-1H-pyrazole-3-carboxylic acid piperidin-1-ylamide
[30] 5-(4-Chloro-phenyl)-1-(2,4-dichloro-phenyl)-4-trifluoromethyl-4,5-dihydro-1H-pyrazole-3-carboxylic acid piperidin-1-ylamide
[31] 5-(4-Chloro-phenyl)-1-(2,4-dichloro-phenyl)-4-hydroxy-4,5-dihydro-1H-pyrazole-3-carboxylic acid
[32] 5-(4-Chloro-phenyl)-1-(2,4-dichloro-phenyl)-4-hydroxy-4,5-dihydro-1H-pyrazole-3-carboxylic acid piperidin-1-ylamide

[33] 5-(4-Chloro-phenyl)-1-(2,4-dichloro-phenyl)-4-(2-hydroxy-ethyl)-4,5-dihydro-1H-pyrazole-3-carboxylic acid piperidin-1-ylamide
[34] 5-(4-Chloro-phenyl)-1-(2,4-dichloro-phenyl)-4-(2-hydroxy-ethyl)-4,5-dihydro-1H-pyrazole-3-carboxylic acid
[35] 5-(4-Chloro-phenyl)-1-(2,4-dichloro-phenyl)-4-fluoromethyl-4,5-dihydro-1H-pyrazole-3-carboxylic acid piperidin-1-ylamide
[36] 5-(4-Chloro-phenyl)-1-(2,4-dichloro-phenyl)-4-fluoromethyl-4,5-dihydro-1H-pyrazole-3-carboxylic acid
[37] 5-(4-Chloro-phenyl)-1-(2,4-dichloro-phenyl)-4-formyl-4,5-dihydro-1H-pyrazole-3-carboxylic acid piperidin-1-ylamide
[38] 5-(4-Chloro-phenyl)-1-(2,4-dichloro-phenyl)-4-formyl-4,5-dihydro-1H-pyrazole-3-carboxylic acid
[39] 1-(2,4-Dichloro-phenyl)-5-(4-fluoro-phenyl)-4-methyl-4,5-dihydro-1H-pyrazole-3-carboxylic acid piperidin-1-ylamide
[40] 5-(4-Chloro-phenyl)-4-cyano-1-(2,4-dichloro-phenyl)-4,5-dihydro-1H-pyrazole-3-carboxylic acid
[41] 1-(2,4-Dichloro-phenyl)-5-(4-fluoro-phenyl)-4-methyl-4,5-dihydro-1H-pyrazole-3-carboxylic acid piperidin-1-ylamide
[42] 1-(2,4-Dichloro-phenyl)-4-ethyl-5-(4-fluoro-phenyl)-4,5-dihydro-1H-pyrazole-3-carboxylic acid piperidin-1-ylamide
[43] 1-(2,4-Dichloro-phenyl)-5-(4-fluoro-phenyl)-4,4-dimethyl-4,5-dihydro-1H-pyrazole-3-carboxylic acid piperidin-1-ylamide
[44] 1-(2,4-Dichloro-phenyl)-5-(4-fluoro-phenyl)-4-trifluoromethyl-4,5-dihydro-1H-pyrazole-3-carboxylic acid piperidin-1-ylamide
[45] 1-(2,4-Dichloro-phenyl)-5-(4-fluoro-phenyl)-4-hydroxy-4,5-dihydro-1H-pyrazole-3-carboxylic acid piperidin-1-ylamide
[46] 1-(2,4-Dichloro-phenyl)-5-(4-fluoro-phenyl)-4-(2-hydroxy-ethyl)-4,5-dihydro-1H-pyrazole-3-carboxylic acid piperidin-1-ylamide
[47] 1-(2,4-Dichloro-phenyl)-4-fluoromethyl-5-(4-fluoro-phenyl)-4,5-dihydro-1H-pyrazole-3-carboxylic acid piperidin-1-ylamide
[48] 1-(2,4-Dichloro-phenyl)-5-(4-fluoro-phenyl)-4-formyl-4,5-dihydro-1H-pyrazole-3-carboxylic acid piperidin-1-ylamide
[49] 4-Cyano-1-(2,4-dichloro-phenyl)-5-(4-fluoro-phenyl)-4,5-dihydro-1H-pyrazole-3-carboxylic acid piperidin-1-ylamide
[50] 5-(4-Bromo-phenyl)-1-(2,4-dichloro-phenyl)-4-methyl-4,5-dihydro-1H-pyrazole-3-carboxylic acid piperidin-1-ylamide
[51] 5-(4-Bromo-phenyl)-1-(2,4-dichloro-phenyl)-4-ethyl-4,5-dihydro-1H-pyrazole-3-carboxylic acid piperidin-1-ylamide
[52] 5-(4-Bromo-phenyl)-1-(2,4-dichloro-phenyl)-4,4-dimethyl-4,5-dihydro-1H-pyrazole-3-carboxylic acid piperidin-1-ylamide
[53] 5-(4-Bromo-phenyl)-1-(2,4-dichloro-phenyl)-4-trifluoromethyl-4,5-dihydro-1H-pyrazole-3-carboxylic acid piperidin-1-ylamide
[54] 5-(4-Bromo-phenyl)-1-(2,4-dichloro-phenyl)-4-hydroxy-4,5-dihydro-1H-pyrazole-3-carboxylic acid piperidin-1-ylamide
[55] 5-(4-Bromo-phenyl)-1-(2,4-dichloro-phenyl)-4-(2-hydroxy-ethyl)-4,5-dihydro-1H-pyrazole-3-carboxylic acid piperidin-1-ylamide
[56] 5-(4-Bromo-phenyl)-1-(2,4-dichloro-phenyl)-4-fluoromethyl-4,5-dihydro-1 H-pyrazole-3-carboxylic acid piperidin-1-ylamide
[57] 5-(4-Bromo-phenyl)-1-(2,4-dichloro-phenyl)-4-formyl-4,5-dihydro-1H-pyrazole-3-carboxylic acid piperidin-1-ylamide
[58] 5-(4-Bromo-phenyl)-4-cyano-1-(2,4-dichloro-phenyl)-4,5-dihydro-1H-pyrazole-3-carboxylic acid piperidin-1-ylamide
[59] cis-5-(5-Chloro-thiophen-2-yl)-1-(2,4-dichloro-phenyl)-4-methyl-4,5-dihydro-1H-pyrazole-3-carboxylic acid piperidin-1-ylamide
[60] trans-5-(5-Chloro-thiophen-2-yl)-1-(2,4-dichloro-phenyl)-4-methyl-4,5-dihydro-1 H-pyrazole-3-carboxylic acid piperidin-1-ylamide
[61] cis-5-(5-Chloro-thiophen-2-yl)-1-(2,4-dichloro-phenyl)-4-ethyl-4,5-dihydro-1 H-pyrazole-3-carboxylic acid piperidin-1-ylamide
[62] trans-5-(5-Chloro-thiophen-2-yl)-1-(2,4-dichloro-phenyl)-4-ethyl-4,5-dihydro-1H-pyrazole-3-carboxylic acid piperidin-1-ylamide
[63] 5-(5-Chloro-thiophen-2-yl)-1-(2,4-dichloro-phenyl)-4,4-dimethyl-4,5-dihydro-1H-pyrazole-3-carboxylic acid piperidin-1-ylamide
[64] 5-(5-Chloro-thiophen-2-yl)-1-(2,4-dichloro-phenyl)-4-trifluoromethyl-4,5-dihydro-1H-pyrazole-3-carboxylic acid piperidin-1-ylamide
[65] 5-(5-Chloro-thiophen-2-yl)-1-(2,4-dichloro-phenyl)-4-hydroxy-4,5-dihydro-1H-pyrazole-3-carboxylic acid piperidin-1-ylamide
[66] 5-(5-Chloro-thiophen-2-yl)-1-(2,4-dichloro-phenyl)-4-(2-hydroxy-ethyl)-4,5-dihydro-1H-pyrazole-3-carboxylic acid piperidin-1-ylamide
[67] 5-(5-Chloro-thiophen-2-yl)-1-(2,4-dichloro-phenyl)-4-fluoromethyl-4,5-dihydro-1H-pyrazole-3-carboxylic acid piperidin-1-ylamide
[68] 5-(5-Chloro-thiophen-2-yl)-1-(2,4-dichloro-phenyl)-4-formyl-4,5-dihydro-1H-pyrazole-3-carboxylic acid piperidin-1-ylamide
[69] 5-(5-Chloro-thiophen-2-yl)-4-cyano-1-(2,4-dichloro-phenyl)-4,5-dihydro-1H-pyrazole-3-carboxylic acid piperidin-1-ylamide
[70] *cis*-5-(5-Bromo-thiophen-2-yl)-1-(2,4-dichloro-phenyl)-4-methyl-4,5-dihydro-1H-pyrazole-3-carboxylic acid piperidin-1-ylamide
[71] *trans*-5-(5-Bromo-thiophen-2-yl)-1-(2,4-dichloro-phenyl)-4-methyl-4,5-dihydro-1H-pyrazole-3-carboxylic acid piperidin-1-ylamide
[72] *cis*-5-(5-Bromo-thiophen-2-yl)-1-(2,4-dichloro-phenyl)-4-ethyl-4,5-dihydro-1H-pyrazole-3-carboxylic acid piperidin-1-ylamide
[73] *trans*-5-(5-Bromo-thiophen-2-yl)-1-(2,4-dichloro-phenyl)-4-ethyl-4,5-dihydro-1H-pyrazole-3-carboxylic acid piperidin-1-ylamide
[74] 5-(5-Bromo-thiophen-2-yl)-1-(2,4-di-chloro-phenyl)-4,4-dimethyl-4,5-dihydro-1H-pyrazole-3-carboxylic acid piperidin-1-ylamide
[75] *cis*-5-(4-Bromo-thiophen-2-yl)-1-(2,4-dichloro-phenyl)-4-methyl-4,5-dihydro-1H-pyrazole-3-carboxylic acid piperidin-1-ylamide
[76] *trans*-5-(4-Bromo-thiophen-2-yl)-1-(2,4-dichloro-phenyl)-4-methyl-4,5-dihydro-1H-pyrazole-3-carboxylic acid piperidin-1-ylamide
[77] *cis*-5-(4-Bromo-thiophen-2-yl)-1-(2,4-dichloro-phenyl)-4-ethyl-4,5-dihydro-1H-pyrazole-3-carboxylic acid piperidin-1-ylamide
[78] *trans*-5-(4-Bromo-thiophen-2-yl)-1-(2,4-dichloro-phenyl)-4-ethyl-4,5-dihydro-1H-pyrazole-3-carboxylic acid piperidin-1-ylamide
[79] 5-(4-Bromo-thiophen-2-yl)-1-(2,4-dichloro-phenyl)-4,4-dimethyl-4,5-dihydro-1H-pyrazole-3-carboxylic acid piperidin-1-ylamide
[80] 5-(5-Bromo-thiophen-2-yl)-1-(2,4-dichloro-phenyl)-4-trifluoromethyl-4,5-dihydro-1H-pyrazole-3-carboxylic acid piperidin-1-ylamide
[81] 5-(5-Bromo-thiophen-2-yl)-1-(2,4-di-chloro-phenyl)-4-hydroxy-4,5-dihydro-1H-pyrazole-3-carboxylic acid piperidin-1-ylamide
[82] 5-(5-Bromo-thiophen-2-yl)-1-(2,4-di-chloro-phenyl)-4-(2-hydroxy-ethyl)-4,5-dihydro-1H-pyrazole-3-carboxylic acid piperidin-1-ylamide
[83] 5-(5-Bromo-thiophen-2-yl)-1-(2,4-di-chloro-phenyl)-4-fluoromethyl-4,5-dihydro-1H-pyrazole-3-carboxylic acid piperidin-1-ylamide
[84] 5-(5-Bromo-thiophen-2-yl)-1-(2,4-di-chloro-phenyl)-4-formyl-4,5-dihydro-1H-pyrazole-3-carboxylic acid piperidin-1-ylamide
[85] 5-(5-Bromo-thiophen-2-yl)-4-cyano-1-(2,4-dichloro-phenyl)-4,5-dihydro-1H-pyrazole-3-carboxylic acid piperidin-1-ylamide
[86] cis-5-(5-Chloro-thiophen-2-yl)-1-(2,4-dichloro-phenyl)-4-methyl-4,5-dihydro-1H-pyrazole-3-carboxylic acid
[87] trans-[5-(5-Chloro-thiophen-2-yl)-1-(2,4-dichloro-phenyl)-4-methyl-4,5-dihydro-1H-pyrazol-3-yl]-piperidin-1-yl-methanone
[88] *cis*-5-(5-Bromo-thiophen-2-yl)-1-(2,4-dichloro-phenyl)-4-methyl-4,5-dihydro-1H-pyrazole-3-carboxylic acid
[89] *trans*-5-(5-Bromo-thiophen-2-yl)-1-(2,4-dichloro-phenyl)-4-methyl-4,5-dihydro-1H-pyrazole-3-carboxylic acid
[90] *cis*-5-(4-Bromo-thiophen-2-yl)-1-(2,4-dichloro-phenyl)-4-methyl-4,5-dihydro-1H-pyrazole-3-carboxylic acid and
[91] *trans*-5-(4-Bromo-thiophen-2-yl)-1-(2,4-dichloro-phenyl)-4-methyl-4,5-dihydro-1 H-pyrazole-3-carboxylic acid;

optionally in form of one of its stereoisomers, preferably enantiomers or diastereomers, a racemate or in form of a mixture of at least two of its stereoisomers, preferably enantiomers and/or diastereomers, in any mixing ratio, or a

corresponding N-oxide thereof, or a physiologically acceptable salt thereof, or a corresponding solvate thereof.

**17.** Process for the preparation of a compound of general formula I according to one or more of claims 1 to 16, wherein $R^5$ represents hydrogen, **characterized in that** at least one compound of general formula II,

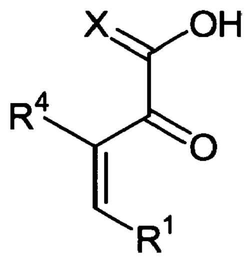

II,

wherein $R^1$, X and $R^4$ have the meaning according to one or more of claims 1 to 16, is reacted with at least one compound of general formula III,

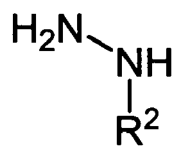

III,

or a corresponding salt thereof, wherein $R^2$ has the meaning according to one or more of claims 1 to 16, in a reaction medium, optionally in an inert atmosphere, optionally in the presence of at least one acid, to yield at least one compound of general formula IV,

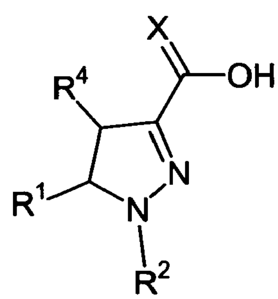

IV,

wherein $R^1$, X, $R^2$ and $R^4$ have the meaning according to one or more of claims 1 to 16, which is optionally isolated and/or purified,
and at least one compound of general formula IV is reacted with an activating agent in a reaction medium, optionally in an inert atmosphere, to yield at least one compound of general formula V,

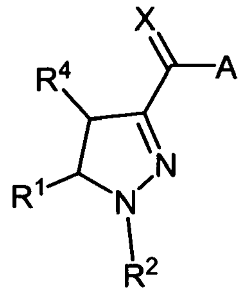

V,

wherein $R^1$, X, $R^2$ and $R^4$ have the meaning according to one or more of claims 1 to 16 and A represents a leaving group, which is optionally purified and/or isolated,
and at least one compound of general formula V is reacted with at least one compound of general formula $R^3$-H, wherein $R^3$ has the meaning according to one or more of claims 1 to 16, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula I, wherein $R^1$, $R^2$, X, $R^3$ and $R^4$ have the meaning according to one or more of claims 1 to 16 and $R^5$ represents hydrogen, which is optionally purified and/or isolated;
or at least one compound of general formula IV is reacted with at least one compound of general formula $R^3$-H, wherein $R^3$ represents a —$NR^7R^8$ moiety, whereby $R^7$ and $R^8$ have the meaning according to one or more of claims 1 to 16, in a reaction medium, in the presence of at least one coupling agent, optionally in the presence of at least one base, to yield at least one compound of general formula I, wherein $R^1$, $R^2$, X and $R^4$ have the meaning according to one or more of claims 1 to 16, $R^3$ represents a -$NR^7R^8$ moiety and $R^5$ represents hydrogen, which is optionally purified and/or isolated.

**18.** Process for the preparation of a compound of general formula I according to one or more of claims 1 to 16, wherein $R^5$ represents hydrogen, **characterized in that** at least one compound of general formula $R^1$-C(=O)-H (general formula VII), wherein $R^1$ has the meaning according to one or more of claims 1 to 16, is reacted with at least one compound of general formula VI,

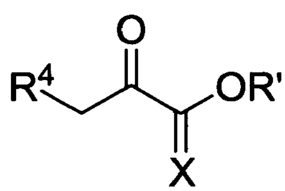

VI,

wherein $R^4$ and X have the meaning according to one or more of claims 1 to 16 and R' represents a linear or branched $C_{1-6}$-alkyl radical, a potassium cation or a sodium cation, in a reaction medium, optionally in an inert atmosphere, optionally in the presence of at least one base, to yield at least one compound of general formula II,

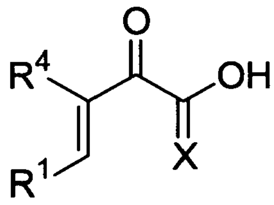

II,

wherein $R^1$, X and $R^4$ have the meaning according to one or more of claims 1 to 16, which is optionally purified and/or isolated,
and at least one compound of general formula II is reacted with an activating agent in a reaction medium, optionally in an inert atmosphere, to yield at least one compound of general formula VIII,

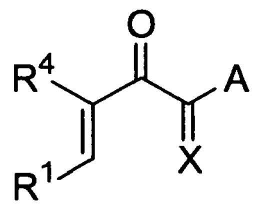

VIII,

wherein $R^1$, X and $R^4$ have the meaning according to one or more of claims 1 to 16 and A represents a leaving group, which is optionally purified and/or isolated,
and at least one compound of general formula VIII is reacted with at least one compound of general formula $R^3$-H, wherein $R^3$ has the meaning according to one or more of claims 1 to 16, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula IX,

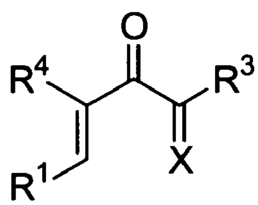

IX,

wherein $R^1$, X, $R^3$ and $R^4$ have the meaning according to one or more of claims 1 to 16, which is optionally purified and/or isolated;
or at least one compound of general formula II is reacted with at least one compound of general formula $R^3$-H, wherein $R^3$ represents a —$NR^7R^8$ moiety, whereby $R^7$ and $R^8$ have the meaning according to one or more of claims 1 to 16, in a reaction medium, in the presence of at least one coupling agent, optionally in the presence of at least one base, to yield at least one compound of general formula IX, wherein $R^3$ represents a —$NR^7R^8$ moiety, which is optionally purified and/or isolated,
and at least one compound of general formula IX is reacted with at least one compound of general formula III,

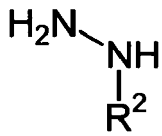

III,

wherein $R^2$ has the meaning according to one or more of claims 1 to 16, in a reaction medium, optionally in an inert atmosphere, optionally in the presence of at least one acid, to yield at least one compound of general formula I, wherein $R^1$, X, $R^2$, $R^3$ and $R^4$ have the meaning according to one or more of claims 1 to 16 and $R^5$ represents hydrogen, which is optionally purified and/or isolated.

**19.** Process for the preparation of a compound of general formula I according to one or more of claims 1 to 16, wherein $R^5$ is unlike hydrogen, **characterized in that** at least one compound of general formula $R^1$-C(=O)-H (general formula Vil), wherein $R^1$ has the meaning according to one or more of claims 1 to 16, is reacted with at least one compound of general formula VIb,

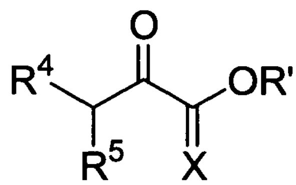

VIb,

wherein $R^4$, $R^5$ and X have the meaning according to one or more of claims 1 to 16; $R^5$ is unlike hydrogen and R' represents a linear or branched $C_{1-6}$-alkyl radical, a potassium cation or a sodium cation, in a reaction medium, optionally in an inert atmosphere, optionally in the presence of at least one base, to yield at least one compound of general formula XXXI,

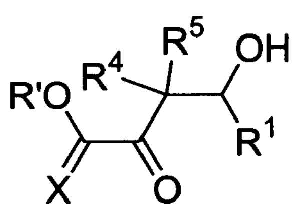

XXXXI

wherein $R^1$, $R^4$, $R^5$ and X have the meaning according to one or more of claims 1 to 16, $R^5$ is unlike hydrogen, and R' represents a linear or branched $C_{1-6}$-alkyl radical, a potassium cation or a sodium cation, and at least one compound of general formula XXXXI is reacted with a reagent, that transforms a hydroxyl group into a leaving group, preferably with a reagent selected from the group consisting of methansulfonylchloride and toluolsulfonylchloride, in a reaction medium, optionally in the presence of at least one base, preferably in the presence of at least one base selected from the group consisting of triethylamine, diisopropylethylamine, pyridine and N-methylmorpholine, to yield a compound of general formula XXXXII,

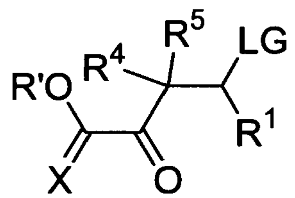

XXXXII

wherein $R^1$, $R^4$, $R^5$ and X have the meaning according to one or more of claims 1 to 16, $R^5$ is unlike hydrogen, R' represents a linear or branched $C_{1-6}$-alkyl radical, a potassium cation or a sodium cation, and LG is a leaving group, preferably mesyl or tosyl;
and at least one compound of general formula XXXXII is reacted with at least one compound of general formula III,

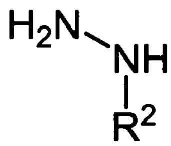

III,

or a corresponding salt thereof, wherein $R^2$ has the meaning according to one or more of claims 1 to 16, in a reaction medium, optionally in an inert atmosphere, optionally in the presence of at least one acid, to yield at least one compound of general formula IVb,

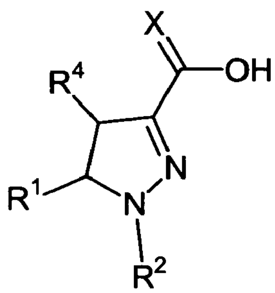

IVb,

wherein $R^1$, X, $R^2$, $R^4$ and $R^5$ have the meaning according to one or more of claims 1 to 16 and $R^5$ is unlike hydrogen, which is optionally isolated and/or purified,
and at least one compound of general formula IVb is reacted with an activating agent in a reaction medium, optionally in an inert atmosphere, to yield at least one compound of general formula Vb,

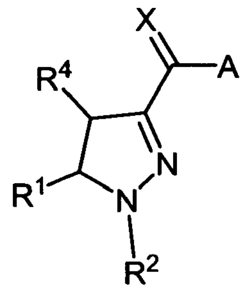

Vb,

wherein $R^1$, X, $R^2$, $R^4$ and $R^5$ have the meaning according to one or more of claims 1 to 16, $R^5$ is unlike hydrogen and A represents a leaving group, which is optionally purified and/or isolated,
and at least one compound of general formula Vb is reacted with at least one compound of general formula $R^3$-H, wherein $R^3$ has the meaning according to one or more of claims 1 to 16, in a reaction medium, optionally in an inert atmosphere, optionally in the presence of at least one base selected from the group consisting of diisopropylethylamine, triethylamine, pyridine, dimethylaminopyridine and N-methylmorpholine, to yield at least one compound of general formula I, wherein $R^1$, $R^2$, X, $R^3$, $R^4$ and $R^5$ have the meaning according to one or more of claims 1 to 16 and $R^5$ is unlike hydrogen, which is optionally purified and/or isolated;
or at least one compound of general formula IVb is reacted with at least one compound of general formula $R^3$-H, wherein $R^3$ represents a $-NR^7R^8$ moiety, whereby $R^7$ and $R^8$ have the meaning according to one or more of claims 1 to 16, in a reaction medium, in the presence of at least one coupling agent, optionally in the presence of at least one base, to yield at least one compound of general formula I, wherein $R^1$, $R^2$, X, $R^4$ and $R^5$ have the meaning according to one or more of claims 1 to 16, $R^3$ represents a $—NR^7R^8$ moiety and $R^5$ is unlike hydrogen, which is optionally purified and/or isolated.

**20.** Medicament comprising at least one compound according to one or more of claims 1 to 16 and optionally at least one physiologically acceptable auxiliary agent.

**21.** Medicament according to claim 20 for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**22.** Medicament according to claim 20 or 21 for the prophylaxis and/or treatment of food intake disorders, preferably bulimia, anorexia, cachexia, obesity, type II diabetes mellitus (non-insuline dependent diabetes mellitus), more preferably obesity.

**23.** Medicament according to claim 20 or 21 for the prophylaxis and/or treatment of psychosis.

**24.** Medicament according to claim 20 or 21 for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**25.** Medicament according to claim 20 or 21 for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**26.** Medicament according to claim 20 or 21 for the prophylaxis and/or treatment of one or more disorders selected from the group consisting of bone disorders, preferably osteoporosis (e.g. osteoporosis associated with a genetic predisposition, sex hormone deficiency, or aging), cancer-associated bone disease or Paget's disease of bone;

schizophrenia, anxiety, depression, epilepsy, neurodegenerative disorders, cerebella disorders, spinocerebella disorders, cognitive disorders, cranial trauma, head trauma, stroke panic attacks, peripheral neuropathy, glaucoma, migraine, Morbus Parkinson, Morbus Huntington, Morbus Alzheimer, Raynaud's disease, tremblement disorders, compulsive disorders, senile dementia, thymus disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

**27.** Use of at least one substituted pyrazoline compound according to one or more of claims 1 to 16 for the preparation of a medicament for the modulation of cannabinoid-receptors, preferably cannabinoid 1 ($CB_1$) receptors, for the prophylaxis and/or treatment of disorders of the central nervous system, disorders of the immune system, disorders of the cardiovascular system, disorders of the endocrinous system, disorders of the respiratory system, disorders of the gastrointestinal tract or reproductive disorders.

**28.** Use of at least one substituted pyrazoline compound according to one or more of claims 1 to 16 for the preparation of a medicament for the prophylaxis and/or treatment of food intake disorders, preferably bulimia, anorexia, cachexia, obesity, type II diabetes mellitus (non-insuline dependent diabetes mellitus), more preferably obesity.

**29.** Use of at least one substituted pyrazoline compound according to one or more of claims 1 to 16 for the preparation of a medicament for the prophylaxis and/or treatment of psychosis.

**30.** Use of at least one substituted pyrazoline compound according to one or more of claims 1 to 16 for the preparation of a medicament for the prophylaxis and/or treatment of alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction.

**31.** Use of at least one substituted pyrazoline compound according to one or more of claims 1 to 16 for the preparation of a medicament for the prophylaxis and/or treatment of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer.

**32.** Use of at least one substituted pyrazoline compound according to one or more of claims 1 to 16 for the preparation of a medicament for the prophylaxis and/or treatment of one or more disorders selected from the group consisting of bone disorders, preferably osteoporosis (e.g. osteoporosis associated with a genetic predisposition, sex hormone deficiency, or aging), cancer-associated bone disease or Paget's disease of bone; schizophrenia, anxiety, depression, epilepsy, neurodegenerative disorders, cerebella disorders, spinocerebella disorders, cognitive disorders, cranial trauma, head trauma, stroke, panic attacks, peripheral neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorrhagic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit.

**33.** A method of modulating cannabinoid-receptors, preferably cannabinoid 1 ($CB_1$) receptors, of preventing and/or treating disorders of the central nervous system, disorders of the immune system, disorders of the cardiovascular system, disorders of the endocrinous system, disorders of the respiratory system, disorders of the gastrointestinal tract or reproductive disorders, comprising administering to a subject, preferably a human, therapeutically effective amount of at least one substituted pyrazoline compound according to one or more of claims 1-16.

**34.** A method of treating food intake disorders, preferably bulimia, anorexia, cachexia, obesity, type II diabetus mellitus (non-insuline dependent diabetes mellitus), more preferably obesity, comprising administering to a subject, preferably a human, a therapeutically effective amount of at least one substituted pyrazoline compound according to one or more of claims 1-16.

**35.** A method of treating psychosis comprising administering to a subject, preferably a human, a therapeutically effective

amount of at least one substituted pyrazoline compound according to one or more of claims 1-16.

**36.** A method of treating alcohol abuse and/or alcohol addiction, nicotine abuse and/or nicotine addiction, drug abuse and/or drug addiction and/or medicament abuse and/or medicament addiction, preferably drug abuse and/or drug addiction and/or nicotine abuse and/or nicotine addiction, comprising administering to a subject, preferably a human, a therapeutically effective amount of at least one substituted pyrazoline compound according to one or more of claims 1-16.

**37.** A method of treating of cancer, preferably for the prophylaxis and/or treatment of one or more types of cancer selected from the group consisting of brain cancer, bone cancer, lip cancer, mouth cancer, esophageal cancer, stomach cancer, liver cancer, bladder cancer, pancreas cancer, ovary cancer, cervical cancer, lung cancer, breast cancer, skin cancer, colon cancer, bowel cancer and prostate cancer, more preferably for the prophylaxis and/or treatment of one or more types of cancer selected from the group consisting of colon cancer, bowel cancer and prostate cancer comprising administering to a subject, preferably a human, a therapeutically effective amount of at least one substituted pyrazoline compound according to one or more of claims 1-16.

**38.** A method of treating one or more disorders selected from the group consisting of bone disorders, preferably osteoporosis (e.g. osteoporosis associated with a genetic predisposition, sex hormone deficiency, or ageing), cancer-associated bone disease or Paget's disease of bone; schizophrenia, anxiety, depression, epilepsy, neurodegenerative disorders, cerebellar disorders, spinocerebellar disorders, cognitive disorders, cranial trauma, head trauma, stroke panic attacks, peripheric neuropathy, glaucoma, migraine, Morbus Parkinson, Morbus Huntington, Morbus Alzheimer, Raynaud's disease, tremblement disorders, compulsive disorders, senile dementia, thymic disorders, tardive dyskinesia, bipolar disorders, medicament-induced movement disorders, dystonia, endotoxemic shock, hemorragic shock, hypotension, insomnia, immunologic disorders, sclerotic plaques, vomiting, diarrhea, asthma, memory disorders, pruritus, pain, or for potentiation of the analgesic effect of narcotic and non-narcotic analgesics, or for influencing intestinal transit, comprising administering to a subject, preferably a human, a therapeutically effective amount of at least one substituted pyrazoline compound according to one or more of claims 1-16.

European Patent Office

# PARTIAL EUROPEAN SEARCH REPORT

which under Rule 45 of the European Patent Convention shall be considered, for the purposes of subsequent proceedings, as the European search report

Application Number
EP 05 38 4029

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 5 624 941 A (BARTH ET AL) 29 April 1997 (1997-04-29) see general formula, especially definItions of R and examples | 1-38 | C07D231/06 C07D403/12 C07D409/04 A61K31/4155 A61K31/415 A61P3/04 A61P25/30 A61P35/00 |
| Y | WO 01/70700 A (SOLVAY PHARMACEUTICALS B.V) 27 September 2001 (2001-09-27) see general formula | 1-38 | |
| X | D POCAR: "preparation of 1-aryl-4,5- and 5,5-diamino-4,5-dihydropyrazoles" SYNTHESIS, vol. 10, 1976, pages 684-685, XP009058075 see compounds 4a,4d | 1,2,4-7 | |
| X | L GARANTI: "intramolecular 1,3-dipolar cycloadditions of nitrile imines bearing an alkenyl substituent" JOURNAL OF ORGANIC CHEMISTRY, vol. 42, no. 8, 1977, pages 1389-1392, XP002357709 see compound 6b | 1-7,13 | TECHNICAL FIELDS SEARCHED (IPC) |
| | -/-- | | C07D A61K |

## INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

see sheet C

1

| Place of search | Date of completion of the search | Examiner |
|---|---|---|
| Munich | 7 December 2005 | Scruton-Evans, I |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

European Patent Office

# PARTIAL EUROPEAN SEARCH REPORT

Application Number
EP 05 38 4029

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | H BIERE: "nichtsteroidale entzündungshemmer.5" EUROPEAN JOURNAL OF MEDICINAL CHEMISTRY, vol. 17, no. 1, 1982, pages 27-34, XP009058170 see 1c,11c and 15c ----- | 1,2,4-7 | |
| X | L GARANTI: "silver carbonate promoted reaction of hydrazonyl chlorides with allylic alcohols" HETEROCYCLES, vol. 40, no. 2, 1995, pages 777-786, XP009058115 see compound 4e ----- | 1,2,4-7 | |
| X | S M ELDIN: "hydrazidoyl halides in heterocyclic synthesis" EGYPTIAN JOURNAL OF PHARMACEUTICAL SCIENCES, vol. 37, no. 1-6, 1996, pages 351-362, XP009058072 see compounds 6d,6e and 13 ----- | 1 | TECHNICAL FIELDS SEARCHED (IPC) |
| X | A DONOHUE: "cycloaddition of nitrile imines to resin bound enamines" JOURNAL OF THE CHEMICAL SOCIETY, PERKIN TRANS 1, vol. 21, 2001, pages 2817-2822, XP002357710 see compounds 4,12a,15 ----- | 1,2,4-7 | |
| X | H.A.ALBAR: "synthesis of dipyrazole ketones" JOURNAL OF SAUDI CHEMICAL SOCIETY, vol. 5, no. 3, 2001, pages 353-362, XP009058073 see compound 10 ----- | 1,2,4-7 | |

1

EPO FORM 1503 03.82 (P04C10)

European Patent Office

INCOMPLETE SEARCH SHEET C

Application Number
EP 05 38 4029

Although claims 33-38 are directed to a method of treatment of the human/animal body (Article 52(4) EPC), the search has been carried out and based on the alleged effects of the compound/composition.

-----

## ANNEX TO THE EUROPEAN SEARCH REPORT ON EUROPEAN PATENT APPLICATION NO. EP 05 38 4029

This annex lists the patent family members relating to the patent documents cited in the above-mentioned European search report.
The members are as contained in the European Patent Office EDP file on
The European Patent Office is in no way liable for these particulars which are merely given for the purpose of information.

07-12-2005

| Patent document cited in search report | | Publication date | Patent family member(s) | | | Publication date |
|---|---|---|---|---|---|---|
| US 5624941 | A | 29-04-1997 | NONE | | | |
| WO 0170700 | A | 27-09-2001 | AU | 4250101 | A | 03-10-2001 |
| | | | BR | 0109457 | A | 03-06-2003 |
| | | | CA | 2401832 | A1 | 27-09-2001 |
| | | | CN | 1419546 | A | 21-05-2003 |
| | | | HK | 1052349 | A1 | 23-09-2005 |
| | | | HU | 0204519 | A2 | 28-05-2003 |
| | | | JP | 2004500401 | T | 08-01-2004 |
| | | | NO | 20024531 | A | 19-11-2002 |
| | | | PL | 358101 | A1 | 09-08-2004 |
| | | | SK | 13522002 | A3 | 04-03-2003 |
| | | | US | 2001053788 | A1 | 20-12-2001 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **HOLLISTER.** *Pharm. Rev.,* 1986, vol. 38, 1-20
- **RENY ; SINGHA.** *Prog. Drug. Res.,* 1991, vol. 36, 71-114
- **CONSROE ; SANDYK.** Marijuana/Cannabinoids, Neurobiology and Neurophysiology. CRC Press, 1992
- *Synthetic Communications,* 1996, vol. 26 (11), 2229-33
- *Synlett,* 2001, 147-149
- **PASCUAL, A.** *J. Prakt Chem.,* 1999, vol. 341 (7), 695-700
- **LIN, S. et al.** *Heterocycles,* 2001, vol. 55 (2), 265-277
- **RAO, P. et al.** *J. Org. Chem.,* 2000, vol. 65 (22), 7323-7344
- **PEARSON D.E ; BUEHLER, C.A.** *Synthesis,* 1972, 533-542
- *Tetrahedron Lett.,* 2004, vol. 45, 4977
- *Tetrahedron Lett.,* 1998, vol. 39, 1487
- *Tetrahedron Lett.,* 2002, vol. 43, 7685
- *Tetrahedron,* 2005, vol. 81, 5235-5240
- *Tetrahedron,* 19 December 2001
- *Tetrahedron Lett.,* 1998, vol. 39 (44), 8163-8166
- *J. Chem. Soc. Perkin Trans 1,* 1999, vol. 21, 3069-3070
- *Tetrahedron,* 1999, vol. 55 (36), 11127-11142
- *J. Heterocyclic Chem.,* 1986, vol. 23, 1199
- *Chemistry of Heterocyclic Compounds,* 1997, vol. 33 (6
- *Indian J. Chem.,* 1981, vol. 20B, 1090
- *Indian J. Chem.,* 1990, vol. 29B, 887
- *J. Indian Chem. Soc.,* 1997, vol. 74 (3), 202-205
- *Tetrahedron,* 1994, vol. 50 (44), 12727-12742
- *Zhumal Obshchei Khimii,* 1986, vol. 56 (2), 347-353
- *J. Chem. Soc. Perkin Trans,* 1995, vol. 1, 741-742
- *J. Chem. Engineering Data,* 1984, vol. 29 (2), 225-229
- *Indian J. Chem.,* 1988, vol. 27B (3), 245-249
- *Bull. Chem. Soc. Japan,* 1984, vol. 57 (3), 787-790
- *Chem. Lett.,* 1982, 543-546
- *Synth. Commun.,* 2001, vol. 31 (1), 111-115
- *Tetrahedron,* 1994, vol. 50 (25), 7543-7556
- *Synthesis,* 1975, vol. 333
- *J. Chem. Soc. Perkin Trans.,* 1977, vol. 1, 2092
- *Chem. Lett.,* 1983, 507-510
- *Bull. Chem. Soc. Japan,* 1984, vol. 57 (9), 2689-2690
- *Synlett,* 1990, vol. 11, 705-706
- *J. Org. Chem.,* 2002, vol. 67, 6237-6239
- Protective groups in Organic Chemistry. Plenum Press, 1973
- **T.W. GREENE ; P.G.M. WUTS.** Protective Groups in Organic Chemistry. John Wiley & sons, 1991
- **R. B. SILVERMAN.** The Organic Chemistry of Drug Design and Drug Action. Academic Press, 1992
- **ECKEL et al.** *The Lancet,* 2005, vol. 365, 1415-1428
- **ALBERTI et al.** *Diabet. Med.,* 1998, vol. 15, 539-53
- *JAMA,* 2001, vol. 285, 2486-97
- **PAGOTTO ; PASQUALI.** *The Lancet,* 2005, vol. 365, 1363, 1364
- Pharmaceutics: The Science of Dosage Forms. Churchill Livingstone, 2002
- Encyclopedia of Pharmaceutical Technology. Marcel Dekker, Inc, 2002
- Modem Pharmaceutics. Marcel Dekker, Inc,
- The Theory and Practice of Industrial Pharmacy. Lea & Febiger, 1986
- Modified-Release Drug Delivery Technology. Marcel Dekker, Inc, 2002
- Handbook of Pharmaceutical Controlled Release Technology. Marcel Dekker, Inc, 2000
- Controlled Drug Delivery. Basic Concepts. CRD Press Inc, 1983, vol. I
- Oral Drug Delivery. **TAKADA, K. ; YOSHIKAWA, H.** Encyclopedia of Controlled Drug Delivery. John Wiley & Sons, Inc, 1999, vol. 2, 728-742
- Oral drug delivery, small intestine and colon. **FIX, J.** Encyclopedia of Controlled Drug Delivery. John Wiley & Sons, Inc, 1999, vol. 2, 698-728
- **RUTH A. ROSS ; HEATHER C. BROCKIE et al.** Agonist-inverse agonist characterization at CB1 and CB2 cannabinoid receptors of L-759633, L759656 and AM630. *British Journal of Pharmacology,* 1999, vol. 126, 665-672

- **A. C. HOWLETT et al.** Intemational Union of Pharmacology XXVII. *Classification of Cannabinoid Receptors, Pharmacol Rev,* 2002, vol. 54, 161-202
- **DAVID R. COMPTON et al.** In-vivo Characterization of a Specific Cannabinoid Receptor Antagonist (SR141716A) :Inhibition of Tetrahydrocannbinol- induced Responses and Apparent Agonist Activity. *J. Pharmacol. Exp. Ther.,* 1996, vol. 277 (2), 586-594
- **WOOLFE D. et al.** The evaluation of analgesic action of pethidine hydrochloride (Demerol. *J. Pharmacol. Exp. Ther.,* 1944, vol. 80, 300-307
- **DESMET L. K. C. et al.** Anticonvulsive properties of Cinarizine and Flunarizine in Rats and Mice. *Arzneim. -Forsch. (Frug Res,* 1975, vol. 25, 9
- **DAVID R. COMPTON et al.** In-vivo Characterization of a Specific Cannabinoid Receptor Antagonist (SR141716A) Inhibition of Tetrahydrocannbinol- induced Responses and Apparent Agonist Activity. *J. Pharmacol Exp Ther.,* 1996, vol. 277 (2), 586-594
- **ALPERMANN H. G. et al.** Pharmacological effets of Hoe 249: A new potential antidepressant. *Drugs Dev. Res.,* 1992, vol. 25, 267-282
- **G. COLOMBO et al.** Appetite Suppression and Weight Loss after the Cannabinoid Antagonist SR 141716. *Life Sciences,* 1998, vol. 63 (8), 113-117